# EUROPEAN PATENT APPLICATION

(11) **EP 4 759 928 A1**
(43) Date of publication of application: **17.06.2026**
(21) Application number: 23948956.0
(22) Date of filing: 15.09.2023
(51) Int. Cl.: C12N 9/88, C12N 15/60, C12N 15/81, C12P 5/00

(54) **VALENCENE SYNTHASE AND USE THEREOF**

(30) Priority: 11.08.2023 CN 202311010898
(71) Applicant: Wuhan Hesheng Technology Co., Ltd., Wuhan, Hubei 430070 (CN)
(72) Inventor: YE, Ziling, Wuhan, Hubei 430070 (CN)
(74) Representative: Elzaburu S.L.P.
(86) International application number: PCT/CN2023/119137
(87) International publication number: WO 2025/035531

(57) **Abstract**

The present invention relates to the technical field of biology, and in particular to a valencene synthase and a use thereof. Pfam domains of terpene synthases PF01397 and PF03936 are used as core domains of novel valencene synthases, a novel valencene synthase having valencene synthase activity is obtained by screening, and an encoding nucleic acid molecule is designed on the basis of the amino acid sequence of the novel enzyme obtained by screening. Furthermore, a recombinant plasmid vector and an expression system for expressing the novel enzyme are prepared; the recombinant plasmid vector and the expression system can be applied to the synthesis of different citrus fragrances, thereby overcoming the time-consuming and labor-consuming problems of extracting essential oil from plants, and promoting the industrial application of the citrus fragrances.

## Description

### Technical Field

The present invention relates to the field of biotechnology, and in particular to a valencene synthase and use thereof.

### Background Art

Valencene, a sesquiterpene compound that has been reported in various plant essential oils such as grape seed essential oil and citrus essential oil, has a unique odor, and can be used in food products or daily chemical products. However, the entire planting and extracting process for plant essential oils is very complex, time-consuming, and labor-intensive, and entails the consumption of large amounts of plant peels, so the extraction cost thereof is quite high, and the value thereof is also quite expensive. Moreover, the content of valencene in natural plant extracts is very low, and it is difficult to separate plant essential oils containing high concentrations of valencene by using natural product separation methods. Therefore, finding a more economical method to obtain an essential oil substance having a high valencene concentration and a unique odor is an inevitable trend to achieve economic efficiency and sustainable development.

### Summary of the Invention

An objective of the present invention is to provide a valencene synthase and a use of the valencene synthase in an expression system in the preparation of a composition having a unique fragrance and valencene as the main component, so as to achieve the purpose of efficient and sustainable production.

In order to achieve the above objective, the present invention provides the following technical solutions:
In the first aspect, the present invention provides a valencene synthase, comprising Pfam domains numbered PF01397 and PF03936 and having valencene synthase activity.

In an optional embodiment, the valencene synthase comprises (a) an intact protein or functional protein fragment derived from a plant; or (b) a derivative valencene synthase having at least 80% or higher amino acid sequence homology to (a).

In an optional embodiment, the plant is selected from plants of the family *Rutaceae* or plants of the family *Ranunculaceae Juss.*

In an optional embodiment, the plant is selected from plants of the genus *Cimicifuga* or plants of the genus *Citrus L.*

In an optional embodiment, the plants of the genus *Cimicifuga* comprise *Actaea asiatica,* preferably *Actaea racemosa,* and the plants of the genus Citrus L. comprise *Citrus maxima.*

In an optional embodiment, the valencene synthase catalyzes the synthesis of valencene in an expression system.

In an optional embodiment, the expression system comprises a fungal expression system.

In an optional embodiment, the fungal expression system comprises a yeast expression system.

In an optional embodiment, the yeast expression system comprises a *Saccharomyces cerevisiae* expression system.

In an optional embodiment, the amino acid sequence of the valencene synthase comprises (a) or (b):
(a) any one of SEQ ID NOs: 1-3;
(b) a derivative valencene synthase having at least 83%, 85%, 87%, 89%, 91%, 93%, 95%, 97%, or 99% or higher amino acid sequence homology to (a).

In the second aspect, the present invention provides a use of the valencene synthase according to any one of the preceding embodiments in the preparation of a fragrance composition.

In the third aspect, the present invention provides a nucleic acid molecule, comprising a nucleotide sequence encoding the valencene synthase according to any one of the preceding embodiments or a nucleotide sequence having bases complementarity thereto.

In an optional embodiment, the nucleic acid molecule comprises an amino acid sequence having at least 90%, 91%, 92%, 93%, 94%, 95%, 96%, 97%, 98%, 99%, or 100% sequence homology to any one of nucleotide sequences as set forth in SEQ ID Nos: 4-6.

In the fourth aspect, the present invention provides a recombinant expression vector, containing the nucleic acid molecule according to any one of the preceding embodiments. The original vector of the recombinant expression vector is pZY900, pYR017, or pYR018.

In the fifth aspect, the present invention provides a transformant. The host cell of the transformant is selected from a fungal expression system. The transformant contains the nucleic acid molecule according to any one of the preceding embodiments or the recombinant expression vector according to the preceding embodiments. Preferably, the fungal expression system is a yeast expression system, further preferably a *Saccharomyces cerevisiae* expression system.

In the sixth aspect, the present invention provides a recombinant *Saccharomyces cerevisiae.* The recombinant *Saccharomyces cerevisiae* contains the nucleic acid molecule according to any one of the preceding embodiments or the recombinant expression vector according to the preceding embodiments. Preferably, the original vector of the recombinant expression vector is pZY900, pYR017, or pYR018.

In the seventh aspect, the present invention provides a fragrance composition comprising 10-99% by mass of valencene. The valencene is synthesized under catalysis of the valencene synthase according to any one of the preceding embodiments.

In the eighth aspect, the present invention provides a preparation method for the fragrance composition according to any one of the preceding embodiments. The preparation method comprises fermenting and culturing the transformant or the recombinant *Saccharomyces cerevisiae* according to the preceding embodiments, and recovering the fragrance composition from a fermentation product.

In an optional embodiment, the recovery method comprises extracting the fermentation product with an oil phase.

In an optional embodiment, the oil phase comprises at least one of isopropyl myristate or n-dodecane.

In an optional embodiment, the recovery method is: during fermentation, adding the oil phase; and after the fermentation is complete, collecting the oil phase and performing separation to obtain a fragrance composition enriched in the oil phase.

In an optional embodiment, a method for collecting the oil phase comprises centrifugation.

In an optional embodiment, a method for the separation comprises rectification.

In the ninth aspect, the present invention provides a use of the fragrance composition according to any one of the preceding embodiments or a fragrance composition obtained by the preparation method according to any one of the preceding embodiments in the preparation of a fragrance product.

In the tenth aspect, the present invention provides a fragrance product containing the fragrance composition according to any one of the preceding embodiments or a fragrance composition prepared by the method according to any one of the preceding embodiments.

In an optional embodiment, the fragrance product comprises: a personal care product, a fabric care product, a cleaning product, a deodorant, a home care product, a fabric product, an air care product, a fragrance delivery system, a cosmetic preparation, a disinfectant, or a food product.

In an optional embodiment, the personal care product comprises: floral water, a skin care product, a hair care product, an antiperspirant, a feminine care product, a baby care product, an oral hygiene product, or a health care product.

In an optional embodiment, the skin care product comprises: a scented soap, a liquid soap, or a shower gel.

In an optional embodiment, the oral hygiene product comprises a dental hygiene product.

In an optional embodiment, the cosmetic preparation comprises a perfume, a fragrance liquid, or an essential oil.

In an optional embodiment, the cleaning product comprises a stain-removing composition.

In an optional embodiment, the stain-removing composition comprises: a dishwashing detergent, a glass cleaner, a metal cleaner, a countertop cleaner, a floor cleaner, a carpet cleaner, a toilet cleaner, or a bleaching additive.

The inventors used Pfam domains PF01397 and PF03936 of terpene synthase as core domains of the valencene synthase, with the expectation of screening out new synthases and finally a novel synthase having valencene synthase activity, and designed an encoding nucleic acid molecule for the amino acid sequence of the screened valencene enzyme, which was then used to prepare a recombinant plasmid vector and an expression system for expressing the valencene synthase. The valencene synthase has the potential to be applied in the synthesis of various citrus fragrances, making up for the time-consuming and labor-intensive plant extraction process and promoting the industrial application of citrus fragrances.

Fermentation and culturing of the above expression system can realize the expression and preparation of the fragrance composition and obtain a fragrance composition with valencene as the main component.

Furthermore, the present invention for the first time provides valencene synthases derived from plants of the family *Rutaceae* and plants of the family *Ranunculaceae Juss.* and combines a yeast expression system for fermentation to produce a composition having citrus fragrance. The amino acid sequences of the two valencene synthases are respectively as set forth in SEQ ID NO: 1 (ArVS derived from *Actaea racemosa),* SEQ ID NO: 2 (ArVS-V8 derived from *Actaea racemosa*), and SEQ ID NO: 3 (CmVS-V4 derived from *Citrus maxima*).

Furthermore, using the microbial fermentation method instead of extracting plant-derived fragrance products from natural plant raw materials can alleviate the impact of climate on natural plant growth, resulting in more stable yield and quality. Furthermore, reducing the demand for plant raw materials can release more planting resources for other energy crops. Therefore, the microbial fermentation method provided by the present invention for preparing the composition having a unique fragrance has application prospects.

### Brief Description of the Drawings

In order to illustrate the technical solutions in the particular embodiments of the present invention or in the prior art more clearly, a brief introduction will be given below to the drawings required in the description of the particular embodiments or the prior art. Apparently, the drawings in the following description show some embodiments of the present invention, and those of ordinary skill in the art would be able to derive other drawings from these drawings without involving any inventive effort.
FIG. 1 shows a schematic diagram of the construction of plasmid pArVS;
FIG. 2 shows a schematic diagram of the construction of plasmid pArVS-V8;
FIG. 3 shows a schematic diagram of the construction of plasmid pCmVS-V4; and
FIG. 4 shows schematic diagrams of the construction of plasmids pVvVS, pCsVS, pShVS, pSlVS, pPvVS, pCnVS, pEgVS, and pAoVS.

### Detailed Description of Embodiments

In order to make the objectives, technical solutions and advantages of the embodiments of the present invention more clear, the technical solutions in the embodiments of the present invention will be described clearly and completely below with reference to the accompanying drawings for the embodiments of the present invention. Apparently, the described embodiments are merely some, rather than all, of the embodiments of the present invention.

### (I) Definition or terminology

The following abbreviations apply to the relevant definitions or terms involved in the present invention. Unless otherwise defined, all scientific and technical terms used herein have the meanings commonly understood by those of ordinary skill in the art. The following terms are provided below.

### 1. Protein family classification database (Pfam database)

The Pfam database is a commonly used protein family database, created based on protein data collected in the UniProt database. To date, it has been updated to version 35.0 (November 2021, 19632 pieces of data). Data corresponding to a certain gene family, e.g., hmm model files, alignment sequences, species information, and HMM logo, can be downloaded from this database. The hmm model file contains sequence feature information about the gene family. In conjunction with hmmer software, the members of the gene family can be identified. The relevant website link is: https://www.ebi.ac.uk/interpro/entry/pfam/.

### 2. Pfam domains

The "Pfam domain" refers to a region within a protein sequence that is identified as Pfam-A or Pfam-B. The identification process or method is designed based on multiple sequence alignments and the presence of hidden Markov motifs ("The Pfam protein families database": R.D. Finn, J. Mistry, J. Tate, P. Coggill, A. Heger, J.E. Pollington, O.L. Gavin, P. Gunesekaran, G. Ceric, K. Forslund, L. Holm, E.L. Sonnhammer, S.R. Eddy, A. Bateman, Nucleic Acids Research (2010) Database Issue 38: D211-222.).

### 3. NIST

The NIST in the present invention means the National Institute of Standards and Technology (NIST), which is directly under the U.S. Department of Commerce. The NIST is engaged in fundamental and application research in physics, biology, and engineering, as well as research in measurement technology and testing methods. It provides standards, standard reference data, and related services and enjoys a high reputation internationally. In the identification of components in a composition obtained by fermentation in the present invention, reference is made to the compound name database of NIST.

### 4. Novel valencene synthase

The novel valencene synthase of the present invention refers to a valencene synthase that has not been successfully separated and purified before the present invention. This is because valencene is a terpene compound. Traditional terpene synthases are usually natural enzymes. Enzymes having terpene synthesis functions that are expressed in plant cells in natural environments are usually adapted to plant cell expression systems and terpene synthesis systems. The valencene synthase provided by the present invention is a novel enzyme that can be successfully expressed in an expression system and can synthesize valencene.

### 5. Homology

The "homology" in the present invention has the meaning well accepted in the art and means that the percentage of sequence identity between two nucleic acids, polypeptides, or regions can be calculated using publicly available techniques. For example, sequence identity can be measured along the full length of a polynucleotide or polypeptide or along a region of the molecule. Numerous methods for measuring identity between two polynucleotides or polypeptides have been currently available and can be selected by those skilled in the art as conventional choices according to actual needs.

### 6. Expression

The expression in the present invention refers to a process by which a polypeptide is produced by the transcription and translation of a polynucleotide. The expression level of a polypeptide may be evaluated by using any method known in the art, including, for example, a method for measuring the amount of a polypeptide produced from a host cell. Such methods can include, but are not limited to, quantitation of a polypeptide in a cell lysate by ELISA, Coomassie blue staining following gel electrophoresis, Lowry protein assay, and Bradford protein assay.

### 7. Host cell

The host cell in the present invention is a cell used for accommodating, maintaining, replicating, and amplifying a vector. The host cell may also be used for expressing a polypeptide encoded by the vector. Upon host cell division, the nucleic acid contained within the vector is replicated, thereby amplifying the nucleic acid. The host cell can be a eukaryotic cell or a prokaryotic cell.

### 8. Vector

The vector in the present invention refers to a replicable nucleic acid. One or more heterologous proteins can be expressed from the vector after the vector is transformed into a suitable host cell. Vectors include those into which a nucleic acid encoding a polypeptide or a fragment thereof can be introduced, typically by restriction digestion and ligation. Vectors also include those comprising a nucleic acid encoding a polypeptide. A vector is used for introducing a nucleic acid encoding a polypeptide into a host cell for the amplification of the nucleic acid or for the expression/display of a polypeptide encoded by the nucleic acid. A vector is generally maintained episomally but can be designed to enable integration of the gene or a portion thereof into a chromosomal genome. Vectors also encompass artificial chromosome vectors, such as yeast artificial vectors and mammalian artificial chromosomes. The selection and use of such vehicles are well-known to those skilled in the art.

### (II) Detailed technical solution

In specific embodiments, in the first aspect, the present invention provides a valencene synthase, comprising Pfam domains numbered PF01397 and PF03936 and having valencene synthase activity.

PF01397 is a conserved domain at the N-terminus of the terpene synthase, and PF03936 is a metal ion-binding domain at the C-terminus of the terpene synthase. Genes containing both Pfam domains PF01397 and PF03936 are screened out, and complete plant terpene synthases can be identified.

The Pfam database is a collection of a range of protein families. Each of the protein families is represented in the form of multiple sequence alignments and hidden Markov models.
1. Pfam database information is downloaded (http://ftp.ebi.ac.uk/pub/databases/Pfam/releases/Pfam35.0/).
2. A local database index is established by using hmmpress software.
3. Input protein sequences are aligned with an HMM domain library by using hmmscan software (E < 0.01), and domains contained in each protein are returned.
4. Results containing both PF03936 and PF01397 domains are screened out.

In an optional embodiment, the valencene synthase comprises (a) an intact protein or functional protein fragment derived from a plant; or (b) a derivative valencene synthase having at least 80% or higher amino acid sequence homology to (a).

Considering the characteristics of plant growth periodicity, yield, etc., developing microbial alternative production methods for plant essential oils or plant-derived fragrance products has greater economic value and social significance than developing microbial alternative production methods for fragrances from other sources.

In some preferred embodiments, the above derivatization process can be replacement at an amino acid site with another amino acid of the same class (having similar chemical properties or functions). By way of example, amino acids can be divided into: (1) non-polar amino acids: Ala (A), Val (V), Leu (L), Ile (I), Pro (P), Phe (F), Trp (W), and Met (M); (2) non-charged polar amino acids: Gly (G), Ser (S), Thr (T), Cys (C), Tyr (Y), Asn (N), and Gln (Q); (3) acidic amino acids: Asp (D) and Glu (E); and (4) alkaline amino acids: Lys(K), Arg(R), and His(H), according to their side chain properties. Alternatively, amino acids can be divided into: (1) hydrophobic amino acids: Met, Ala, Val, Leu, and Ile; (2) neutral hydrophilic amino acids: Cys, Ser, Thr, Asn, and Gln; (3) acidic amino acids: Asp and Glu; (4) alkaline amino acids: His, Lys, and Arg; (5) amino acids affecting chain orientation: Gly and Pro; and (6) aromatic amino acids: Trp, Tyr, and Phe, according to the common side chain characteristics. Conservative substitution of amino acids in the above homology classification has little to no effect on the function, activity, or other biological properties of the polypeptide.

In an optional embodiment, the plant is selected from plants of the family *Rutaceae* or plants of the family *Ranunculaceae Juss.*

In an optional embodiment, the plant is selected from plants of the genus *Cimicifuga* or plants of the genus *Citrus L.*

In an optional embodiment, the plants of the genus *Cimicifuga* comprise *Actaea asiatica,* preferably *Actaea racemosa,* and the plants of the genus Citrus L. comprise *Citrus maxima.*

The plants of the genus *Cimicifuga* are widely distributed and have been used as medicinal plants for a long time. In existing phytochemical research reports, extensively studied species include *Actaea asiatica* in China, and *Actaea racemosa* widely distributed across countries such as those in North America and Europe.

In an optional embodiment, the valencene synthase catalyzes the synthesis of valencene in an expression system.

In an optional embodiment, the expression system comprises a fungal expression system.

In an optional embodiment, the fungal expression system comprises a yeast expression system.

In an optional embodiment, the yeast expression system comprises a *Saccharomyces cerevisiae* expression system.

In an optional embodiment, the amino acid sequence of the valencene synthase comprises (a) or (b):
(a) any one of SEQ ID NOs: 1-3;
(b) a derivative valencene synthase having at least 83%, 85%, 87%, 89%, 91%, 93%, 95%, 97%, or 99% or higher amino acid sequence homology to (a).

In the second aspect, the present invention provides a use of the valencene synthase according to any one of the preceding embodiments in the preparation of a fragrance composition.

In the third aspect, the present invention provides a nucleic acid molecule, comprising a nucleotide sequence encoding the valencene synthase according to any one of the preceding embodiments or a nucleotide sequence having bases complementarity thereto.

In an optional embodiment, the nucleic acid molecule comprises an amino acid sequence having at least 90%, 91%, 92%, 93%, 94%, 95%, 96%, 97%, 98%, 99%, or 100% sequence homology to any one of nucleotide sequences as set forth in SEQ ID Nos: 4-6.

In the fourth aspect, the present invention provides a recombinant expression vector, containing the nucleic acid molecule according to any one of the preceding embodiments. The original vector of the recombinant expression vector is pZY900, pYR017, or pYR018.

In the fifth aspect, the present invention provides a transformant. The host cell of the transformant is selected from a fungal expression system. The transformant contains the nucleic acid molecule according to any one of the preceding embodiments or the recombinant expression vector according to the preceding embodiments. Preferably, the fungal expression system is a yeast expression system, further preferably a *Saccharomyces cerevisiae* expression system.

In the sixth aspect, the present invention provides a fragrance composition comprising 10-99% by mass of valencene, including but not limited to 10%, 20%, 30%, 40%, 50%, 60%, 70%, 80%, 90%, or 99%. The valencene is synthesized under catalysis of the valencene synthase according to any one of the preceding embodiments.

In the seventh aspect, the present invention provides a recombinant *Saccharomyces cerevisiae.* The recombinant *Saccharomyces cerevisiae* contains the nucleic acid molecule according to any one of the preceding embodiments or the recombinant expression vector according to the preceding embodiments. Preferably, the original vector of the recombinant expression vector is pZY900, pYR017, or pYR018.

In the eighth aspect, the present invention provides a preparation method for the fragrance composition according to any one of the preceding embodiments. The preparation method comprises fermenting and culturing the transformant or the recombinant *Saccharomyces cerevisiae* according to the preceding embodiments, and recovering the fragrance composition from a fermentation product.

In an optional embodiment, the recovery method comprises extracting the fermentation product with an oil phase.

In an optional embodiment, the oil phase comprises at least one of isopropyl myristate or n-dodecane.

In an optional embodiment, the recovery method is: during fermentation, adding the oil phase; and after the fermentation is complete, collecting the oil phase and performing separation to obtain a fragrance composition enriched in the oil phase.

In an optional embodiment, a method for collecting the oil phase comprises centrifugation.

In an optional embodiment, a method for the separation comprises rectification.

In the ninth aspect, the present invention provides a use of the fragrance composition according to any one of the preceding embodiments or a fragrance composition obtained by the preparation method according to any one of the preceding embodiments in the preparation of a fragrance product.

In the tenth aspect, the present invention provides a fragrance product containing the fragrance composition according to any one of the preceding embodiments or a fragrance composition prepared by the method according to any one of the preceding embodiments.

In an optional embodiment, the fragrance product comprises: a personal care product, a fabric care product, a cleaning product, a deodorant, a home care product, a fabric product, an air care product, a fragrance delivery system, a cosmetic preparation, a disinfectant, or a food product.

In an optional embodiment, the personal care product comprises: floral water, a skin care product, a hair care product, an antiperspirant, a feminine care product, a baby care product, an oral hygiene product, or a health care product.

In an optional embodiment, the skin care product comprises: a scented soap, a liquid soap, or a shower gel.

In an optional embodiment, the oral hygiene product comprises a dental hygiene product.

In an optional embodiment, the cosmetic preparation comprises a perfume, a fragrance liquid, or an essential oil.

In an optional embodiment, the cleaning product comprises a stain-removing composition.

In an optional embodiment, the stain-removing composition comprises: a dishwashing detergent, a glass cleaner, a metal cleaner, a countertop cleaner, a floor cleaner, a carpet cleaner, a toilet cleaner, or a bleaching additive.

Some embodiments of the present invention will be described below in detail with reference to the accompanying drawings. Without conflict, the following examples and the features in these examples can be combined with each other.

### Example 1. Preparation and use of valencene synthase derived from Actaea racemosa

### 1.1 Mining of valencene synthase genes from Actaea racemosa

Referring to published protein sequence information about *Actaea racemosa* (https://ftp.cngb.org/pub/gigadb/pub/10.5524/100001_101000/100627/assemblies/CYVA-Cim icifuga_racemosa/), protein sequences containing both Pfam domains PF01397 and PF03936 of terpene synthase were searched. A total of 13 potential terpene synthase protein sequences were obtained.

### 1.2 Identification of synthases and genes for citrus fragrance substances in Actaea racemosa

### 1.2.1 Construction of yeast expression vector

Primers were designed to construct the genes to be verified on universal vector pZY900 (using the same method as that in patent CN 202210473488.4).

The process of the verification of ArVS and ArVS-V8 derived from *Actaea racemosa* was demonstrated here. The amino acid sequences of ArVS and ArVS-V8 were as set forth in SEQ ID NO: 1 and SEQ ID NO: 2, respectively:

MEVTRRSANYHPSVWGDKFLEYSSPDHLKNDSDHLKNDAVTEKKHEKLKEEVK
KMLVETVQKPQQQLNLINEIQRLGLSFHFEPEIEAALRQISVTYHEYCCSTNVDDLHN
VALCFRILREHGHKVSCDVFHKFMDSNGKLKGSFLNDAIGLLSLYEATHFRVHGEDLL
EDVLSLTTSRLEYLKSQMKYPLEDEITRALKHPLHKELNRLGARYYISIYEKYDSHNK
LLLEFAKLDFNRMQKMYQHELAELTRWWKDLDFTNKLPFARDRIVEGYFWILGMYF
EPECKDVREFLNRVFALISVVDDTYDVYGTFEEVILFTHAIERWETSALDQLPEYMKII
YQALMDVYSQMEEKLAKKADCPTYRLEFAIETVKALFRSYLEEARWSKERYIPTMEE
YMTVALVSVGYKTILTNSFVGMGDTATREVFEWVFSSPLIVRASDLIARLGDDIGGHE
EEQKKGDAATAVECYMKEHGVTKQEAYAEFQKQIDNAWKDLNQEALRPFPVPMTYI
TRVVHFTRAIHVIYADSDDGYTRPEKKIKGYITSLLVDPIPM (SEQ ID NO: 1).

MSLNVLSTSGSAPTTKSSEVTRRSANYHPSVWGDKFLEYSSPDHLKNDSDHLKN
DAVTEKKHEKLKEEVKKMLVETVQKPQQQLNLINEIQRLGLSFHFEPEIEAALRQISVT
YHEYCCSTNVDDLHNVALCFRILREHGHKVSCDVFHKFMDSNGKLKGSFLNDAIGLL
SLYEATHFRVHGEDLLEDVLSLTTSRLEYLKSQMKYPLEDEITRALKHPLHKELNRLG
ARYYISIYEKYDSHNKLLLEFAKLDFNRMQKMYQHELAELTRWWKDLDFTNKLPFA
RDRIVEGYFWILGMYFEPECKDVREFLNRVFALISVVDDTYDVYGTFEEVILFTHAIER
WETSALDQLPEYMKIIYQALMDVYSQMEEKLAKKADCPTYRLEFAIETVKALFRSYL
EEARWSKERYIPTMEEYMTVALVSVGYKTILTNSFVGMGDTATREVFEWVFSSPLIVR
ASDLIARLGDDIGGHEEEQKKGDAATAVECYMKEHGVTKQEAYAEFQKQIDNAWKD
LNQEALRPFPVPMTYITRVVHFTRAIHVIYADSDDGYTRPEKKIKGYITSLLVDPIPM
(SEQ ID NO: 2).

The genes ArVS and ArVS-V8 derived from *Actaea racemosa* were directly synthesized following codon optimization for *Saccharomyces cerevisiae,* and the nucleotide sequences thereof were as set forth in SEQ ID NO: 4 and 5, respectively.

ATGGAAGTTACCAGAAGAAGTGCCAACTACCACCCATCTGTCTGGGGTGACA
AGTTCTTGGAATACTCTTCTCCAGACCATTTGAAGAACGACTCCGATCACTTGAAG
AACGACGCTGTCACTGAAAAGAAGCACGAAAAGTTGAAGGAAGAAGTCAAAAA
GATGTTGGTCGAAACTGTCCAAAAGCCACAACAACAATTGAACTTAATCAACGAA
ATCCAAAGATTGGGTTTGTCCTTCCACTTCGAACCAGAAATCGAAGCTGCCTTAAG

ACAAATCTCCGTCACATATCACGAATACTGTTGTTCTACCAACGTTGATGACTTGCA
CAACGTCGCTTTGTGTTTCAGAATCTTGAGAGAACACGGTCACAAGGTTTCCTGC
GATGTCTTCCATAAGTTCATGGACTCTAACGGTAAGTTGAAAGGTTCTTTCTTGAA
CGACGCTATCGGTTTATTGAGCTTGTACGAAGCTACTCACTTCCGTGTTCACGGTG
AAGATTTGTTGGAAGATGTATTATCCTTGACTACCTCTAGATTGGAATACCTTAAGT
CTCAGATGAAGTACCCATTAGAAGACGAAATAACCAGAGCTCTCAAGCATCCACTC
CACAAGGAATTGAACAGATTGGGTGCTAGATACTACATTTCCATTTACGAAAAATA
CGATTCCCACAACAAGTTATTGCTGGAATTTGCTAAGTTGGATTTCAACAGAATGC
AAAAAATGTACCAACACGAATTGGCTGAGTTGACTAGATGGTGGAAGGACCTTGA
CTTCACTAATAAGCTACCTTTCGCCAGAGACAGAATCGTTGAAGGTTACTTCTGGA
TTTTGGGTATGTACTTTGAACCAGAATGTAAGGACGTTAGAGAATTTTTGAACAGA
GTTTTTGCTTTAATCTCTGTTGTCGATGATACATACGACGTCTACGGTACTTTCGAA
GAAGTTATTTTATTCACCCACGCTATTGAACGTTGGGAAACTTCCGCCTTGGACCA
ATTGCCAGAATATATGAAGATCATTTACCAAGCTTTGATGGATGTTTACTCCCAAAT
GGAAGAAAAGTTGGCCAAGAAGGCTGACTGTCCAACCTACAGATTGGAATTTGCC
ATTGAAACCGTTAAGGCTTTGTTCAGATCTTACTTGGAAGAAGCCCGTTGGTCAAA
GGAAAGATATATTCCAACCATGGAGGAATATATGACTGTTGCTTTGGTTTCTGTTGG
TTACAAGACTATCTTGACTAACTCTTTCGTTGGTATGGGTGACACTGCTACCAGAG
AAGTTTTCGAATGGGTTTTCTCCTCCCCATTGATTGTCAGAGCTTCTGATTTGATTG
CTAGGTTGGGCGACGACATTGGTGGTCACGAAGAAGAACAAAAGAAGGGTGATG
CCGCTACCGCTGTCGAATGTTACATGAAAGAACATGGTGTCACCAAGCAAGAAGC
TTACGCCGAATTCCAAAAGCAAATTGACAACGCTTGGAAGGACTTAAACCAAGAA
GCCTTAAGACCATTCCCAGTTCCAATGACCTACATCACTCGTGTTGTTCATTTCACC
AGAGCTATTCACGTTATCTACGCTGATAGTGACGATGGTTACACCCGTCCTGAAAA
GAAAATCAAGGGTTACATCACTTCTTTGTTGGTAGACCCAATCCCAATGTGA (SEQ
ID NO: 4).

ATGTCGTTAAATGTGCTATCCACTTCTGGTTCTGCTCCAACTACCAAATCTTCT
GAAGTTACCAGAAGAAGTGCCAACTACCACCCATCTGTCTGGGGTGACAAGTTCT
TGGAATACTCTTCTCCAGACCATTTGAAGAACGACTCCGATCACTTGAAGAACGAC
GCTGTCACTGAAAAGAAGCACGAAAAGTTGAAGGAAGAAGTCAAAAAGATGTTG
GTCGAAACTGTCCAAAAGCCACAACAACAATTGAACTTAATCAACGAAATCCAAA
GATTGGGTTTGTCCTTCCACTTCGAACCAGAAATCGAAGCTGCCTTAAGACAAATC
TCCGTCACATATCACGAATACTGTTGTTCTACCAACGTTGATGACTTGCACAACGTC
GCTTTGTGTTTCAGAATCTTGAGAGAACACGGTCACAAGGTTTCCTGCGATGTCTT

CCATAAGTTCATGGACTCTAACGGTAAGTTGAAAGGTTCTTTCTTGAACGACGCTA
TCGGTTTATTGAGCTTGTACGAAGCTACTCACTTCCGTGTTCACGGTGAAGATTTGT
TGGAAGATGTATTATCCTTGACTACCTCTAGATTGGAATACCTTAAGTCTCAGATGA
AGTACCCATTAGAAGACGAAATAACCAGAGCTCTCAAGCATCCACTCCACAAGGA
ATTGAACAGATTGGGTGCTAGATACTACATTTCCATTTACGAAAAATACGATTCCCA
CAACAAGTTATTGCTGGAATTTGCTAAGTTGGATTTCAACAGAATGCAAAAAATGT
ACCAACACGAATTGGCTGAGTTGACTAGATGGTGGAAGGACCTTGACTTCACTAAT
AAGCTACCTTTCGCCAGAGACAGAATCGTTGAAGGTTACTTCTGGATTTTGGGTAT
GTACTTTGAACCAGAATGTAAGGACGTTAGAGAATTTTTGAACAGAGTTTTTGCTT
TAATCTCTGTTGTCGATGATACATACGACGTCTACGGTACTTTCGAAGAAGTTATTTT
ATTCACCCACGCTATTGAACGTTGGGAAACTTCCGCCTTGGACCAATTGCCAGAAT
ATATGAAGATCATTTACCAAGCTTTGATGGATGTTTACTCCCAAATGGAAGAAAAG
TTGGCCAAGAAGGCTGACTGTCCAACCTACAGATTGGAATTTGCCATTGAAACCGT
TAAGGCTTTGTTCAGATCTTACTTGGAAGAAGCCCGTTGGTCAAAGGAAAGATATA
TTCCAACCATGGAGGAATATATGACTGTTGCTTTGGTTTCTGTTGGTTACAAGACTA
TCTTGACTAACTCTTTCGTTGGTATGGGTGACACTGCTACCAGAGAAGTTTTCGAA
TGGGTTTTCTCCTCCCCATTGATTGTCAGAGCTTCTGATTTGATTGCTAGGTTGGGC
GACGACATTGGTGGTCACGAAGAAGAACAAAAGAAGGGTGATGCCGCTACCGCT
GTCGAATGTTACATGAAAGAACATGGTGTCACCAAGCAAGAAGCTTACGCCGAAT
TCCAAAAGCAAATTGACAACGCTTGGAAGGACTTAAACCAAGAAGCCTTAAGACC
ATTCCCAGTTCCAATGACCTACATCACTCGTGTTGTTCATTTCACCAGAGCTATTCA
CGTTATCTACGCTGATAGTGACGATGGTTACACCCGTCCTGAAAAGAAAATCAAGG
GTTACATCACTTCTTTGTTGGTAGACCCAATCCCAATGTGA (SEQ ID NO: 5).

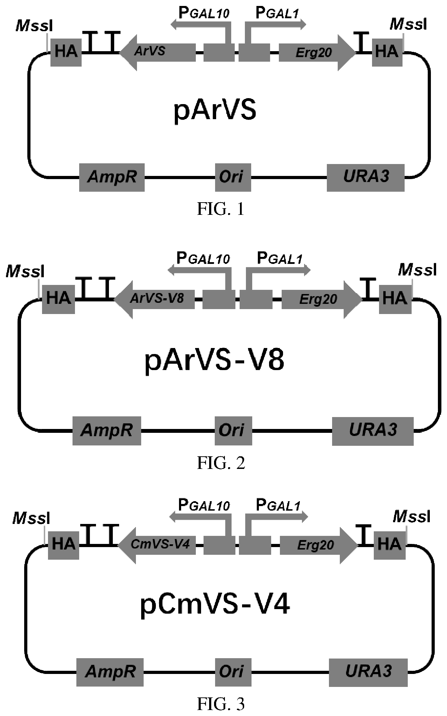

Primer pairs ArVS-1F/R and ArVS-V8-1F/R were designed for specific genes; using synthesized genes as templates, ArVS and ArVS-V8 gene fragments were obtained by amplification using PCR with GXL high-fidelity enzyme from Vazyme; following gel recovery using a gel recovery kit from TIANGEN, the fragments were ligated into BsaI-digested yeast expression vector pZY900 using a homologous recombination kit from Yeasen by a homologous recombination method; and after the resulting constructs were confirmed error-free by sequencing, yeast expression vectors containing the ArVS and ArVS-V8 gene fragments were obtained and named pArVS and pArVS-V8. The plasmid maps are as shown in FIGS. 1 and 2, respectively. They were obtained by separately replacing the gene lacZ in pZY900 with the genes ArVS and ArVS-V8.

The primer sequences of ArVS-1F/R are as shown in Table 1 below:

**Table 1**

| Plasmid | Primer | Sequence (from 5' to 3') |
|---|---|---|
| pArVS | ArVS-1F | CTAATTACATGATATCGACAAAGGAAAAGGGGCCTGTTC ACATTGGGATTGGGTCTAC (SEQ ID NO: 7) |
| | ArVS-1R | gtaagaatttttgaaaattcaatataaATGGAAGTTACCAGAAGAAGTG CC (SEQ ID NO: 8) |

The primer sequences of ArVS-V8-1F/R are as shown in Table 1 below:

**Table 2**

| Plasmid | Primer | Sequence (from 5' to 3') |
|---|---|---|
| pArVS-V 8 | ArVS-V8 -1F | CTAATTACATGATATCGACAAAGGAAAAGGGGCCTGTTC ACATTGGGATTGGGTCTAC (SEQ ID NO: 9) |
| | ArVS-V8 -1R | gtaagaatttttgaaaattcaatataaATGTCGTTAAATGTGCTATCCACT TC (SEQ ID NO: 10) |

### 1.2.2 Strain construction

Plasmids pArVS, pArVS-V8, and pZY900 were separately introduced into strain JCR27 (for the construction of the yeast strain JCR27, see the literature Siemon, Thomas et al. "Semisynthesis of Plant-Derived Englerin A Enabled by Microbe Engineering of Guaia-6,10(14)-diene as Building Block." Journal of the American Chemical Society vol. 142,6 (2020): 2760-2765. doi:10.1021/jacs.9b12940) by a lithium acetate method (LI, Xiaowei, Engineering Acetyl-CoA Pathway to Construct Efficient Synthesis Platform of Saccharomyces cerevisiae [D]. Wuhan University, 2015. 2.3.14 LiAc transformation of *Saccharomyces cerevisiae*). The engineered strains were named JArVS, JArVS-V8, and S900.

### 1.2.3 Strain fermentation and product identification

The strains JArVS, JArVS-V8, and S900 were inoculated into SC-Ura liquid medium (LI, Xiaowei, Engineering Acetyl-CoA Pathway to Construct Efficient Synthesis Platform of Saccharomyces cerevisiae [D]. Wuhan University, 2015, Uracil-deficient medium) and cultured on a shaker overnight at 30°C and 200 rpm. The next day, the strains were transferred into 45 milliliters of YPDHG liquid medium (20 g/L peptone, 10 g/L yeast powder, 10 g/L glucose, and 10 g/L galactose) at an inoculum size of 1%, and 5 milliliters of isopropyl myristate was added, followed by culturing on a shaker at 30°C and 200 rpm for 72 hours, and an oil layer was collected, diluted with n-hexane to an appropriate concentration, and detected for products using GC-MS.

GCMS detection conditions were: Thermo Fisher Scientific equipped with AS 3000 autosampler, TRACE GC ULTRA gas chromatography with split/splitless injector, and TSQ QUANTUM XLS MS equipped with a triple quadrupole detector. The chromatographic column was TR-5MS column (30 m × 0.25 mm × 0.25 µm). The carrier gas was high-purity helium gas at a flow rate of 1 mL/min. Acetone was used as a needle washing liquid. The injection volume was 1 µL and the split ratio was 50. The temperature at an injection port was 240°C, and the temperature of an ion transport tube was 270°C. Detection program: the initial column temperature was 50°C and maintained for 1 min; the temperature was raised to 280°C at 15°C/min; and the temperature was raised to 300°C at 20°C/min and maintained for 2 min.

The results showed that no valencene was detected in the oil phase corresponding to blank control strain S900, whereas valencene could be detected in both the oil phases corresponding to the strains JArVS and JArVS-V8. The valencene yields were 110 mg/L and 230 mg/L, respectively.

### Example 2. Functional identification of valencene synthase derived from Citrus maxima (Pomelo, pummelo)

### 2.1 Mining of genes involved in synthesis of citrus fragrance substances in Citrus maxima (Pomelo, pummelo)

Referring to published protein sequence information about *Citrus maxima* (http://citrus.hzau.edu.cn/data/Genome_info/HWB.v1.0/HWB.v1.0.protein.fa), protein sequences containing both Pfam domains PF01397 and PF03936 of terpene synthase were searched. A total of 91 potential terpene synthase protein sequences were obtained.

### 2.2 Identification of synthases and genes of citrus fragrance substances in Citrus maxima (Pomelo, pummelo)

### 2.2.1 Construction of yeast expression vector

Primers were designed to construct the genes to be verified on universal vector pZY900 (see patent CN 202210473488.4).

The process of the verification of CmVS-V4 derived from *Citrus maxima* (*Pomelo, pummelo*) was demonstrated here. The amino acid sequence of CmVS-V4 was:

MSSGETFRPTADFHPSLWRNHFLKGASDFKTVDHTATQERHEALKEEVRRMITD
AEDKPVQKLRLIDEVQRLGVAYHFEKEIEDAIQKLCPNYIHSNSPDLHTVSLHFRLLRQ
QGIKISCDVFEKFKDDEGRFKSSLINDVQGMLSLYEAAYMAVRGEHILDEAIAFTTTHL
KSLVAQDHVTPKLAEQINHALYRPLRKTLPRLEARYFMSMINSTSDHLYNKTLLNFAK
LDFNILLELHKEELNELTKWWKELDFTTKLPYARDRLVELYFWDLGTYFEPQYAFGR
KIMTQLNYILSIIDDTYDAYGTLEELSLFTEAVQRWNIEAVDTLPEYMKLIYRTLLDAF
NEIEEDMAKQGRSHCVRYAKEANQKVIGAYFVQAKWFSEGYVPTIEEYMPIALTSCA
YTFVITNSFLGMGDFATKEVFEWISDNPKVVKAASVICRLLDDMQGHEFEQKRGHVA

SAIECYTKQHGVSKEEAIKMFEEEVANAWKDINEELMMKPTVVARPLLGTILNLARAI
DFIYKEDDGYTHSYLIKDQIASVLGDHVPF (SEQ ID NO: 3).

The gene CmVS-V4 derived from *Citrus maxima* (*Pomelo, pummelo*) was directly synthesized following codon optimization for *Saccharomyces cerevisiae,* and the sequence was as set forth in SEQ ID NO: 6:

ATGTCTTCTGGTGAAACTTTCAGACCAACTGCTGATTTCCACCCATCTTTGTGG
AGAAACCACTTCTTGAAGGGTGCCAGCGACTTCAAAACTGTTGACCACACTGCCA
CCCAAGAAAGACACGAAGCTTTGAAAGAAGTCAGACGTATGATTACTGACGC
TGAAGACAAGCCAGTTCAAAAGCTAAGATTGATAGACGAAGTCCAAAGATTAGGT
GTTGCTTACCATTTTGAAAAGGAAATCGAAGATGCTATCCAAAAGTTATGTCCAAA
CTACATTCACTCCAACTCTCCAGACTTGCACACTGTCAGTCTTCATTTCAGGTTGTT
GAGACAACAAGGTATCAAGATCTCTTGTGATGTCTTCGAAAAGTTCAAGGATGAC
GAAGGTAGATTCAAGTCCTCACTGATTAACGATGTTCAAGGTATGTTGTCCTTGTAC
GAAGCCGCCTACATGGCTGTTAGAGGTGAACACATTTTGGATGAAGCCATCGCTTT
CACCACCACTCACTTGAAGTCTCTAGTGGCTCAGGATCATGTTACCCCAAAGTTGG
CTGAACAAATCAATCACGCTCTCTACCGTCCTTTAAGAAAGACTTTGCCAAGATTG
GAAGCTCGTTACTTCATGTCCATGATCAACTCCACTTCTGACCATTTGTACAACAAG
ACTCTATTGAACTTCGCTAAATTGGACTTCAACATATTGTTGGAATTGCACAAGGA
AGAATTGAATGAACTGACTAAATGGTGGAAGGAATTAGATTTTACTACAAAGTTGC
CATACGCTAGAGACAGATTGGTTGAATTATATTTCTGGGACTTAGGCACATACTTCG
AACCACAATACGCCTTCGGTAGAAAGATTATGACTCAATTGAACTACATCTTGAGC
ATCATTGACGACACTTACGACGCTTACGGTACTTTAGAAGAGTTGTCCTTGTTCACT
GAAGCCGTCCAAAGATGGAACATTGAAGCAGTCGATACCTTGCCAGAATATATGAA
ATTGATCTACAGAACCTTGTTAGATGCTTTTAACGAAATTGAAGAAGACATGGCCA
AACAAGGTCGTTCCCATTGTGTCAGATACGCTAAGGAAGCTAACCAAAAGGTCATT
GGTGCTTACTTCGTTCAAGCTAAGTGGTTCTCTGAAGGTTACGTTCCAACCATTGA
AGAATATATGCCAATCGCCCTCACCTCTTGCGCTTACACTTTTGTCATCACCAACTC
TTTCTTGGGTATGGGTGACTTTGCTACCAAGGAAGTCTTTGAATGGATTTCTGACA
ACCCAAAGGTTGTCAAGGCTGCTTCTGTTATCTGTAGATTGTTGGATGATATGCAAG
GTCACGAATTCGAGCAAAAGAGAGGTCACGTTGCTTCTGCTATCGAATGTTACACC
AAGCAACACGGTGTCTCCAAGGAAGAAGCTATCAAGATGTTCGAAGAAGAAGTT
GCCAACGCTTGGAAGGACATTAACGAAGAACTGATGATGAAGCCTACCGTTGTTG
CTAGACCATTGTTGGGTACCATCTTGAACTTGGCCAGAGCTATTGATTTCATCTACA

AGGAAGATGATGGTTACACCCACTCTTACTTGATTAAGGACCAAATTGCTTCCGTT
TTGGGTGACCACGTCCCATTCTGA.

Primer pair CmVS-V4-1F/R was designed for a specific gene; using the synthesized gene as a template, a CmVS-V4 gene fragment was obtained by amplification using PCR with GXL high-fidelity enzyme from Vazyme; following gel recovery using a gel recovery kit from TIANGEN, the fragment was ligated into BsaI-digested yeast expression vector pZY900 using a homologous recombination kit from Yeasen by a homologous recombination method; and after being confirmed by sequencing without errors, a yeast expression vector containing CmVS-V4 was obtained and named pCmVS-V4. A schematic diagram of the construction of plasmid pCmVS-V4 is as shown in FIG. 3. The plasmid was obtained by replacing the gene lacZ in pZY900 with the gene CmVS-V4.

The primer sequences of CmVS-V4-1F/R are as shown in Table 3 below.

**Table 3**

| Plasmid | Primer | Sequence (from 5' to 3') |
|---|---|---|
| pCmVS-V4 | CmVS-V4-1F | CATGATATCGACAAAGGAAAAGGGGCCTGTTCAGAATG GGACGTGGTCAC (SEQ ID NO: 11) |
| | CmVS-V4-1R | agtaagaatttttgaaaattcaatataaATGTCTTCTGGTGAAACTTTCAG AC (SEQ ID NO: 12) |

### 2.2.2 Strain construction

Plasmid pCmVS-V4 was introduced into strain JCR27 by a lithium acetate method. The engineered strain was named JCmVS-V4.

### 2.2.3 Strain fermentation and product identification

The strain JCmVS-V4 was subjected to fermentation and product extraction according to Example 1.2.3.

The results showed that the strain JCmVS-V4 had a valencene yield of 215 mg/L.

### Comparative Example. Expression of existing valencene synthase genes

### 1.1 Construction of plasmids containing valencene synthase genes derived from Vitis vinifera (Grape), Citrus sinensis (Sweet orange) (Citrus aurantium var. sinensis), Solanum habrochaites (Wild tomato) (Lycopersicon hirsutum), Solanum lycopersicum (Tomato) (Lycopersicon esculentum), Switchgrass (Panicum virgatum L.), Callitropsis nootkatensis, Eryngium glaciale, and Alpinia oxyphylla Miq.

The valencene synthases derived from various sources used in this comparative example are valencene synthases that are currently extensively studied in the art and widely accepted for relatively high yields.

Primers were designed to construct the above codon-optimized valencene synthase encoding gene on the universal vector pZY900.

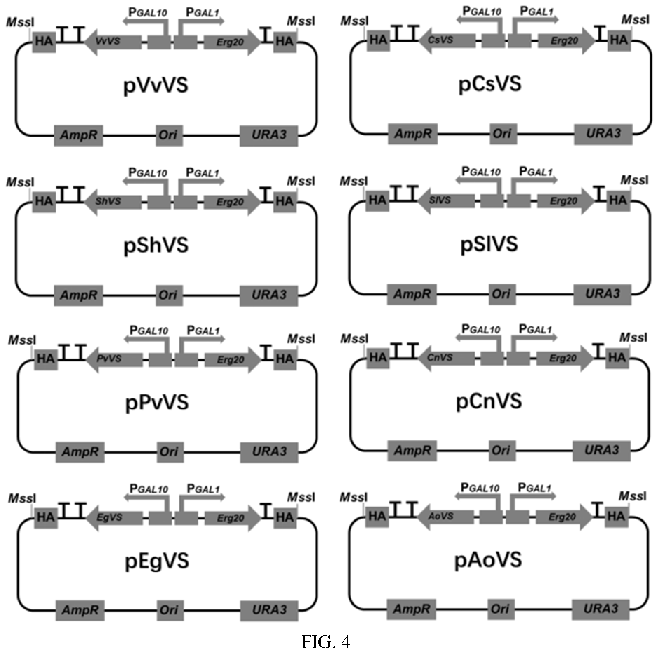

The sequences of primers designed for each gene are as shown in the table below. Using the above synthesized genes as templates, yeast expression vectors containing the genes VvVS, CsVS, ShVS, SlVS, PvVS, CnVS, EgVS, and AoVS were separately constructed by using the same method as in section 2.1 in Example 1 and named pVvVS, pCsVS, pShVS, pSlVS, pPvVS, pCnVS, pEgVS, and pAoVS. The schematic diagram of the construction of the plasmids pVvVS, pCsVS, pShVS, pSlVS, pPvVS, pCnVS, pEgVS, and pAoVS are shown in FIG. 4. They were obtained by replacing the gene lacZ in pZY900 with the genes VvVS, CsVS, ShVS, SlVS, PvVS, CnVS, EgVS, and AoVS, respectively.

**Table 4**

| Plasmid | Primer | Sequence (from 5' to 3') |
|---|---|---|
| pVvVS | VvVS-1F | CGACAAAGGAAAAGGGGCCTGTTCAAATGACAGCGTTGA TGAAAAC (SEQ ID NO: 13) |
| | VvVS-1R | gtaagaatttttgaaaattcaatataaATGTCCACTCAAGTTTCAGCCAG (SEQ ID NO: 14) |
| pCsVS | CsVS-1 F | TATCGACAAAGGAAAAGGGGCCTGTTCAGAATGGGACGT GGTCAC (SEQ ID NO: 15) |
| | CsVS-1 R | aagaatttttgaaaattcaatataaATGTCTTCTGGTGAAACCTTC (SEQ ID NO: 16) |
| pShVS | ShVS-1 F | TATCGACAAAGGAAAAGGGGCCTGTTCAGATAATTGGGTC AACTAAAATATC (SEQ ID NO: 17) |
| | ShVS-1 R | aagaatttttgaaaattcaatataaATGGAATTATGCACTCAAACCGTC (SEQ ID NO: 18) |
| SlVS | SlVS-1 F | ACAAAGGAAAAGGGGCCTGTTCAAATGATAGGATCGACA AGAATG (SEQ ID NO: 19) |
| | SlVS-1 R | tttttgaaaattcaatataaATGGAATTGTGTACTCAAACTGTTG (SEQ ID NO: 20) |
| pPvVS | PvVS-1 F | ACAAAGGAAAAGGGGCCTGTTCAGATGGCTGGACCATTTA GAAAC (SEQ ID NO: 21) |
| | PvVS-1 R | tttttgaaaattcaatataaATGGCCGCCTCCGAAGCCGCCTC (SEQ ID NO: 22) |
| pCnVS | CnVS-1F | CATGATATCGACAAAGGAAAAGGGGCCTGTTTAAGGAATA ATAGGTTCAACAAAGAACT (SEQ ID NO: 23) |
| | CnVS-1R | tccaaaaaaaaagtaagaatttttgaaaattcaatataaATGGCTGAAATGTTCAA CGG (SEQ ID NO: 24) |
| pEgVS | EgVS-1 F | TACATGATATCGACAAAGGAAAAGGGGCCTGTTTATAATGG AATTGGATCAACCAACAA (SEQ ID NO: 25) |
| | EgVS-1 R | aaaagtaagaatttttgaaaattcaatataaATGTCATTGAATGTTTTATCTAC ATCAG (SEQ ID NO: 26) |
| pAoVS | AoVS-1F | ACAAAGGAAAAGGGGCCTGTTTAAATTGGGATAGATTCAA CCAAAACC (SEQ ID NO: 27) |
| | AoVS-1R | aatttttgaaaattcaatataaATGGAAAAGCAATCTGTTACATTG (SEQ ID NO: 28) |

The amino acid sequence of the valencene synthase derived from *Vitis vinifera (Grape)* was:

MSTQVSASSLAQIPQPKNRPVANFHPNIWGDQFITYTPEDKVTRACKEEQIEDLK
KEVKRKLTAAAVANPSQLLNFIDAVQRLGVAYHFEQEIEEALQHICNSFHDCNDMDGD
LYNIALGFRLLRQQGYTISCDIFNKFTDERGRFKEALISDVRGMLGLYEAAHLRVHGE
DILAKALAFTTTHLKAMVESLGYHLAEQVAHALNRPIRKGLERLEARWYISVYQDEA
FHDKTLLELAKLDFNLVQSLHKEELSNLARWWKELDFATKLPFARDRLVEGYFWMH
GVYFEPQYLRGRRILTKVIAMTSILDDIHDAYGTPEELKLFIEAIERWDINSINQLPEYM
KLCYVALLDVYKEIEEEMEKEGNQYRVHYAKEVMKNQVRAYFAEAKWLHEEHVPA
FEEYMRVALASSGYCLLATTSFVGMGEIATKEAFDWVTSDPKIMSSSNFITRLMDDIKS
HKFEQKRGHVASAVECYMKQYGVSEEQVYSEFQKQIENAWLDINQECLKPTAVSMPL
LARLLNLTRTMDVIYKEQDSYTHVGKVMRDNIASVFINAVI (SEQ ID NO: 29).

The nucleotide sequence of VvVS after codon optimization for *Saccharomyces cerevisiae* was:

ATGTCCACTCAAGTTTCAGCCAGCTCCTTAGCTCAAATTCCACAACCAAAGAA
CAGACCTGTTGCTAACTTCCATCCAAACATCTGGGGTGACCAATTCATCACTTACA
CTCCAGAAGACAAGGTTACTCGTGCTTGTAAGGAAGAACAAATTGAAGATTTGAA
GAAGGAAGTCAAGAGAAAGTTGACTGCTGCTGCCGTCGCCAACCCATCTCAATTA
TTGAACTTTATTGACGCTGTCCAAAGATTGGGTGTCGCCTACCACTTCGAACAAGA
AATTGAAGAAGCATTGCAACACATCTGTAATTCTTTTCATGACTGTAACGACATGG
ACGGTGACTTGTACAACATTGCTCTCGGTTTCAGACTTTTAAGACAACAAGGTTAC
ACCATTTCTTGTGATATCTTCAATAAATTCACTGATGAACGTGGTAGATTCAAGGAG
GCTTTGATCTCTGACGTTAGAGGTATGTTGGGTTTGTACGAAGCTGCTCATTTGAG
AGTCCACGGTGAAGATATACTGGCCAAGGCTTTGGCGTTTACCACCACTCACTTGA
AGGCTATGGTTGAATCTTTGGGCTACCACTTGGCTGAACAAGTTGCTCACGCTTTG
AACAGACCAATCAGAAAGGGTTTGGAAAGATTGGAAGCCAGATGGTACATCTCTG
TCTACCAAGACGAAGCTTTCCACGACAAGACTTTGTTGGAATTGGCTAAGTTGGAT
TTCAACTTGGTTCAATCCTTGCATAAAGAAGAATTGTCCAATTTAGCCAGATGGTG
GAAGGAGCTGGATTTTGCTACCAAGTTGCCATTCGCTCGTGACAGATTAGTCGAAG
GTTACTTCTGGATGCACGGTGTTTACTTCGAACCACAATACTTGAGAGGTAGAAGA

ATTTTGACAAAGGTCATCGCTATGACTTCTATCTTGGATGACATTCACGATGCTTAC
GGTACTCCTGAAGAACTAAAATTATTCATTGAAGCCATTGAAAGATGGGATATTAAC
TCCATCAACCAGTTGCCAGAATATATGAAATTGTGTTACGTTGCTTTGTTGGATGTAT
ACAAGGAAATCGAAGAAGAAATGGAAAAGGAAGGTAACCAATACAGAGTTCACT
ACGCTAAGGAAGTAATGAAGAACCAAGTCAGAGCTTATTTCGCTGAAGCTAAGTG
GTTGCACGAAGAACACGTTCCAGCTTTTGAAGAATACATGAGAGTTGCTTTGGCTT
CCTCTGGTTACTGTTTGCTTGCTACTACCTCTTTCGTTGGTATGGGTGAAATTGCCA
CCAAGGAAGCCTTCGACTGGGTCACCTCTGACCCAAAGATCATGTCTTCTTCTAAC
TTCATCACCAGATTGATGGATGATATCAAGTCTCACAAGTTCGAACAAAAGCGTGG
TCACGTTGCCTCAGCTGTTGAGTGCTACATGAAGCAATATGGTGTTTCCGAAGAAC
AAGTCTACTCTGAATTCCAAAAACAAATCGAAAACGCTTGGTTAGACATTAACCAA
GAATGTTTGAAGCCAACCGCTGTCTCCATGCCATTGTTGGCTCGTCTATTAAACTTG
ACCAGAACCATGGACGTTATCTACAAAGAACAAGATTCTTACACTCACGTTGGTAA
GGTCATGAGAGACAACATCGCCTCCGTTTTCATCAACGCTGTCATTTGA (SEQ ID
NO: 30).

The amino acid sequence of the valencene synthase derived from *Citrus sinensis (Sweet orange) (Citrus aurantium var. sinensis)* was:

MSSGETFRPTADFHPSLWRNHFLKGASDFKTVDHTATQERHEALKEEVRRMITD
AEDKPVQKLRLIDEVQRLGVAYHFEKEIGDAIQKLCPIYIDSNRADLHTVSLHFRLLRQ
QGIKISCDVFEKFKDDEGRFKSSLINDVQGMLSLYEAAYMAVRGEHILDEAIAFTTTHL
KSLVAQDHVTPKLAEQINHALYRPLRKTLPRLEARYFMSMINSTSDHLCNKTLLNFAK
LDFNILLELHKEELNELTKWWKDLDFTTKLPYARDRLVELYFWDLGTYFEPQYAFGR
KIMTQLNYILSIIDDTYDAYGTLEELSLFTEAVQRWNIEAVDMLPEYMKLIYRTLLDAF
NEIEEDMAKQGRSHCVRYAKEENQKVIGAYSVQAKWFSEGYVPTIEEYMPIALTSCA
YTFVITNSFLGMGDFATKEVFEWISNNPKVVKAASVICRLMDDMQGHEFEQKRGHVA
SAIECYTKQHGVSKEEAIKMFEEEVANAWKDINEELMMKPTVVARPLLGTILNLARAI
DFIYKEDDGYTHSYLIKDQIASVLGDHVPF (SEQ ID NO: 31).

The nucleotide sequence of CsVS after codon optimization for *Saccharomyces cerevisiae* was:

ATGTCTTCTGGTGAAACCTTCAGGCCAACTGCCGACTTCCACCCATCCTTGTG
GAGAAACCATTTTTTGAAGGGTGCCTCTGATTTCAAGACTGTCGACCACACTGCTA
CCCAAGAAAGACACGAAGCTTTGAAGGAAGAAGTCAGAAGAATGATCACTGACG
CTGAGGACAAGCCAGTCCAAAAGTTGCGTCTGATTGACGAAGTTCAAAGATTGGG
TGTCGCTTACCACTTCGAAAAGGAAATTGGTGATGCTATTCAAAAACTATGTCCAA

TCTACATTGACTCCAACAGAGCTGACTTGCACACCGTTTCCTTACACTTCAGATTAT
TGAGACAACAAGGTATCAAGATTTCTTGTGATGTTTTCGAAAAGTTCAAGGATGAT
GAAGGTAGATTCAAGTCTAGTTTGATTAACGATGTCCAAGGTATGTTGTCCTTGTAC
GAAGCCGCTTACATGGCTGTTAGAGGTGAACACATTTTGGATGAAGCTATCGCTTT
TACCACAACCCACTTGAAGTCCCTTGTTGCTCAAGATCATGTTACTCCAAAGTTAG
CCGAACAAATCAACCACGCTTTATACAGACCATTGAGAAAGACCCTACCAAGATTG
GAAGCCAGATACTTCATGTCCATGATTAATAGCACTTCCGACCATTTGTGTAACAAG
ACCTTACTGAACTTTGCTAAATTGGACTTCAACATCTTATTGGAATTGCACAAGGA
AGAATTAAACGAATTGACTAAGTGGTGGAAGGATTTGGACTTCACAACTAAGTTG
CCATACGCTAGAGACAGATTGGTTGAATTGTATTTCTGGGATTTGGGTACTTACTTC
GAACCACAATACGCCTTCGGTAGAAAGATCATGACTCAATTGAACTACATCTTGTC
TATCATTGATGACACCTACGATGCCTACGGTACTTTGGAAGAACTATCATTATTCAC
TGAAGCTGTTCAAAGATGGAACATCGAAGCTGTCGATATGTTACCAGAATATATGA
AATTGATTTACAGAACCTTGTTGGATGCTTTCAACGAAATCGAAGAAGACATGGCC
AAACAAGGACGTTCTCATTGTGTTCGTTATGCTAAAGAAGAAAACCAAAAGGTCA
TTGGTGCTTACTCTGTCCAAGCTAAATGGTTCTCTGAAGGTTACGTTCCTACCATCG
AAGAATACATGCCAATTGCTTTGACTTCCTGCGCTTACACTTTCGTTATCACCAACT
CTTTCTTGGGTATGGGTGATTTTGCTACTAAGGAAGTCTTTGAATGGATCTCTAACA
ACCCAAAGGTTGTTAAGGCTGCTTCCGTTATCTGTAGATTGATGGACGACATGCAA
GGTCACGAATTCGAACAAAAGAGAGGTCATGTTGCTTCTGCTATTGAATGTTACAC
CAAGCAACACGGTGTTTCTAAGGAAGAAGCTATCAAGATGTTCGAAGAGGAAGTC
GCCAACGCTTGGAAGGACATCAATGAAGAATTGATGATGAAACCTACCGTCGTAG
CCCGTCCATTGTTGGGCACCATTTTGAACTTGGCTAGAGCTATTGACTTCATCTACA
AGGAAGATGACGGTTACACTCACTCTTACTTAATCAAGGACCAAATTGCGTCCGTT
TTGGGTGACCACGTCCCATTCTGA (SEQ ID NO: 32).

The amino acid sequence of the valencene synthase derived from *Solanum habrochaites* (Wild tomato) *(Lycopersicon hirsutum)* was:

MELCTQTVAADHEVIITRRSGSHHPTLWGDHFLAYADLRGANEGEEKQNEDLKE
EVRKMLVMAPSNSLEKLELINTIQCLGLAYHFQSEIDESLSYMYTHYEEYSIGDLHAIA
LCFRLLRQQGYYVSCDAFKKFTNDQGNFKEELVKDVEGMLSLYEAAQFRVHGEQILD
EALNFTITKLKQILPKLSNSQLAQQITNALKFPIKDGIVRVETRKYISFYQQNQNHNQV
LLNFAKLDFNILQTLHKKELSDMTRWWKKMELVNTLPYARDRLVECYFWCLGTYFE
PQYSVARKMLTKISFFISIIDDTYDIYGKLDELTLFTQAIERWNIDASEQLPLYMKIIYRD
LLDVYDEIEKELANENKSFLVNYSINEMKKVVRGYFQEAKWYYGKKVPKMEQYMK

NAISTSAYILLTTTSWLAMGNVATKDVFDWVATEPKLVVASCHIIRLLNDLVSHEEEQK
RGNAASAVECYMNEYSVTQEEAHVKIRDIIENYWKDLNEEYFKVDMIIIPRVLLMCII
NLTRVAEFIYKDEDAYTFSKNNLKDVISDILVDPII (SEQ ID NO: 33).

The nucleotide sequence after codon optimization for *Saccharomyces cerevisiae* was:

ATGGAATTATGCACTCAAACCGTCGCTGCCGACCACGAAGTTATCATTACCAG
AAGATCTGGTTCCCATCATCCAACTTTGTGGGGTGACCATTTCCTGGCTTACGCCGA
TTTGAGAGGTGCTAACGAAGGTGAAGAAAAGCAAAACGAAGATTTGAAGGAAGA
AGTCAGAAAGATGTTGGTTATGGCTCCATCTAACTCTTTGGAAAAGTTGGAATTGA
TCAACACTATCCAATGTTTGGGCTTGGCTTACCACTTCCAATCTGAAATCGACGAAT
CCTTGAGCTACATGTACACTCACTACGAGGAATACTCTATTGGTGACCTTCACGCTA
TCGCTTTGTGTTTCCGTTTGTTGAGACAACAAGGTTACTACGTTTCTTGTGATGCTT
TCAAAAAATTCACCAACGACCAAGGTAACTTCAAGGAAGAATTGGTTAAGGATGT
TGAAGGTATGTTGTCTTTGTACGAAGCTGCCCAATTCAGAGTTCACGGTGAACAAA
TTTTGGACGAAGCTTTGAATTTCACTATTACAAAGTTGAAGCAAATCTTGCCAAAG
TTGTCCAACTCTCAATTAGCTCAACAAATCACTAACGCCCTAAAGTTCCCAATCAA
GGACGGTATTGTCCGTGTTGAAACCAGAAAGTACATCTCATTCTACCAACAGAACC
AAAACCACAACCAAGTTTTATTGAACTTTGCTAAGTTGGATTTCAACATTTTGCAA
ACTTTACACAAGAAGGAGTTGTCTGACATGACTAGATGGTGGAAAAAGATGGAAC
TCGTTAATACGTTGCCATACGCTAGAGACAGATTGGTTGAATGTTACTTCTGGTGTT
TGGGTACTTACTTCGAACCACAATACTCCGTTGCTCGTAAAATGCTGACCAAGATT
TCTTTCTTCATCTCTATCATTGATGACACCTACGACATTTACGGTAAGTTGGATGAAT
TGACCTTGTTTACTCAAGCTATTGAAAGATGGAACATTGATGCTTCCGAACAATTG
CCTTTATACATGAAAATCATCTACAGAGACTTGTTAGATGTCTACGATGAAATTGAA
AAGGAATTGGCTAACGAAAACAAGTCCTTTTTGGTCAATTACTCTATCAACGAAAT
GAAGAAGGTCGTTCGTGGTTACTTTCAAGAAGCCAAGTGGTACTATGGTAAGAAG
GTACCAAAGATGGAACAATATATGAAGAACGCCATTAGTACTTCCGCCTACATTCTT
TTGACCACTACTTCTTGGTTAGCTATGGGTAACGTTGCCACCAAGGACGTCTTCGA
CTGGGTTGCTACTGAACCAAAATTGGTTGTCGCCTCCTGTCACATCATTAGATTGTT
GAACGATTTAGTCTCTCACGAAGAAGAACAAAAGAGAGGTAACGCCGCTTCTGCT
GTCGAATGTTACATGAACGAATACTCCGTTACCCAAGAAGAAGCTCACGTCAAGAT
CAGAGATATCATCGAAAACTACTGGAAGGACCTGAATGAGGAATATTTCAAGGTCG
ATATGATTATTATTCCAAGAGTCTTGTTGATGTGTATCATCAACTTGACCAGAGTTGC
TGAATTCATCTACAAGGACGAAGACGCTTACACCTTCTCTAAGAACAACCTAAAAG
ACGTCATCTCAGATATTTTAGTTGACCCAATTATCTGA (SEQ ID NO: 34).

The amino acid sequence of the valencene synthase derived from *Solanum lycopersicum (Tomato) (Lycopersicon esculentum)* was:

MELCTQTVAADHEVIITRRSGSHHPTLWGDHFLAYADLRGANEGEEKQNEDLKE
EVRKMLVMAPSKSLEKLELINTIQCLGLGYHFQSEIDESLSYMYTHYEEYSIGDLHAIA
LCFRLLRQQGYYVSCDAFKKFTNDQGNFKEELVKDVEGMLSLYEAAQFRVHGEQILD
EALNFTIAQLKQILPKLSNSQLAQQITNALKYPIKDGIVRVETRKYISFYQQNQNHNEV
LLNFAKLDFNILQTLHKKELSDMTRWWKKMELVNTLPYARDRLVECYFWCLGTYFE
PQYSVARKMLTKISFYISIIDDTYDIYGKLDELTLFTQAIERWNIDASEQLPLYMKIIYRD
LLDVYDEIEKELANENKSFLVNYSINEMKKVVRGYFQEAKWYYGKKVPTMEQYMK
NGISTSAYILLTTTSWLAMGNVATKDAFDWVATEPPIVVASCYIIRLLNDLVSHEEEQK
RGNAASAVECYMNEYSVTKEEAHIKIRDIIENYWKDLNEEYFKVDMIIIPRVLLMCIIN
LTRVAEFIYKDEDAYTFSKNNLKDVISDILVDPII (SEQ ID NO: 35).

The nucleotide sequence after codon optimization for *Saccharomyces cerevisiae* was:

ATGGAATTGTGTACTCAAACTGTTGCTGCTGATCACGAAGTTATAATCACCAG
AAGATCTGGCTCTCATCACCCAACTTTGTGGGGTGACCATTTCTTGGCTTACGCTG
ACCTCCGTGGTGCTAACGAAGGTGAAGAAAAGCAAAACGAGGATCTTAAAGAAG
AAGTCAGAAAGATGTTGGTTATGGCTCCATCCAAGTCCCTCGAAAAATTGGAATTG
ATTAACACCATCCAATGTTTGGGTTTGGGTTACCACTTCCAATCTGAAATCGACGAA
TCTTTGTCTTACATGTACACTCATTACGAAGAATACTCCATTGGTGACTTGCACGCT
ATTGCTCTATGTTTCAGATTGTTGAGACAACAAGGTTACTACGTTAGCTGCGATGCT
TTCAAGAAGTTCACCAACGACCAAGGTAACTTCAAGGAAGAATTGGTCAAGGAC
GTTGAAGGTATGTTATCTTTGTACGAAGCTGCTCAATTCAGAGTCCACGGTGAACA
AATTTTGGACGAAGCTTTGAACTTCACTATCGCTCAATTGAAACAAATTTTGCCAA
AGTTGTCCAACTCTCAATTGGCCCAACAAATCACTAACGCCTTGAAGTACCCAATC
AAGGATGGTATTGTCCGTGTGGAAACCAGAAAGTACATTTCTTTCTACCAACAAAA
CCAAAATCACAACGAAGTCCTGTTGAACTTTGCTAAGTTAGACTTCAACATCTTGC
AAACCTTGCACAAGAAAGAATTGTCCGACATGACTAGATGGTGGAAGAAGATGGA
ATTGGTCAACACTTTGCCATACGCGAGAGACAGATTAGTTGAATGTTATTTCTGGTG
TTTGGGTACTTACTTCGAACCACAATACTCCGTCGCTAGAAAGATGTTGACCAAGA
TCTCTTTCTACATCTCAATTATTGATGACACTTACGATATTTACGGTAAGTTGGATGA
ACTGACTTTGTTCACCCAAGCTATCGAAAGATGGAACATTGACGCAAGTGAACAA
TTACCATTGTACATGAAAATTATTTACAGAGATTTGTTGGATGTCTACGACGAAATT
GAAAAGGAATTAGCCAATGAAAACAAGTCTTTTTTGGTTAACTACTCCATCAATGA
AATGAAAAAGGTCGTTAGAGGTTACTTCCAAGAAGCCAAGTGGTACTATGGTAAG

AAGGTTCCAACCATGGAACAATATATGAAGAACGGTATCTCCACTTCTGCCTACAT
CTTGCTAACCACCACATCTTGGTTAGCTATGGGTAACGTTGCTACTAAGGATGCTTT
TGACTGGGTTGCCACTGAACCACCAATTGTTGTCGCCTCTTGTTACATCATCAGACT
ATTGAACGATTTAGTCTCCCACGAAGAAGAACAAAAGAGAGGTAACGCTGCCTCC
GCTGTTGAATGTTATATGAACGAGTACTCTGTTACCAAGGAAGAAGCTCACATTAA
GATTAGAGATATTATTGAAAATTACTGGAAGGACTTGAACGAAGAATACTTCAAGG
TTGACATGATCATCATCCCAAGAGTTTTGTTGATGTGTATCATCAACTTGACTCGTG
TAGCTGAATTCATCTACAAGGATGAAGACGCTTACACCTTTTCTAAGAACAACTTG
AAGGACGTTATCTCTGACATTCTTGTCGATCCTATCATTTGA (SEQ ID NO: 36).

The amino acid sequence of the valencene synthase derived from *Switchgrass (Panicum virgatum L.)* was:

MAASEAASPPVSVEMMMGAAGLTGFEPCVWGDFFITLAPPFSQEAEKRMRERV
EQLRGELRHRVFEAAGEAMSVADTVALVDTLERLGVDSHFREEIGAALRRVVNSGPG
SCKDGDLRVVALRFRLLRQHGYWVPTDVFDRFRDDTGSFRASLSSDPGGLLSLYNAA
HMATPGERVLDEAISFCRRHLESMRGELASPLAEQVSRALDIPLPRLPKRLETVRYIVE
YEKEEGHDPVALELARLDFDLVRALHLEELRALSLWWKEVYGDVKLSYARDRLVEN
YFWTCGVFHEEEYSRARMLFAKTFGMLSLMDDTYDVYATLEECHVLNEAIQRWDES
AVSTLPEYMKKFYINLLKTFQECEDSLQPDEKYRVSYAKKAFQLSSKYYLDEAKWSS
EKYAPSFEEHVEVSVMSSGFPTLAVVLLMGAGDLATREAFEWAIGVPAVVSASGEVAR
FLNDIASYRKGKNKKDALSSVECYARERGVSGEEAAAAIAGMAEHAWRTINRSCME
VGGALLPAARLVVNLTKTLEVIYLGGRDAYTFAGDLRDLVVSLFLNGPAI (SEQ ID NO:
37).

The nucleotide sequence after codon optimization for *Saccharomyces cerevisiae* was:

ATGGCCGCCTCCGAAGCCGCCTCCCCACCAGTCTCTGTCGAAATGATGATGGG
TGCCGCTGGCCTTACTGGTTTCGAACCATGCGTCTGGGGTGATTTCTTCATCACATT
GGCTCCTCCATTCTCTCAAGAAGCTGAAAAGAGAATGAGAGAAAGAGTTGAACAA
TTGAGAGGTGAATTGAGACACAGAGTCTTTGAAGCCGCTGGTGAAGCTATGTCCG
TCGCTGACACTGTCGCCTTGGTCGATACCCTGGAAAGATTGGGTGTTGACTCCCAC
TTCCGTGAAGAAATCGGTGCTGCTTTGAGAAGAGTTGTCAACTCTGGTCCAGGTT
CCTGTAAGGATGGTGACTTGAGAGTCGTTGCTTTGCGTTTTAGATTGCTAAGACAA
CACGGTTACTGGGTTCCAACTGATGTTTTCGACAGATTCAGAGACGACACCGGTTC
TTTCAGAGCTTCTTTGTCTTCTGACCCAGGTGGTTTGCTATCCTTGTACAACGCTGC
TCACATGGCTACTCCAGGAGAACGTGTTCTAGATGAAGCTATCTCCTTCTGTAGAC
GGCATTTGGAATCCATGAGAGGTGAATTAGCTTCTCCATTGGCTGAACAAGTTTCT

CGTGCTTTGGATATCCCATTGCCAAGACTACCAAAGAGATTGGAAACTGTTAGATA
CATTGTTGAATACGAAAAAGAAGAAGGTCATGACCCAGTTGCTTTGGAATTGGCTA
GATTGGACTTCGATCTCGTCAGAGCTTTGCACTTGGAGGAATTGAGAGCCTTATCT
TTGTGGTGGAAGGAAGTCTACGGTGATGTCAAGTTGTCTTACGCTCGTGACAGATT
GGTTGAAAACTACTTCTGGACTTGTGGTGTCTTCCACGAAGAAGAATACTCAAGA
GCTAGAATGTTGTTCGCCAAGACCTTCGGTATGTTGTCCTTGATGGACGATACTTAC
GATGTTTACGCTACCCTCGAAGAATGTCACGTTTTGAACGAAGCTATCCAAAGATG
GGATGAATCTGCTGTCTCCACTTTGCCAGAATATATGAAGAAGTTCTACATCAACTT
GTTGAAGACTTTCCAAGAATGTGAAGACTCTTTGCAACCAGACGAAAAGTACAGA
GTTTCCTATGCCAAAAAAGCTTTCCAATTATCCTCTAAGTACTACTTGGATGAAGCT
AAATGGTCTTCTGAAAAGTACGCTCCATCCTTCGAAGAACATGTCGAAGTTTCCGT
TATGTCTTCTGGTTTCCCAACTTTGGCTGTTGTTTTGTTGATGGGTGCTGGTGACTT
AGCTACCAGAGAAGCTTTCGAATGGGCTATTGGTGTACCTGCTGTCGTCTCCGCTT
CGGGTGAAGTTGCCAGATTTTTGAACGATATTGCTTCATACAGAAAGGGTAAGAAC
AAGAAGGACGCACTATCTTCTGTTGAATGTTACGCCAGGGAAAGAGGCGTGTCCG
GTGAAGAAGCTGCAGCCGCCATTGCCGGTATGGCCGAACACGCTTGGAGAACCAT
TAACAGAAGTTGTATGGAAGTTGGTGGTGCTCTTCTCCCGGCTGCTAGGTTGGTTG
TTAACTTGACCAAGACCTTGGAAGTTATTTATTTAGGTGGTCGTGATGCTTACACCT
TTGCTGGTGACTTGAGAGACTTAGTCGTGTCTTTGTTTCTAAATGGTCCAGCCATCT
GA (SEQ ID NO: 38).

The amino acid sequence of the valencene synthase derived from *Callitropsis nootkatensis* was:

MAEMFNGNSSNDGSSCMPVKDALRRTGNHHPNLWTDDFIQSLNSPYSDSSYHK
HREILIDEIRDMFSNGEGDEFGVLENIWFVDVVQRLGIDRHFQEEIKTALDYIYKFWN
HDSIFGDLNMVALGFRILRLNRYVASSDVFKKFKGEEGQFSGFESSDQDAKLEMMLN
LYKASELDFPDEDILKEARAFASMYLKHVIKEYGDIQESKNPLLMEIEYTFKYPWRCR
LPRLEAWNFIHIMRQQDCNISLANNLYKIPKIYMKKILELAILDFNILQSQHQHEMKLIS
TWWKNSSAIQLDFFRHRHIESYFWWASPLFEPEFSTCRINCTKLSTKMFLLDDIYDTY
GTVEELKPFTTTLTRWDVSTVDNHPDYMKIAFNFSYEIYKEIASEAERKHGPFVYKYL
QSCWKSYIEAYMQEAEWIASNHIPGFDEYLMNGVKSSGMRILMIHALILMDTPLSDEI
LEQLDIPSSKSQALLSLITRLVDDVKDFEDEQAHGEMASSIECYMKDNHGSTREDALN
YLKIRIESCVQELNKELLEPSNMHGSFRNLYLNVGMRVIFFMLNDGDLFTHSNRKEIQ
DAITKFFVEPIIP (SEQ ID NO: 39).

The nucleotide sequence after codon optimization for *Saccharomyces cerevisiae* was:

ATGGCTGAAATGTTCAACGGTAATTCTTCTAATGATGGTTCATCATGTATGCCA
GTTAAGGATGCTTTGAGGAGAACTGGCAATCATCACCCTAACTTGTGGACTGATGA
CTTTATACAATCTTTAAATTCTCCATACTCAGATTCTTCATACCATAAACACAGAGAA
ATTTTAATTGATGAAATAAGAGATATGTTTTCAAACGGTGAGGGTGATGAGTTCGGT
GTTTTGGAAAATATATGGTTTGTTGATGTTGTCCAGAGGTTGGGTATTGATAGACAC
TTTCAAGAAGAGATTAAAACTGCTTTGGATTACATATACAAATTCTGGAACCATGAT
TCAATTTTCGGTGATTTGAATATGGTTGCTTTGGGATTTAGAATTTTGAGATTAAATA
GATATGTTGCTTCTTCAGATGTCTTTAAAAAATTCAAAGGTGAGGAAGGTCAATTC
TCTGGTTTTGAATCTTCTGACCAAGATGCAAAATTGGAAATGATGTTGAATTTATAT
AAGGCTTCTGAATTGGATTTTCCAGATGAAGATATATTGAAAGAAGCTAGAGCTTT
TGCTTCTATGTACTTGAAACATGTCATTAAGGAATACGGTGATATTCAAGAATCAAA
GAACCCATTGTTGATGGAGATTGAATATACATTTAAGTACCCTTGGAGGTGTAGATT
GCCAAGATTGGAGGCTTGGAATTTCATTCATATTATGAGACAACAAGATTGTAACAT
ATCTTTGGCAAACAATTTGTACAAAATTCCTAAAATATATATGAAGAAGATTTTGGA
ATTGGCTATTTTAGATTTCAATATTTTGCAATCACAACATCAACATGAAATGAAGTT
GATTTCTACTTGGTGGAAAAACTCTTCTGCTATACAGTTAGATTTCTTTCGTCATAG
ACATATTGAATCTTACTTCTGGTGGGCTTCTCCTTTATTCGAACCAGAGTTTTCTACT
TGTAGAATTAATTGTACTAAGTTGTCTACTAAGATGTTTTTGTTGGATGATATTTACG
ATACTTATGGAACTGTTGAAGAATTGAAGCCATTTACAACAACTTTAACTAGATGG
GATGTTTCTACTGTTGACAACCACCCAGATTATATGAAGATTGCTTTCAATTTTTCTT
ATGAAATTTATAAAGAAATTGCATCAGAAGCAGAGAGGAAGCACGGTCCATTTGTC
TATAAATATTTGCAATCTTGTTGGAAATCATACATTGAGGCTTATATGCAAGAAGCA
GAGTGGATAGCTTCAAACCACATTCCCGGTTTTGATGAATACTTGATGAACGGTGT
CAAATCTTCTGGTATGAGAATATTGATGATTCATGCTTTGATTTTGATGGATACACCA
TTGTCTGATGAAATATTGGAACAATTAGACATACCATCTTCAAAGTCACAAGCCTTA
TTGTCTTTGATTACTAGGTTGGTTGATGATGTTAAAGATTTCGAAGATGAACAAGCT
CATGGTGAAATGGCTTCTTCTATTGAATGTTATATGAAGGATAACCACGGTTCAACT
AGGGAGGACGCATTGAATTACTTAAAAATTAGGATTGAATCTTGCGTTCAAGAATT
GAATAAAGAATTATTAGAGCCTTCAAACATGCACGGTTCTTTTCGTAATTTATATTTA
AATGTTGGTATGAGGGTTATCTTTTTTATGTTGAATGATGGTGATTTGTTTACACATT
CTAATAGAAAAGAAATTCAAGACGCTATTACAAAGTTCTTTGTTGAACCTATTATTC
CTTAA (SEQ ID NO: 40).

The amino acid sequence of the valencene synthase derived from *Eryngium glaciale* was:

MSLNVLSTSGSAPTTKSSEITRRSANYHPSLWGDKFLEYSSPDHLKNDSFTEKKH
EQLKEEVKKMLVETVQKPQQQLNLINEIQRLGLSYLFEPEIEAALQEISVTYDEFCCST
DADDLHNVALSFRILREHGHNVSSDVFQKFMDSNGKLKDYLVNDARGLLSLYEATHF
RVHNDDKLEELLSVTTSRLEHLKSHVKYPLEDEISRALKHPLHKELNRLGARYYISIYE
KFDSHNKLLLEFAKLDFNRLQKMYQHELAHLTRWWKDLDFTNKLPFARDRIVEGYF
WILGMYFEPERKDVREFLNRVFALITVVDDTYDVYGTFKELLLFTDAIERWGTSDLD
QLPGYMRIIYQALMDVYNQMEEKLSMKADCPTYRLEFAIETVKAMFRSYLEEARWS
KEHYIPSMEEYMTVALVSVGYKTILTNSFVGMGDIATREVFEWVFNSPLIIRASDLIAR
LGDDIGGHEEEQKKGDAATAIECYIKENHVTKHEAYDEFQKQIDNAWKDLNKEALRP
FPVPMTFITRVVHFTRAIHVIYADFSDGYTRSDKAIRGYITSLLVDPIPL (SEQ ID NO:
41).

The nucleotide sequence after codon optimization for *Saccharomyces cerevisiae* was:

ATGTCATTGAATGTTTTATCTACATCAGGTTCTGCTCCAACTACAAAGTCTTCA
GAAATCACTAGAAGATCAGCAAACTACCATCCATCTTTATGGGGTGACAAATTTTT
GGAATACTCTTCACCAGATCATTTGAAAAATGATTCTTTTACAGAAAAGAAACATG
AACAATTAAAAGAAGAAGTTAAGAAAATGTTGGTTGAAACTGTTCAAAAGCCACA
ACAACAATTGAATTTGATTAATGAAATCCAAAGATTGGGTTTGTCATACTTATTCGA
ACCAGAAATCGAAGCTGCATTGCAAGAAATCTCAGTTACTTACGATGAATTCTGTT
GTTCTACAGATGCTGATGATTTGCATAACGTTGCATTGTCTTTTAGAATCTTGAGAG
AACATGGTCATAATGTTTCTTCAGATGTTTTTCAAAAGTTTATGGATTCAAACGGTA
AATTGAAGGATTATTTGGTTAATGATGCTAGAGGTTTGTTATCTTTGTACGAAGCAA
CACATTTTAGAGTTCATAACGATGATAAGTTGGAAGAATTGTTGTCAGTTACTACAT
CTAGATTGGAACATTTGAAGTCACATGTTAAGTACCCATTGGAAGATGAAATCTCTA
GAGCTTTGAAGCATCCATTGCATAAGGAATTGAACAGATTGGGTGCAAGATACTAC
ATCTCAATCTATGAAAAGTTTGATTCTCATAATAAGTTGTTATTGGAATTTGCTAAAT
TAGATTTTAATAGATTGCAAAAGATGTATCAACATGAATTGGCACATTTGACAAGAT
GGTGGAAGGATTTGGATTTCACTAATAAGTTGCCATTTGCTAGAGATAGAATCGTTG
AAGGTTACTTCTGGATCTTGGGCATGTACTTCGAACCAGAAAGAAAGGATGTTAGA
GAATTCTTGAACAGAGTTTTCGCTTTGATCACTGTTGTTGATGATACATACGATGTT
TACGGTACTTTTAAAGAATTGTTGTTGTTTACTGATGCAATTGAAAGATGGGGTACT
TCTGATTTGGATCAATTGCCAGGTTACATGAGAATCATCTATCAAGCTTTGATGGAT
GTTTACAACCAAATGGAAGAAAAGTTGTCAATGAAAGCAGATTGTCCAACATACA
GATTGGAATTCGCTATCGAAACTGTTAAGGCAATGTTCAGATCTTATTTGGAAGAA
GCTAGATGGTCAAAGGAACATTACATCCCATCTATGGAAGAATACATGACAGTTGC

ATTAGTTTCAGTTGGTTACAAGACTATCTTGACAAACTCTTTCGTTGGTATGGGTGA
CATTGCTACTAGAGAAGTTTTCGAATGGGTTTTTAATTCACCATTAATCATCAGAGC
TTCTGATTTGATTGCAAGATTAGGTGACGATATTGGTGGTCATGAAGAAGAACAAA
AGAAAGGTGACGCTGCAACAGCTATCGAATGTTACATCAAGGAAAACCATGTTACT
AAGCATGAAGCATACGATGAATTCCAAAAGCAAATCGATAATGCTTGGAAAGATTT
GAATAAGGAAGCATTGAGACCATTTCCAGTTCCAATGACTTTTATTACAAGAGTTG
TTCATTTCACTAGAGCTATCCATGTTATCTATGCAGATTTCTCAGATGGTTACACAAG
ATCTGATAAGGCTATCAGAGGTTACATCACTTCTTTGTTGGTTGATCCAATTCCATTA
TAA (SEQ ID NO: 42).

After plasmid construction using the valencene synthase gene derived from Alpinia *oxyphylla Miq.,* the amino acid sequence of the valencene synthase expressed thereby was:

MEKQSVTLVRDDQGIVRKSTKYHPSVWGDYFIRNSPLNLSEESTQRMIERVEELK
VQVKSMFKGTSDVLQIMNLIDSIQLLRLEYHFENEIDGALRLIYEVDDKNYGLYETSL
RFRLLRQHGYNVSADTFNKFKDENGSFISILNGDAKGLLSLYNASYLATHGETILDEA
NNYTKSQLVSLLSELEQPLATQVSLFLEAPLCRRMKSILARKYIPIYEKEAMRSDDILE
LAKLDFNLLQSLHQEELKKASIWWNDLALAKSLSFTRDRIVEGYYWILSMCYEPQYS
RARVMCAKAFCLLSIMDDIYDNYSILEERRLLTEAIKRWNHEAVDSLPEYIKDFYLKL
LKAFEEFEAELEFNEKYRVQYLQNEFKAIAISYFEESKWCVERYVPSLDEHLRVSMITS
GCSMVVCSMYLGMGEVATKEIFDWCSSFPKAMEASGVIARLLNDIRSHETEQGRDHA
ASTVESYMKEHGVDVKVARKKLQEIVEKAWKDLNKELLNPTPVARPIIERILNLTMSM
EDIYRYIDEYTSPDNKTNGDVSLVLVESIPI (SEQ ID NO: 43).

The nucleotide sequence after codon optimization for *Saccharomyces cerevisiae* was:

ATGGAAAAGCAATCTGTTACATTGGTTAGAGATGATCAAGGTATTGTTAGAAA
ATCTACAAAGTACCATCCATCTGTTTGGGGTGATTATTTTATTAGAAACTCTCCATTG
AACCTGTCTGAAGAATCTACTCAAAGAATGATTGAAAGAGTTGAAGAATTGAAGG
TTCAAGTTAAATCTATGTTCAAGGGTACATCTGATGTTTTGCAAATTATGAATCTGAT
CGATTCTATCCAATTGTTAAGATTAGAGTACCATTTCGAAAACGAAATTGATGGTGC
TTTAAGATTAATCTACGAAGTTGATGATAAGAACTACGGTTTGTATGAAACATCTTT
AAGATTCAGACTGTTGAGACAACATGGTTATAATGTTTCTGCTGATACTTTTAACAA
GTTCAAAGATGAAAACGGTTCTTTTATCTCTATCTTAAACGGTGATGCTAAAGGTTT
GTTATCTTTATATAACGCTTCCTATCTGGCTACACATGGTGAAACAATTTTAGATGAA
GCTAATAACTACACCAAGTCTCAATTAGTTTCTTTGTTGTCTGAATTGGAACAACCA
TTAGCTACTCAAGTTTCTTTATTTTTGGAGGCTCCATTATGTAGAAGAATGAAATCTA
TTCTGGCTAGAAAATACATCCCAATTTATGAAAAGGAGGCTATGAGATCTGATGATA

TTTTGGAATTGGCTAAATTGGATTTCAACTTATTGCAATCTCTGCATCAAGAAGAAT
TAAAAAAGGCTTCTATCTGGTGGAATGATTTGGCTTTAGCTAAATCTTTGTCTTTTA
CAAGAGACAGAATCGTTGAAGGTTATTATTGGATTTTGTCTATGTGTTACGAGCCAC
AATATTCTAGAGCTAGAGTTATGTGTGCTAAAGCTTTTTGTTTATTGTCTATCATGGA
CGATATCTATGATAATTACTCTATCTTGGAGGAAAGAAGATTGTTAACAGAAGCTAT
TAAGAGATGGAATCATGAAGCTGTTGATTCTTTGCCAGAATATATTAAAGACTTCTA
CTTGAAGCTGTTGAAAGCTTTTGAAGAATTTGAAGCTGAATTGGAATTCAATGAAA
AGTATAGAGTCCAATACTTGCAAAATGAATTCAAAGCTATCGCTATTTCTTACTTTG
AAGAATCTAAGTGGTGTGTTGAAAGATATGTTCCATCTTTGGATGAACATTTGAGA
GTTTCTATGATTACTTCTGGTTGTTCTATGGTTGTTTGTTCTATGTATTTGGGTATGGG
TGAAGTTGCTACAAAAGAAATTTTTGATTGGTGTTCTTCCTTCCCAAAAGCTATGG
AAGCTTCTGGTGTTATTGCTAGATTATTAAATGACATCAGGTCACATGAAACAGAAC
AAGGTAGAGATCATGCTGCTTCTACAGTTGAATCTTATATGAAAGAACACGGTGTT
GATGTTAAAGTTGCTAGAAAAAAACTGCAAGAAATCGTTGAAAAGGCTTGGAAAG
ATTTGAATAAAGAATTGTTGAACCCCACACCAGTTGCTAGACCAATTATTGAAAGA
ATTTTGAACCTGACTATGTCTATGGAAGATATTTATAGATACATCGACGAATACACAT
CTCCAGATAATAAAACAAACGGTGATGTTTCTTTGGTTTTGGTTGAATCTATCCCAA
TTTAA (SEQ ID NO: 44).

### 1.2 Construction and shake flask fermentation of strains containing valencene synthase genes derived from Vitis vinifera (Grape), Citrus sinensis (Sweet orange) (Citrus aurantium var. sinensis), Solanum habrochaites (Wild tomato) (Lycopersicon hirsutum), Solanum lycopersicum (Tomato) (Lycopersicon esculentum), Switchgrass (Panicum virgatum L.), Callitropsis nootkatensis, Eryngium glaciale, and Alpinia oxyphylla Miq.

Plasmids pVvVS, pCsVS, pShVS, pSlVS, pPvVS, pCnVS, pEgVS, and pAoVS were separately introduced into strain JCR27 by a lithium acetate method. The engineered strains were named JVvVS, JCsVS, JShVS, JSlVS, JPvVS, JCnVS, JEgVS, JAoVS, respectively. According to the same shake flask fermentation conditions and method as in sections 1.2.3 and 1.3 in Example 1, the strains were fermented. The shake flask fermentation yields of the strains JVvVS, JCsVS, JShVS, JSlVS, JPvVS, JCnVS, JEgVS, and JAoVS were 0.35 mg/L, 15 mg/L, 0.06 mg/L, 0.07 mg/L, 1.5 mg/L, 40 mg/L, 80 mg/L, and 65 mg/L, respectively.

By comparing Examples 1 and 2 with the comparative example, it can be seen that the valencene synthases derived from *Actaea racemosa* and *Citrus maxima (Pomelo, pummelo),* as provided by the present invention, had higher synthesis activity as compared with existing valencene synthases.

### Example 3. Construction of high-valencene-yield strains

### 3.1 Construction of recombinant plasmids

### 3.1.1 Construction of high-yield plasmid corresponding to ArVS

Using primers ZL-ArVS-1F/R and ZL-ArVS-2F/R, pYR017 (see patent 202210473488.4) and pArVS separately used as templates were amplified to obtain long fragments (comprising Ura3 left homologous arm, a histidine selectable marker, a CYC1 terminator, a tHMG1 gene, a GAL1-GAL10 promoter, a PGK1 terminator, Ura3 right homologous arm, and a plasmid backbone) and ArVS. Subsequently, using a yeast assembly method, the above fragments were recombined in *Saccharomyces cerevisiae* to construct pArVS-P2.

The primer sequences of ZL-ArVS-1F/R and ZL-ArVS-2F/R are as shown in the following table:

| Plasmid | Primer | Sequence (from 5' to 3') |
|---|---|---|
| | ZL-ArVS -1F | GTTGGTAGACCCAATCCCAATGTGAATTGAATTGAATTGA AATCGATAGATCAAT (SEQ ID NO: 45) |
| pArVS-P 2/P3 | ZL-ArVS -1R | GTTGGCACTTCTTCTGGTAACTTCCATtatagttttttctccttgacgttaa agtat (SEQ ID NO: 46) |
| | ZL-ArVS -2F | ctctatactttaacgtcaaggagaaaaaactataATGGAAGTTACCAGAAGA AGTG (SEQ ID NO: 47) |
| | ZL-ArVS -2R | TTGATCTATCGATTTCAATTCAATTCAATTCACATTGGGAT TGGGTCTAC (SEQ ID NO: 48) |

Using primers ZL-ArVS-1F/R and ZL-ArVS-2F/R, pYR018 (see patent 202210473488.4) and pArVS separately used as templates were amplified to obtain long fragments (comprising yprcdelta15 left homologous arm, a tryptophan selectable marker, a GPM1 terminator, a GAL1-GAL10 promoter, a PGK1 terminator, yprcdelta15 right homologous arm, and a plasmid backbone) and ArVS. Subsequently, using a yeast assembly method, the above fragments were recombined in *Saccharomyces cerevisiae* to construct pArVS-P3.

### 3.1.2 Construction of high-yield plasmid corresponding to ArVS-V8

Using primers ZL-ArVS-V8-1F/R and ZL-ArVS-V8-2F/R, pYR017 (see patent 202210473488.4) and pArVS-V8 separately used as templates were amplified to obtain long fragments (comprising Ura3 left homologous arm, a histidine selectable marker, a CYC1 terminator, a tHMG1 gene, a GAL1-GAL10 promoter, a PGK1 terminator, Ura3 right homologous arm, and a plasmid backbone) and ArVS-V8. Subsequently, using a yeast assembly method, the above fragments were recombined in *Saccharomyces cerevisiae* to construct pArVS-V8-P2.

The primer sequences of ZL-ArVS-V8-1F/R and ZL-ArVS-V8-2F/R are as shown in the following table:

| Plasmid | Primer | Sequence (from 5' to 3') |
|---|---|---|
| | ZL-ArVS-V8-1F | CTTTGTTGGTAGACCCAATCCCAATGTGAATTGAATTGA ATTGAAATCGATAGATCAAT (SEQ ID NO: 49) |
| pArVS-V8-P2/P 3 | ZL-ArVS-V8-1R | AACCAGAAGTGGATAGCACATTTAACGACATtatagttttttctc cttgacgttaaagt (SEQ ID NO: 50) |
| | ZL-ArVS-V8-2F | tactttaacgtcaaggagaaaaaactataATGTCGTTAAATGTGCTATCC ACTTCTGGT (SEQ ID NO: 51) |
| | ZL-ArVS-V8-2R | TATCGATTTCAATTCAATTCAATTCACATTGGGATTGGG TCTACCAACAAAG (SEQ ID NO: 52) |

Using primers ZL-ArVS-V8-1F/R and ZL-ArVS-V8-2F/R, pYR018 (see patent 202210473488.4) and pArVS-V8 separately used as templates were amplified to obtain long fragments (comprising yprcdelta15 left homologous arm, a tryptophan selectable marker, a GPM1 terminator, a GAL1-GAL10 promoter, a PGK1 terminator, yprcdelta15 right homologous arm, and a plasmid backbone) and ArVS-V8. Subsequently, using a yeast assembly method, the above fragments were recombined in *Saccharomyces cerevisiae* to construct pArVS-V8-P3.

### 3.1.3 Construction of high-yield plasmid corresponding to CmVS-V4

Using primers ZL-CmVS-V4-1F/R and ZL-CmVS-V4-2F/R, pYR017 (see patent 202210473488.4) and pCmVS-V4 separately used as templates were amplified to obtain long fragments (comprising Ura3 left homologous arm, a histidine selectable marker, a CYC1 terminator, a tHMG1 gene, a GAL1-GAL10 promoter, a PGK1 terminator, Ura3 right homologous arm, and a plasmid backbone) and CmVS-V4. Subsequently, using a yeast assembly method, the above fragments were recombined in *Saccharomyces cerevisiae* to construct pCmVS-V4-P2.

The primer sequences of ZL-ArVS-1F/R and ZL-ArVS-2F/R are as shown in the following table:

| Plasmid | Primer | Sequence (from 5' to 3') |
|---|---|---|
| | ZL-CmVS -V4-1F | TTTGGGTGACCACGTCCCATTCTGAATTGAATTGAATTG AAATCGATAGATCAAT (SEQ ID NO: 53) |
| pCmVS-V4-P2/P 3 | ZL-CmVS -V4-1R | GTCTGAAAGTTTCACCAGAAGACATtatagttttttctccttgacgttaa agt (SEQ ID NO: 54) |
| | ZL-CmVS -V4-2F | tactttaacgtcaaggagaaaaaactataATGTCTTCTGGTGAAACTTTCA GAC (SEQ ID NO: 175) |
| | ZL-CmVS -V4-2R | CTATCGATTTCAATTCAATTCAATTCAGAATGGGACGTGG TCAC (SEQ ID NO: 176) |

Using primers ZL-CmVS-V4-1F/R and ZL-CmVS-V4-2F/R, pYR018 (see patent 202210473488.4) and pCmVS-V4 separately used as templates were amplified to obtain long fragments (comprising yprcdelta15 homologous arm, a tryptophan selectable marker, a GPM1 terminator, a GAL1-GAL10 promoter, a PGK1 terminator, yprcdelta15 right homologous arm, and a plasmid backbone) and CmVS-V4. Subsequently, using a yeast assembly method, the above fragments were recombined in *Saccharomyces cerevisiae* to construct pCmVS-V4-P3.

### 3.1.4 Construction of gene-editing plasmid

Using primers GHX07-1F/R, pKlURA100 (see patent 202210473488.4) used as a template was amplified to obtain a fragment. Subsequently, using a Goldengate method, the above fragment and pCas (see patent 202210473488.4) were assembled to construct plasmid pGHX07, which targeted the gene ACH-TPS07 in strain LXF-5 (see patent 202210473488.4).

The primer sequences of GHX07-1F/R are as shown in the following table:

| Plasmid | Primer | Sequence (from 5' to 3') |
|---|---|---|
| pGHX0 7 | GHX07-1F | aaaggtctcagatctgagtccatcctacaaagtcgttttagagctagaaatagcaagtt (SEQ ID NO: 177) |
| | GHX07-1R | aaaggtctcaaaactctagactttttcgatgatgtagtttct (SEQ ID NO: 178) |

### 3.2 Construction of high-yield strains

### 3.2.1 Construction of high-yield strain corresponding to ArVS

The plasmids pArVS and pArVS-P2 were separately linearized by MssI. The plasmid pArVS-P3 was linearized by NotI. Gel recovery was carried out to obtain fragments containing ArVS.

The plasmid pGHX07 (transfer plasmid), the linearized pArVS fragment, the linearized pArVS-P2 fragment, and the linearized pArVS-P3 fragment were co-transferred into strain LXF-5 (see patent 202210473488.4) by a lithium acetate method. Colonies were verified by PCR to confirm the correctness of the strain. Subsequently, after culturing using YPD (peptone, yeast extract, and glucose), the strain was washed with water and streaked onto a 5-FOA plate. Colonies were picked from the plate to continue verification of the colonies by PCR for knockout correctness. The correct strain was named JArVS-P1. A pZY521 knockout cassette (see patent 202210473488.4) was transferred into strain JArVS-P1 to knock out transcriptional repressor GAL80 and obtain strain JArVS-P2.

### 3.2.2 Construction of high-yield strain corresponding to ArVS-V8

The plasmids pArVS-V8 and pArVS-V8-P2 were separately linearized by MssI. The plasmid pArVS-V8-P3 was linearized by NotI. Gel recovery was carried out to obtain fragments containing ArVS-V8.

The plasmid pGHX07 (transfer plasmid), the linearized pArVS-V8 fragment, the linearized pArVS-V8-P2 fragment, and the linearized pArVS-V8-P3 fragment were co-transferred into strain LXF-5 (see patent 202210473488.4) by a lithium acetate method. Colonies were verified by PCR to confirm the correctness of the strain. Subsequently, after culturing using YPD (peptone, yeast extract, and glucose), the strain was washed with water and streaked onto a 5-FOA plate. Colonies were picked from the plate to continue verification of the colonies by PCR for knockout correctness. The correct strain was named JArVS-V8-P1. A pZY521 knockout cassette (see patent 202210473488.4) was transferred into strain JArVS-V8-P1 to knock out transcriptional repressor GAL80 and obtain strain JArVS-V8-P2.

### 3.2.3 Construction of high-yield strain corresponding to CmVS-V4

The plasmids pCmVS-V4 and pCmVS-V4-P2 were separately linearized by MssI. The plasmid pCmVS-V4-P3 was linearized by NotI. Gel recovery was carried out to obtain fragments containing CmVS-V4.

The plasmid pGHX07 (transfer plasmid), the linearized pCmVS-V4 fragment, the linearized pCmVS-V4-P2 fragment, and the linearized pCmVS-V4-P3 fragment were co-transferred into strain LXF-5 (see patent 202210473488.4) by a lithium acetate method. Colonies were verified by PCR to confirm the correctness of the strain. Subsequently, after culturing using YPD (peptone, yeast extract, and glucose), the strain was washed with water and streaked onto a 5-FOA plate. Colonies were picked from the plate to continue verification of the colonies by PCR for knockout correctness. The correct strain was named JCmVS-V4-P1. A pZY521 knockout cassette (see patent 202210473488.4) was transferred into strain JCmVS-V4-P1 to knock out transcriptional repressor GAL80 and obtain strain JCmVS-V4-P2.

### 3.3 Shake flask fermentation of strains and fermenter evaluation

The strains JArVS-P1, JArVS-P2, JArVS-V8-P1, JArVS-V8-P2, JCmVS-V4-P1, and JCmVS-V4-P2 were separately inoculated into YPD liquid medium and cultured overnight at 30°C and 200 rpm. The next day, they were transferred to 45 milliliters of YPDHG liquid medium (JArVS-P1, JArVS-V8-P1, and JCmVS-V4-P1) or YPD liquid medium (JArVS-P2, JArVS-V8-P2, and JCmVS-V4-P2) with an initial OD600 = 0.1. 5 milliliters of isopropyl myristate was added, followed by culturing for 72 hours at 30°C and 200 rpm. An oil layer was collected. The valencene yields of JArVS-P1, JArVS-P2, JArVS-V8-P1, JArVS-V8-P2, JCmVS-V4-P1, and JCmVS-V4-P2 were 186 mg/L, 203 mg/L, 414 mg/L, 603 mg/L, 344 mg/L, and 468 mg/L, respectively.

Referring to a fermenter medium and fermentation method described in the literature (Ye Z, Huang Y, Shi B, et al. Coupling cell growth and biochemical pathway induction in Saccharomyces cerevisiae for production of (+)-valencene and its chemical conversion to (+)-nootkatone [published online ahead of print, 2022 Mar 13]. Metab Eng. 2022;72:107-115. doi:10.1016/j.ymben.2022.03.005), the constructed strains JArVS-P2, JArVS-V8-P2, and JCmVS-V4-P2 were subjected to fed-batch fermentation. A covering agent was added during fermentation to achieve *in situ* extraction. The covering agent was isopropyl myristate. The fermentation process was controlled such that the dissolved oxygen was 20% or more, the pH was 5, the glucose concentration was 1-2 g/L, and the ethanol concentration was 5 g/L or less. Eventually, on a 1 L fermenter, the valencene yields of the strains reached 6.1 g/L, 17.5 g/L, and 13.1 g/L, respectively.

### Example 4. Fermentation using valencene synthases having high amino acid sequence homology

On the basis of the amino acid sequences of the ArVS and ArVS-V8 provided in Example 1 and the CmVS-V4 provided in Example 2, amino acid sequences respectively having 80%, 83%, 85%, 87%, 89%, 91%, 93%, 95%, 97%, and 99% homology thereto, corresponding nucleotide sequences codon-optimized for *Saccharomyces cerevisiae,* and corresponding upstream and downstream primers were designed. Subsequently, expression strains for these valencene synthases having the homologous amino acid sequences were constructed according to the method in Example 1 and subjected to fermentation. The results showed that the valencene yields obtained by fermentation using the strains corresponding to ArVS, ArVS-V8, and CmVS-V4 that had 83% or more amino acid sequence homology were comparable those in Examples 1 and 2, respectively. However, the valencene yields obtained by fermentation using the strains corresponding to ArVS, ArVS-V8, and CmVS-V4 that had 80% homology were reduced by 93% or more as compared with the corresponding yields of the strains having 83% homology.

The different homologous amino acid sequences, nucleotide sequences, and corresponding primers are as shown in the following table:to

| Valencene synthase | Homology ratio | Amino acid sequence | Nucleotide sequence | Primer | Sequence (from 5' to 3') |
|---|---|---|---|---|---|
| ArVS | 80% | (SEQ ID NO: 55) | (SEQ ID NO: 56) | 1F | (SEQ ID NO: 57) |
| | | | | 1R | (SEQ ID NO: 58) |
| | 83% | (SEQ ID NO: 59) | (SEQ ID NO: 60) | 1F | (SEQ ID NO: 61) |
| | | | | 1R | (SEQ ID NO: 62) |
| | 85% | (SEQ ID NO: 63) | (SEQ ID NO: 64) | 1F | (SEQ ID NO: 65) |
| | | | | 1R | (SEQ ID NO: 66) |
| | 87% | (SEQ ID NO: 67) | (SEQ ID NO: 68) | 1F | (SEQ ID NO: 69) |
| | | | | 1R | (SEQ ID NO: 70) |
| | 89% | (SEQ ID NO: 71) | (SEQ ID NO: 72) | 1F | (SEQ ID NO: 73) |
| | | | | 1R | (SEQ ID NO: 74) |
| | 91% | (SEQ ID NO: 75) | (SEQ ID NO: 76) | 1F | (SEQ ID NO: 77) |
| | | | | 1R | (SEQ ID NO: 78) |
| | 93% | (SEQ ID NO: 79) | (SEQ ID NO: 80) | 1F | (SEQ ID NO: 81) |
| | | | | 1R | (SEQ ID NO: 82) |
| | 95% | (SEQ ID NO: 83) | (SEQ ID NO: 84) | 1F | (SEQ ID NO: 85) |
| | | | | 1R | (SEQ ID NO: 86) |
| | 97% | (SEQ ID NO: 87) | (SEQ ID NO: 88) | 1F | (SEQ ID NO: 89) |
| | | | | 1R | (SEQ ID NO: 90) |
| | 99% | (SEQ ID NO: 91) | (SEQ ID NO: 92) | 1F | (SEQ ID NO: 93) |
| | | | | 1R | (SEQ ID NO: 94) |
| | 80% | (SEQ ID NO: 95) | (SEQ ID NO: 96) | 1F | (SEQ ID NO: 97) |
| | | | | 1R | (SEQ ID NO: 98) |
| | 83% | (SEQ ID NO: 99) | (SEQ ID NO: 100) | 1F | (SEQ ID NO: 101) |
| | | | | 1R | (SEQ ID NO: 102) |
| | 85% | (SEQ ID NO: 103) | (SEQ ID NO: 104) | 1F | (SEQ ID NO: 105) |
| | | | | 1R | (SEQ ID NO: 106) |
| | 87% | (SEQ ID NO: 107) | (SEQ ID NO: 108) | 1F | (SEQ ID NO: 109) |
| | | | | 1R | (SEQ ID NO: 110) |
| ArVS-V8 | 89% | (SEQ ID NO: 111) | (SEQ ID NO: 112) | 1F | (SEQ ID NO: 113) |
| | | | | 1R | (SEQ ID NO: 114) |
| | 91% | (SEQ ID NO: 115) | (SEQ ID NO: 116) | 1F | (SEQ ID NO: 117) |
| | | | | 1R | (SEQ ID NO: 118) |
| | 93% | (SEQ ID NO: 119) | (SEQ ID NO: 120) | 1F | (SEQ ID NO: 121) |
| | | | | 1R | (SEQ ID NO: 122) |
| | 95% | (SEQ ID NO: 123) | (SEQ ID NO: 124) | 1F | (SEQ ID NO: 125) |
| | | | | 1R | (SEQ ID NO: 126) |
| | 97% | (SEQ ID NO: 127) | (SEQ ID NO: 128) | 1F | (SEQ ID NO: 129) |
| | | | | 1R | (SEQ ID NO: 130) |
| | 99% | (SEQ ID NO: 131) | (SEQ ID NO: 132) | 1F | (SEQ ID NO: 133) |
| | | | | 1R | (SEQ ID NO: 134) |
| CmVS-V4 | 80% | (SEQ ID NO: 135) | (SEQ ID NO: 136) | 1F | (SEQ ID NO: 137) |
| | | | | 1R | (SEQ ID NO: 138) |
| | 83% | (SEQ ID NO: 139) | (SEQ ID NO: 140) | 1F | (SEQ ID NO: 141) |
| | | | | 1R | (SEQ ID NO: 142) |
| | 85% | (SEQ ID NO: 143) | (SEQ ID NO: 144) | 1F | (SEQ ID NO: 145) |
| | | | | 1R | (SEQ ID NO: 146) |
| | 87% | (SEQ ID NO: 147) | (SEQ ID NO: 148) | 1F | (SEQ ID NO: 149) |
| | | | | 1R | (SEQ ID NO: 150) |
| | 89% | (SEQ ID NO: 151) | (SEQ ID NO: 152) | 1F | (SEQ ID NO: 153) |
| | | | | 1R | (SEQ ID NO: 154) |
| | 91% | (SEQ ID NO: 155) | (SEQ ID NO: 156) | 1F | (SEQ ID NO: 157) |
| | | | | 1R | (SEQ ID NO: 158) |
| | 93% | (SEQ ID NO: 159) | (SEQ ID NO: 160) | 1F | (SEQ ID NO: 161) |
| | | | | 1R | (SEQ ID NO: 162) |
| | 95% | (SEQ ID NO: 163) | (SEQ ID NO: 164) | 1F | (SEQ ID NO: 165) |
| | | | | 1R | (SEQ ID NO: 166) |
| | 97% | (SEQ ID NO: 167) | (SEQ ID NO: 168) | 1F | (SEQ ID NO: 169) |
| | | | | 1R | (SEQ ID NO: 170) |
| | 99% | (SEQ ID NO: 171) | (SEQ ID NO: 172) | 1F | (SEQ ID NO: 173) |
| | | | | 1R | (SEQ ID NO: 174) |

Finally, it needs to be noted that the above various examples are only used to illustrate, rather than limiting, the technical solution of the present invention. Although the present invention has been illustrated in detail with reference to the above various examples, those of ordinary skill in the art should understand that the technical solutions described in the above various examples can still be modified, or some or all of the technical features thereof can be equivalently replaced; and these modifications or replacements do not make the essence of the corresponding technical solutions deviate from the scope of the technical solutions of the various examples of the present invention.

Some nucleotide and amino acid sequences are as follows:
(SEQ ID NO: 55) 
MEVKRRSANYKPSVWGDKFLEYSSPDALKADAKEAKKDAVTEKKLEKLSEEVAKKL
VELVDKPQQALNLINEIERLGLSHHFLPEIEAALRQISVTYDELCESLSVDDLDNVALC
LRILRSHGHDVSAGVFEKFMDSNGKLKESFLNDAIGLLSLYEATHYRVHGEDLLESLL
SETTSLLEALKSQLKYPLKDLIELALKLPLYDLVNLLGARYYIKIYEKYSSHNKLLLEF
AKLSGNRMQKQYQHELAELVRDWKDLDTENKLPFARDNIVEGYFNILGMYAEPEHI
DVREFLNRVFDLLSHIDDTYVVYGTFEEVILFTNALERWEESALDQLPEDFKIIYQAL
MDVYSQEEEKLAKKEDAPKYRLEFAIETVKALTRSYLEIARWSKERYIPTVEEYMSVA
LVSCGASTILTCSFVGMGSEATREVFEWVSSSPLIVRASDLINALGRDIGSHEEEQKWG
LAATYIECYMKEYGVTKQEAYAEAQKQIDNAKKDLNQEALRPFPVPREYITRVVNST
RTIRVIYADKHDGYTHPEKKIKGLIHSLLVQPIPM.
(SEQ ID NO: 56) 
ATGGAAGTCAAGCGTAGATCTGCTAACTACAAGCCATCTGTCTGGGGAGACAAATT
CTTGGAATACTCCTCCCCAGATGCCTTAAAGGCTGATGCCAAGGAAGCTAAGAAG
GACGCTGTCACTGAAAAAAAGTTGGAAAAGTTGTCCGAGGAAGTCGCGAAGAAG
TTGGTTGAATTAGTTGACAAGCCACAACAAGCTTTGAATCTGATCAACGAAATTGA
AAGATTGGGTTTGTCTCATCACTTCCTACCAGAAATCGAAGCAGCTTTGAGACAAA
TCTCTGTTACCTACGACGAGTTGTGCGAATCTTTGTCTGTCGATGATTTGGACAACG
TTGCTTTGTGTTTGAGAATTTTGAGATCCCATGGTCACGACGTCTCCGCTGGTGTTT
TCGAAAAGTTCATGGACTCTAACGGTAAGTTAAAAGAATCCTTCTTGAACGATGCT
ATTGGTTTGTTGTCCTTGTATGAAGCCACACATTACAGAGTTCACGGTGAAGACTT
GTTAGAATCTTTGTTGTCTGAAACTACTTCTTTGTTGGAAGCCTTGAAGTCTCAATT
GAAATACCCATTGAAGGACTTGATTGAATTGGCTTTGAAGTTGCCTCTGTACGATTT
GGTTAACTTGTTGGGTGCGAGATACTACATCAAGATATACGAAAAGTACTCTTCAC
ACAACAAGTTGTTGCTTGAGTTTGCCAAGCTGTCTGGTAACAGAATGCAAAAGCA
ATACCAACACGAATTGGCCGAATTGGTCAGAGACTGGAAAGATTTGGATACCGAA
AACAAGTTACCTTTCGCTCGTGATAACATTGTTGAAGGTTACTTCAACATCTTGGGT
ATGTACGCTGAACCAGAACACATTGACGTCCGTGAATTCTTAAACAGAGTTTTTGA
CTTGCTGTCCCACATCGATGACACTTACGTTGTTTACGGTACTTTCGAAGAAGTTAT
CTTGTTCACCAACGCTTTGGAAAGATGGGAAGAATCCGCTTTGGACCAATTGCCA
GAAGATTTCAAGATCATTTACCAAGCTTTGATGGATGTCTACTCACAAGAAGAAGA
AAAGTTGGCTAAGAAGGAAGACGCTCCAAAGTACAGATTGGAATTCGCCATTGAA
ACTGTTAAGGCTCTCACCCGTTCTTACTTGGAAATCGCCAGATGGTCTAAGGAAAG
ATACATCCCAACTGTCGAAGAATATATGAGTGTCGCCTTAGTGTCTTGTGGTGCTTC
CACTATTTTGACTTGTTCTTTCGTTGGTATGGGTTCCGAAGCTACCAGAGAAGTTTT
TGAATGGGTTTCCTCCTCTCCACTTATCGTTAGAGCTTCTGACTTAATTAATGCTTTA
GGTAGAGATATCGGTTCTCACGAAGAAGAACAAAAGTGGGGCTTAGCTGCTACTTA
TATCGAATGTTACATGAAGGAATACGGTGTCACCAAGCAAGAAGCTTACGCCGAA
GCTCAAAAGCAAATTGACAACGCTAAGAAAGACTTGAACCAAGAAGCTCTAAGA
CCATTCCCAGTTCCAAGAGAATATATTACCCGCGTTGTCAACTCCACCAGAACTATC
AGAGTAATCTACGCTGACAAGCACGATGGTTACACCCACCCAGAAAAGAAAATCA
AGGGTTTGATTCACTCTTTATTGGTCCAACCAATTCCAATGTGA.
(SEQ ID NO: 59) 
MEVKRRSANYKPSVWGDKFLEYSSPDALKADAKEAKKDAVTEKKLEKLKEEVAKM
LVETVDKPQQALNLINEIQRLGLSHHFLPEIEAALRQISVTYDELCESLSVDDLDNVAL
CLRILRSHGHDVSAGVFEKFMDSNGKLKGSFLNDAIGLLSLYEATHFRVHGEDLLESL
 
LSLTTSLLEALKSQLKYPLKDEITLALKLPLYDEVNLLGARYYIKIYEKYSSHNKLLLEF
AKLDGNRMQKQYQHELAELVRDWKDLDTENKLPFARDRIVEGYFNILGMYAEPEHK
DVREFLNRVFDLLSHIDDTYVVYGTFEEVILFTNAIERWEESALDQLPEDMKIIYQAL
MDVYSQEEEKLAKKEDAPKYRLEFAIETVKALTRSYLEIARWSKERYIPTVEEYMSVA
LVSCGASTILTCSFVGMGDEATREVFEWVSSSPLIVRASDLINALGRDIGSHEEEQKWG
LAATYIECYMKEYGVTKQEAYAEAQKQIDNAKKDLNQEALRPFPVPREYITRVVNST
RTIRVIYADKHDGYTHPEKKIKGLIHSLLVQPIPM.
(SEQ ID NO: 60) 
ATGGAAGTTAAGAGAAGATCTGCTAACTACAAGCCTTCCGTCTGGGGTGACAAATT
CTTGGAATACTCTTCTCCAGATGCCTTGAAGGCTGACGCTAAGGAAGCTAAGAAG
GATGCTGTCACTGAAAAGAAGTTAGAGAAGTTGAAGGAAGTCGCTAAGATGT
TGGTTGAAACTGTTGACAAGCCACAACAAGCTTTGAACTTGATCAACGAAATCCA
AAGATTGGGTTTGTCCCATCACTTCTTGCCAGAAATTGAAGCTGCCTTGAGACAAA
TCTCTGTAACTTACGATGAATTGTGTGAATCCTTGTCTGTCGATGATTTGGACAACG
TTGCTCTATGTTTGAGAATTTTGAGATCCCATGGTCACGATGTTTCCGCTGGTGTCT
TCGAAAAGTTCATGGACTCCAACGGTAAGTTGAAGGGTTCTTTCTTGAACGATGCT
ATTGGTCTATTGTCCTTGTACGAAGCTACCCATTTCAGGGTCCACGGTGAAGACTTA
TTGGAATCTTTGCTGTCTTTGACCACTTCTTTGTTGGAAGCACTGAAGTCTCAATTG
AAATACCCATTGAAGGACGAAATCACTTTGGCTTTGAAGTTGCCATTGTACGACGA
GGTTAATCTATTAGGTGCCAGATACTACATCAAGATCTACGAAAAGTACTCATCACA
CAACAAGTTGTTATTAGAATTTGCCAAATTGGATGGTAACAGAATGCAAAAGCAAT
ACCAACACGAATTAGCTGAATTGGTCCGTGACTGGAAGGACTTGGACACTGAAAA
CAAATTACCATTTGCAAGAGACAGAATCGTCGAAGGTTACTTCAACATCTTGGGTA
TGTACGCTGAACCAGAACACAAGGACGTTAGAGAATTCTTGAACAGAGTTTTTGA
TCTTTTGTCTCACATTGATGACACCTACGTTGTTTACGGTACCTTCGAAGAAGTCAT
CTTGTTCACCAACGCCATTGAAAGATGGGAAGAATCTGCCCTCGACCAATTACCAG
AAGACATGAAGATCATCTACCAAGCTTTGATGGATGTCTACTCCCAGGAAGAAGAA
AAGTTGGCCAAGAAGGAAGATGCTCCAAAGTACAGATTGGAATTCGCTATCGAAA
CCGTCAAGGCCCTAACAAGATCTTACTTGGAAATTGCTAGATGGTCTAAGGAACGT
TACATTCCAACCGTTGAAGAATATATGTCTGTTGCTTTAGTTTCTTGTGGTGCTTCC
ACTATTTTGACTTGCTCCTTCGTCGGTATGGGTGATGAAGCCACCCGTGAAGTTTTC
GAATGGGTTTCTAGTTCCCCTTTGATTGTCAGAGCTTCTGACTTGATTAATGCTTTG
GGTAGAGATATCGGTTCCCACGAAGAAGAACAAAAGTGGGGATTGGCTGCTACTT
ACATAGAATGTTACATGAAAGAATATGGTGTTACCAAGCAAGAAGCTTACGCTGAA
 
GCTCAAAAGCAAATTGATAACGCTAAGAAAGACTTGAACCAAGAAGCCTTAAGAC
CATTCCCAGTTCCAAGAGAATACATTACCCGTGTTGTCAACTCTACCAGAACTATC
AGAGTGATCTATGCTGACAAGCACGACGGCTACACTCACCCAGAAAAGAAGATTA
AGGGTTTGATTCACTCCTTACTAGTTCAACCAATCCCAATGTGA.
(SEQ ID NO: 63) 
MEVKRRSANYKPSVWGDKFLEYSSPDALKADSKEAKKDAVTEKKLEKLKEEVAKML
VETVQKPQQALNLINEIQRLGLSFHFLPEIEAALRQISVTYDELCESLSVDDLDNVALC
LRILRSHGHDVSAGVFEKFMDSNGKLKGSFLNDAIGLLSLYEATHFRVHGEDLLESLL
SLTTSLLEALKSQLKYPLKDEITLALKLPLYDEVNRLGARYYIKIYEKYSSHNKLLLEF
AKLDGNRMQKMYQHELAELTRDWKDLDTENKLPFARDRIVEGYFNILGMYAEPEHK
DVREFLNRVFDLLSHIDDTYVVYGTFEEVILFTNAIERWETSALDQLPEDMKIIYQAL
MDVYSQEEEKLAKKEDAPKYRLEFAIETVKALTRSYLEIARWSKERYIPTVEEYMSVA
LVSCGASTILTCSFVGMGDEATREVFEWVSSSPLIVRASDLINALGRDIGSHEEEQKWG
LAATYIECYMKEYGVTKQEAYAEAQKQIDNAKKDLNQEALRPFPVPREYITRVVNFT
RAIRVIYADKHDGYTHPEKKIKGYITSLLVQPIPM.
(SEQ ID NO: 64) 
ATGGAAGTCAAGCGGAGATCTGCTAACTACAAGCCATCCGTCTGGGGTGACAAAT
TTTTGGAATATTCATCCCCAGACGCCTTGAAAGCTGATTCCAAGGAAGCCAAGAAG
GATGCTGTCACTGAAAAGAAATTAGAAAAATTAAAGGAAGAAGTTGCCAAGATGT
TGGTTGAAACTGTCCAAAAGCCACAACAAGCTTTAAACTTGATCAACGAAATCCA
AAGATTGGGTTTGTCTTTCCACTTTCTGCCAGAAATTGAAGCCGCTTTGAGACAAA
TCTCCGTTACTTACGACGAATTGTGTGAATCTTTGTCTGTCGATGACTTGGACAACG
TTGCATTGTGCTTGAGAATCTTACGTTCTCACGGTCACGATGTTTCCGCTGGTGTAT
TCGAAAAGTTCATGGACTCTAACGGTAAGTTAAAGGGTTCTTTCTTGAACGATGCC
ATTGGTTTATTGTCATTGTACGAAGCTACTCACTTCAGAGTTCACGGTGAAGACCTT
TTGGAAAGCCTCTTGTCTTTGACCACTTCCCTATTGGAAGCTTTGAAGTCCCAATT
GAAGTACCCATTGAAGGACGAAATCACCTTGGCCTTGAAGTTGCCATTATATGATG
AAGTCAACAGATTGGGTGCTAGATACTACATCAAGATCTACGAAAAGTACTCCTCT
CACAACAAGCTGTTGTTGGAGTTTGCTAAGCTCGACGGAAACAGAATGCAAAAGA
TGTACCAACACGAATTAGCTGAATTGACCAGAGATTGGAAAGACTTGGATACTGA
AAACAAGCTACCTTTCGCTAGAGACAGAATTGTCGAAGGTTACTTCAACATTTTGG
GTATGTATGCTGAACCAGAACACAAGGACGTTCGTGAATTCTTGAATCGTGTCTTC
GACTTGTTGTCTCACATTGACGACACTTACGTTGTTTACGGTACTTTCGAAGAAGTT
ATCTTGTTCACCAACGCTATCGAAAGATGGGAAACCAGTGCCTTGGACCAATTACC
 
TGAAGACATGAAAATTATCTACCAAGCTTTGATGGACGTCTACTCTCAAGAAGAAG
AAAAGTTGGCAAAGAAAGAAGATGCTCCAAAGTACAGATTAGAATTCGCTATTGA
AACCGTTAAGGCTTTAACTCGTTCCTACTTGGAAATCGCCAGATGGTCTAAGGAAA
GATACATTCCAACCGTCGAAGAATATATGTCTGTCGCTTTGGTTTCTTGTGGTGCCT
CCACTATCTTGACCTGTTCCTTCGTTGGTATGGGTGATGAAGCTACAAGAGAAGTT
TTTGAGTGGGTTAGCTCTTCTCCATTGATTGTTAGAGCTTCTGATCTAATTAACGCTT
TGGGTAGAGACATTGGTTCCCATGAAGAAGAACAAAAGTGGGGTTTGGCTGCTAC
CTACATCGAATGTTACATGAAGGAATACGGTGTCACCAAGCAAGAAGCTTACGCTG
AAGCTCAAAAGCAAATTGATAATGCTAAGAAGGATTTGAACCAAGAAGCCTTAAG
ACCATTCCCAGTTCCAAGAGAATACATTACTAGAGTTGTTAACTTCACCAGAGCCA
TCAGAGTCATCTACGCTGACAAGCATGATGGTTACACCCATCCAGAAAAAAAGATT
AAGGGTTACATCACTTCTTTATTGGTCCAACCAATCCCAATGTGA.
(SEQ ID NO: 67) 
MEVKRRSANYKPSVWGDKFLEYSSPDALKADSKEAKKDAVTEKKLEKLKEEVAKML
VETVQKPQQALNLINEIQRLGLSFHFEPEIEAALRQISVTYDELCESLSVDDLDNVALC
LRILRSHGHKVSAGVFEKFMDSNGKLKGSFLNDAIGLLSLYEATHFRVHGEDLLESLL
SLTTSRLEYLKSQLKYPLEDEITRALKLPLYDEVNRLGARYYIKIYEKYDSHNKLLLEF
AKLDGNRMQKMYQHELAELTRDWKDLDTENKLPFARDRIVEGYFNILGMYAEPEHK
DVREFLNRVFDLISHVDDTYVVYGTFEEVILFTNAIERWETSALDQLPEDMKIIYQAL
MDVYSQEEEKLAKKADAPKYRLEFAIETVKALTRSYLEIARWSKERYIPTVEEYMSVA
LVSCGASTILTCSFVGMGDEATREVFEWVSSSPLIVRASDLINALGRDIGSHEEEQKWG
LAATYVECYMKEYGVTKQEAYAEAQKQIDNAKKDLNQEALRPFPVPREYITRVVNFT
RAIRVIYADKHDGYTHPEKKIKGYITSLLVQPIPM.
(SEQ ID NO: 68) 
ATGGAAGTCAAGAGAAGATCCGCTAACTACAAGCCATCTGTCTGGGGTGACAAAT
TCTTAGAATATTCCTCTCCAGACGCTTTGAAGGCTGACTCCAAGGAAGCCAAAAA
GGATGCCGTTACTGAAAAGAAATTGGAAAAGTTAAAGGAAGAAGTTGCCAAGATG
CTGGTCGAAACTGTTCAAAAGCCACAACAAGCATTAAACTTGATTAACGAAATCC
AAAGATTGGGTTTGTCCTTCCACTTCGAACCAGAAATTGAAGCTGCCTTGAGACA
AATCTCTGTTACCTACGATGAATTGTGTGAATCATTGTCCGTTGACGATTTGGATAA
CGTTGCTCTATGCTTGAGAATCTTGAGATCTCACGGTCACAAGGTTTCCGCTGGTG
TTTTTGAAAAGTTCATGGATTCTAACGGTAAGTTGAAAGGTTCTTTCTTGAACGAT
GCTATTGGTTTGCTTTCCTTGTACGAAGCCACTCATTTCAGAGTTCACGGTGAAGAT
TTGTTGGAATCCTTGCTCTCTCTCACTACTTCCAGATTGGAATATTTGAAGTCCCAA
 
TTGAAGTACCCATTGGAAGACGAAATCACCAGAGCTCTAAAGTTACCTTTATACGA
CGAAGTTAACCGTTTGGGCGCCAGATACTACATCAAGATTTACGAAAAGTATGACT
CTCACAACAAGTTGTTGTTGGAATTCGCCAAATTGGATGGTAACAGAATGCAAAA
GATGTACCAACACGAATTAGCTGAATTAACCAGAGACTGGAAGGATTTGGACACC
GAAAACAAGTTGCCATTTGCTAGAGACAGAATTGTTGAAGGTTACTTCAACATCTT
GGGTATGTACGCTGAACCAGAACACAAAGACGTTAGAGAATTTTTAAATCGTGTCT
TCGATCTAATATCTCACGTCGATGACACTTACGTCGTCTACGGTACTTTCGAAGAAG
TCATTTTGTTCACCAACGCCATTGAAAGATGGGAAACCTCCGCTTTGGACCAATTG
CCAGAGGACATGAAAATCATCTACCAAGCTTTGATGGACGTTTACTCTCAAGAAGA
AGAAAAACTGGCTAAGAAGGCTGATGCCCCTAAGTACAGATTGGAATTCGCTATTG
AAACTGTCAAGGCCTTGACCCGTTCTTACTTGGAAATTGCTAGATGGTCCAAGGAA
AGATACATTCCAACCGTTGAAGAGTATATGTCTGTTGCTCTAGTCTCTTGTGGTGCT
TCTACAATCTTGACTTGTTCTTTCGTTGGTATGGGTGATGAAGCTACCCGTGAAGTC
TTCGAATGGGTTTCTTCCAGTCCATTGATTGTCAGAGCTTCTGACTTGATCAATGCT
TTAGGTAGAGATATCGGTAGCCACGAAGAAGAACAAAAGTGGGGTTTGGCTGCCA
CTTACGTCGAATGTTACATGAAGGAATACGGTGTCACCAAGCAAGAAGCTTACGCT
GAAGCTCAAAAGCAAATTGACAACGCTAAGAAGGACTTGAACCAAGAAGCTTTA
AGACCATTCCCAGTTCCAAGAGAATACATCACTCGTGTTGTCAACTTCACTAGAGC
TATCAGAGTTATCTACGCCGACAAGCATGACGGTTACACTCACCCAGAAAAGAAG
ATCAAGGGTTACATTACCTCTTTGTTGGTCCAACCAATCCCAATGTGA.
(SEQ ID NO: 71) 
MEVKRRSANYKPSVWGDKFLEYSSPDHLKNDSDELKKDAVTEKKLEKLKEEVAKML
VETVQKPQQALNLINEIQRLGLSFHFEPEIEAALRQISVTYHELCESLNVDDLDNVALC
LRILREHGHKVSAGVFEKFMDSNGKLKGSFLNDAIGLLSLYEATHFRVHGEDLLESLL
SLTTSRLEYLKSQLKYPLEDEITRALKLPLYKEVNRLGARYYIKIYEKYDSHNKLLLEF
AKLDGNRMQKMYQHELAELTRDWKDLDTENKLPFARDRIVEGYFNILGMYAEPECK
DVREFLNRVFDLISHVDDTYVVYGTFEEVILFTNAIERWETSALDQLPEDMKIIYQAL
MDVYSQEEEKLAKKADAPKYRLEFAIETVKALTRSYLEIARWSKERYIPTVEEYMSVA
LVSCGAKTILTCSFVGMGDEATREVFEWVSSSPLIVRASDLINALGDDIGSHEEEQKW
GLAATYVECYMKEYGVTKQEAYAEAQKQIDNAKKDLNQEALRPFPVPREYITRVVNF
TRAIRVIYADKHDGYTHPEKKIKGYITSLLVQPIPM.
(SEQ ID NO: 72) 
ATGGAAGTCAAGAGAAGATCCGCTAACTACAAGCCATCTGTCTGGGGTGACAAAT
TCTTAGAATACTCCTCTCCAGACCACTTGAAGAACGATTCCGATGAACTAAAGAAG
 
GATGCTGTCACTGAAAAGAAATTGGAAAAGTTGAAGGAAGAAGTTGCTAAGATGT
TGGTCGAAACAGTCCAAAAGCCTCAACAAGCCTTGAACTTGATCAACGAAATCCA
AAGATTAGGTTTGTCTTTCCACTTTGAACCAGAAATTGAAGCAGCTTTGAGACAAA
TCTCCGTCACCTACCACGAATTGTGTGAATCCCTGAATGTCGATGATTTGGACAAC
GTTGCCTTGTGTTTGAGAATCTTGCGTGAACACGGTCACAAGGTTTCTGCTGGTGT
CTTCGAAAAGTTCATGGACTCTAACGGTAAGTTGAAGGGTTCCTTCCTAAACGACG
CTATCGGTTTGTTGAGTTTGTACGAAGCCACTCACTTCAGAGTCCATGGTGAAGAC
TTGTTGGAATCTTTGTTGTCCTTGACCACCTCAAGATTGGAATACTTGAAGTCTCAA
TTGAAATACCCATTGGAAGATGAAATCACTAGAGCTTTAAAGTTACCTTTATACAAA
GAAGTTAACCGTTTGGGAGCTAGATACTATATCAAGATTTACGAAAAGTACGATTCT
CACAACAAGTTGTTGTTGGAATTCGCTAAATTGGATGGTAACAGAATGCAAAAGAT
GTACCAACACGAATTAGCTGAACTGACCAGAGACTGGAAGGACCTAGACACCGA
AAACAAATTGCCATTTGCTCGTGACAGAATTGTTGAAGGTTACTTCAACATTCTGG
GTATGTACGCTGAACCAGAATGTAAGGATGTTAGAGAATTCTTGAACAGAGTTTTC
GATTTGATTTCTCACGTTGACGACACTTACGTCGTCTACGGTACTTTCGAAGAAGT
CATCTTGTTCACCAACGCCATTGAAAGATGGAAACTTCTGCTTTGGACCAATTGC
CAGAAGACATGAAGATCATCTATCAAGCTTTGATGGATGTTTACTCTCAAGAAGAA
GAGAAGTTGGCCAAGAAGGCTGATGCTCCAAAGTACAGACTAGAATTTGCCATTG
AAACTGTTAAAGCATTGACCAGATCTTACTTGGAAATTGCTAGATGGTCTAAGGAA
CGTTACATTCCAACTGTTGAAGAATACATGTCCGTTGCTTTGGTTTCTTGTGGTGCT
AAGACCATCTTGACTTGCTCCTTCGTTGGTATGGGTGACGAAGCTACCCGCGAAGT
TTTCGAATGGGTTTCTTCTTCCCCATTGATTGTCAGAGCTTCTGACTTGATTAATGC
TTTGGGTGATGACATTGGTTCCCATGAAGAAGAACAAAAGTGGGGTTTAGCCGCT
ACTTACGTTGAATGTTACATGAAAGAATACGGTGTTACCAAGCAAGAAGCTTACGC
CGAAGCTCAAAAGCAAATTGACAATGCCAAGAAGGACTTAAACCAAGAAGCTCTA
AGACCATTCCCAGTTCCAAGAGAATATATCACTAGAGTCGTCAACTTCACTAGAGC
CATTCGTGTTATCTATGCTGACAAGCACGATGGCTACACTCATCCAGAAAAGAAGA
TCAAGGGTTACATCACCTCTTTATTGGTTCAACCAATCCCAATGTGA.
(SEQ ID NO: 75) 
MEVKRRSANYKPSVWGDKFLEYSSPDHLKNDSDELKKDAVTEKKLEKLKEEVAKML
VETVQKPQQALNLINEIQRLGLSFHFEPEIEAALRQISVTYHELCESLNVDDLDNVALC
FRILREHGHKVSAGVFHKFMDSNGKLKGSFLNDAIGLLSLYEATHFRVHGEDLLEDLL
SLTTSRLEYLKSQMKYPLEDEITRALKHPLYKEVNRLGARYYIKIYEKYDSHNKLLLEF
AKLDGNRMQKMYQHELAELTRDWKDLDTTNKLPFARDRIVEGYFNILGMYAEPECK
 
DVREFLNRVFALISHVDDTYDVYGTFEEVILFTNAIERWETSALDQLPEDMKIIYQAL
MDVYSQEEEKLAKKADAPTYRLEFAIETVKALTRSYLEIARWSKERYIPTVEEYMSVA
LVSCGAKTILTCSFVGMGDEATREVFEWVSSSPLIVRASDLINALGDDIGSHEEEQKW
GDAATYVECYMKEHGVTKQEAYAEAQKQIDNAKKDLNQEALRPFPVPRTYITRVVN
FTRAIRVIYADKHDGYTHPEKKIKGYITSLLVQPIPM.
(SEQ ID NO: 76) 
ATGGAAGTGAAGAGAAGATCTGCCAACTACAAGCCTTCTGTCTGGGGTGACAAAT
TCTTAGAATACTCTTCCCCAGACCACCTCAAGAACGACTCCGATGAATTGAAGAAG
GACGCTGTTACTGAAAAAAAATTAGAAAAGTTGAAGGAAGAAGTCGCTAAGATGT
TGGTCGAAACCGTCCAAAAGCCACAACAAGCTTTGAACTTAATCAACGAAATCCA
AAGATTAGGTTTGTCTTTCCATTTCGAACCAGAAATTGAAGCTGCTTTGAGACAAA
TCTCTGTTACTTACCATGAATTGTGTGAATCCTTGAATGTCGATGATTTGGACAACG
TTGCTTTATGTTTCAGAATCCTACGTGAACACGGACACAAAGTTTCCGCTGGTGTC
TTCCACAAGTTCATGGACTCAAACGGTAAGTTGAAGGGTTCTTTTTTGAACGATGC
TATTGGTCTTTTGTCCTTGTATGAAGCCACCCACTTCAGAGTCCATGGTGAAGATTT
GCTTGAAGACTTGTTATCATTGACCACCTCCAGATTGGAATATTTGAAGTCTCAAAT
GAAGTACCCACTGGAAGATGAAATCACGAGAGCTTTGAAGCATCCATTGTACAAG
GAAGTCAACAGACTAGGTGCCAGATACTACATCAAGATTTACGAAAAGTACGATTC
CCACAACAAGTTGTTGTTGGAATTTGCTAAATTGGATGGTAACAGAATGCAAAAGA
TGTACCAACACGAACTGGCCGAATTGACCCGTGACTGGAAGGATTTAGACACCAC
TAACAAGCTACCTTTTGCCAGAGACAGAATTGTTGAAGGTTACTTCAACATTTTGG
GTATGTATGCTGAACCAGAATGCAAAGATGTTAGAGAATTCTTGAACAGAGTTTTC
GCTTTGATATCTCACGTTGATGACACTTACGATGTTTACGGTACTTTCGAAGAAGTT
ATCTTGTTCACCAACGCCATCGAAAGATGGGAAACTTCTGCCTTGGACCAATTGCC
AGAAGACATGAAGATTATCTACCAAGCTTTGATGGATGTCTACTCCCAAGAAGAGG
AAAAGTTGGCTAAGAAGGCTGATGCTCCAACCTACCGTTTGGAATTCGCTATCGAA
ACAGTCAAGGCTCTTACTAGGTCTTACTTGGAAATTGCTAGATGGTCTAAGGAACG
TTACATTCCAACTGTTGAAGAATACATGTCCGTTGCTTTGGTATCTTGTGGCGCCAA
GACCATTTTGACTTGTTCTTTCGTTGGTATGGGTGACGAGGCTACCAGAGAAGTCT
TTGAATGGGTTTCTTCCTCTCCATTGATTGTCAGAGCCTCTGACTTGATTAACGCTT
TGGGTGATGACATCGGTTCGCACGAAGAAGAACAAAAATGGGGTGACGCAGCTAC
TTACGTCGAATGTTACATGAAAGAACACGGTGTCACCAAGCAAGAAGCTTACGCT
GAAGCTCAAAAGCAGATTGACAATGCTAAGAAGGATCTGAACCAAGAAGCCTTAA
GACCATTCCCAGTTCCAAGAACCTATATTACTAGAGTTGTTAACTTCACTAGAGCTA
 
TCAGAGTCATTTACGCTGACAAGCACGACGGTTACACTCACCCAGAAAAGAAGAT
CAAGGGTTACATCACTTCCTTGTTGGTTCAACCAATCCCAATGTGA.
(SEQ ID NO: 79) 
MEVKRRSANYKPSVWGDKFLEYSSPDHLKNDSDELKKDAVTEKKLEKLKEEVAKML
VETVQKPQQALNLINEIQRLGLSFHFEPEIEAALRQISVTYHELCESTNVDDLDNVALC
FRILREHGHKVSADVFHKFMDSNGKLKGSFLNDAIGLLSLYEATHFRVHGEDLLEDLL
SLTTSRLEYLKSQMKYPLEDEITRALKHPLYKELNRLGARYYISIYEKYDSHNKLLLEF
AKLDGNRMQKMYQHELAELTRDWKDLDTTNKLPFARDRIVEGYFNILGMYFEPECK
DVREFLNRVFALISHVDDTYDVYGTFEEVILFTHAIERWETSALDQLPEYMKIIYQAL
MDVYSQMEEKLAKKADAPTYRLEFAIETVKALFRSYLEIARWSKERYIPTVEEYMSV
ALVSCGAKTILTCSFVGMGDTATREVFEWVSSSPLIVRASDLINALGDDIGSHEEEQK
WGDAATYVECYMKEHGVTKQEAYAEAQKQIDNAKKDLNQEALRPFPVPRTYITRVV
NFTRAIRVIYADSHDGYTHPEKKIKGYITSLLVQPIPM.
(SEQ ID NO: 80) 
ATGGAAGTTAAGAGAAGATCTGCTAACTACAAGCCATCTGTCTGGGGTGACAAATT
CTTGGAATACTCTTCTCCAGACCACTTGAAGAACGACTCTGATGAATTGAAGAAG
GATGCTGTCACTGAAAAGAAATTGGAAAAATTAAAGGAAGAAGTTGCCAAGATGT
TGGTTGAAACTGTTCAAAAGCCACAACAAGCCTTAAACTTGATCAACGAAATCCA
AAGACTAGGTTTGTCCTTCCATTTCGAACCTGAAATTGAAGCTGCCTTGAGACAAA
TTTCCGTTACTTACCACGAATTGTGTGAATCTACTAACGTCGACGATTTGGATAACG
TCGCTCTCTGTTTCAGAATCTTACGTGAACATGGTCACAAGGTTTCCGCTGATGTCT
TCCACAAGTTCATGGACTCTAACGGTAAGTTGAAGGGTTCTTTCTTGAACGATGCA
ATTGGCCTATTATCATTGTACGAAGCTACTCACTTCAGAGTCCACGGTGAAGATTTA
TTGGAAGACTTGTTGAGCTTGACTACTTCTAGATTGGAATACTTGAAGTCTCAAAT
GAAGTATCCATTGGAAGACGAAATCACCAGAGCTCTTAAGCACCCATTATACAAGG
AATTGAACCGTTTAGGTGCTCGTTACTACATCTCGATCTACGAAAAGTACGATTCTC
ACAACAAGTTGTTGCTCGAATTCGCTAAGTTGGACGGTAACAGAATGCAAAAGAT
GTACCAACACGAATTGGCTGAATTAACTAGAGACTGGAAGGACTTGGATACCACC
AACAAGTTGCCATTCGCTAGAGACAGAATTGTCGAAGGTTACTTCAACATTTTGGG
TATGTATTTTGAACCTGAATGTAAGGATGTTAGAGAGTTCTTGAATAGAGTTTTTGC
CTTGATTTCCCATGTCGACGACACTTACGACGTTTACGGTACTTTCGAGGAGGTCA
TCTTGTTCACCCACGCAATTGAAAGATGGGAAACATCTGCCTTAGACCAATTGCCA
GAATATATGAAAATCATCTACCAAGCTTTGATGGATGTCTACTCCCAAATGGAAGAA
AAATTGGCCAAAAAAGCTGATGCTCCAACCTACAGATTGGAATTTGCTATCGAAAC
 
CGTCAAGGCTTTGTTTCGTTCTTACTTGGAAATTGCCAGATGGTCTAAGGAAAGAT
ACATTCCAACTGTCGAAGAATACATGTCTGTGGCTTTGGTTTCTTGCGGTGCTAAG
ACCATCTTGACCTGTTCCTTCGTCGGTATGGGTGACACTGCCACGAGAGAAGTTTT
CGAATGGGTTTCTAGCTCTCCATTGATTGTTCGCGCTTCCGATTTGATCAATGCTTT
GGGTGATGACATTGGTTCCCACGAAGAAGAACAAAAATGGGGTGACGCTGCTACC
TACGTTGAATGTTACATGAAGGAACACGGTGTTACCAAGCAAGAAGCTTACGCTG
AAGCTCAAAAGCAAATCGACAACGCTAAGAAGGACTTGAACCAAGAAGCTCTAA
GACCATTCCCAGTTCCAAGAACTTACATTACCAGAGTTGTTAACTTCACTCGTGCC
ATCAGAGTCATTTACGCTGACTCCCACGATGGTTACACCCATCCAGAAAAGAAGAT
CAAGGGTTATATTACCTCCTTGCTGGTCCAACCAATCCCAATGTGA.
(SEQ ID NO: 83) 
MEVTRRSANYHPSVWGDKFLEYSSPDHLKNDSDELKNDAVTEKKLEKLKEEVKKML
VETVQKPQQALNLINEIQRLGLSFHFEPEIEAALRQISVTYHELCESTNVDDLDNVALC
FRILREHGHKVSADVFHKFMDSNGKLKGSFLNDAIGLLSLYEATHFRVHGEDLLEDLL
SLTTSRLEYLKSQMKYPLEDEITRALKHPLYKELNRLGARYYISIYEKYDSHNKLLLEF
AKLDGNRMQKMYQHELAELTRDWKDLDFTNKLPFARDRIVEGYFNILGMYFEPECK
DVREFLNRVFALISHVDDTYDVYGTFEEVILFTHAIERWETSALDQLPEYMKIIYQAL
MDVYSQMEEKLAKKADAPTYRLEFAIETVKALFRSYLEIARWSKERYIPTMEEYMSV
ALVSCGAKTILTNSFVGMGDTATREVFEWVSSSPLIVRASDLINALGDDIGGHEEEQK
WGDAATAVECYMKEHGVTKQEAYAEAQKQIDNAKKDLNQEALRPFPVPMTYITRVV
NFTRAIRVIYADSDDGYTHPEKKIKGYITSLLVQPIPM.
(SEQ ID NO: 84) 
ATGGAAGTTACTAGAAGATCCGCTAACTATCATCCATCTGTCTGGGGTGACAAATTC
TTGGAATACTCTTCTCCAGACCATTTGAAGAATGATAGCGACGAATTGAAGAACGA
CGCTGTCACTGAAAAGAAGCTGGAAAAACTTAAGGAAGAAGTCAAGAAGATGTT
GGTGGAAACCGTCCAAAAGCCACAACAAGCTTTGAACTTGATCAACGAAATCCAA
AGATTGGGTTTGAGTTTCCACTTCGAGCCTGAAATTGAAGCTGCTTTGAGACAAAT
CTCTGTTACCTACCACGAATTGTGCGAATCCACCAACGTCGATGATTTAGACAACG
TTGCTTTGTGTTTCAGAATTCTCAGAGAACACGGTCACAAGGTTTCTGCTGATGTT
TTCCACAAGTTCATGGACTCCAACGGTAAGTTGAAGGGTTCTTTCTTGAACGATGC
TATCGGTTTGTTGTCCTTGTACGAGGCCACCCACTTCCGTGTCCATGGTGAAGACTT
ATTGGAAGATTTGTTGTCTTTGACTACTTCCAGATTGGAATACTTGAAATCCCAAAT
GAAGTACCCATTGGAAGATGAAATCACCAGAGCTTTAAAGCACCCATTGTACAAG
GAATTAAATAGATTAGGTGCTAGATACTACATCTCGATTTACGAAAAGTATGATTCT
 
CACAACAAATTGTTGCTGGAATTCGCTAAGTTGGATGGTAACAGAATGCAAAAGAT
GTACCAACACGAATTGGCTGAACTTACACGTGACTGGAAGGACTTGGACTTCACT
AACAAGCTACCATTCGCCAGAGACAGAATCGTTGAAGGTTACTTCAACATCTTGGG
TATGTATTTTGAACCAGAATGTAAGGATGTTAGAGAATTCTTAAACAGAGTTTTTGC
CTTGATTTCCCACGTTGACGACACTTACGACGTCTACGGTACTTTCGAGGAAGTTA
TCTTGTTCACCCATGCCATTGAAAGATGGGAAACTTCTGCCTTGGACCAATTGCCA
GAATATATGAAGATCATTTACCAAGCTTTGATGGACGTTTACTCCCAAATGGAAGAA
AAGTTGGCTAAGAAGGCTGATGCTCCAACTTACAGATTGGAATTTGCTATCGAAAC
TGTCAAGGCCTTATTCAGATCTTACTTGGAAATTGCTAGATGGTCTAAGGAACGTTA
CATTCCAACCATGGAAGAATACATGTCTGTCGCTTTGGTTTCTTGTGGTGCCAAGA
CCATTTTGACTAACTCATTCGTTGGTATGGGTGACACTGCTACTCGTGAAGTCTTTG
AATGGGTTTCTTCCTCCCCTTTGATCGTCAGAGCTTCTGACTTGATCAATGCTCTGG
GTGACGATATTGGTGGTCACGAAGAAGAACAAAAGTGGGGTGATGCCGCTACCGC
CGTCGAATGTTACATGAAAGAACACGGTGTTACCAAGCAAGAAGCTTACGCTGAG
GCTCAAAAGCAAATTGACAACGCTAAGAAGGATTTGAACCAAGAAGCCTTAAGAC
CATTCCCAGTTCCAATGACCTACATTACCCGTGTTGTTAACTTCACCAGAGCTATCA
GAGTCATTTACGCTGACTCTGATGACGGTTACACTCACCCAGAAAAAAAAATCAA
GGGCTACATCACTTCTTTGTTAGTTCAACCAATTCCAATGTGA.
(SEQ ID NO: 87) 
MEVTRRSANYHPSVWGDKFLEYSSPDHLKNDSDHLKNDAVTEKKLEKLKEEVKKML
VETVQKPQQALNLINEIQRLGLSFHFEPEIEAALRQISVTYHELCESTNVDDLDNVALC
FRILREHGHKVSCDVFHKFMDSNGKLKGSFLNDAIGLLSLYEATHFRVHGEDLLEDLL
SLTTSRLEYLKSQMKYPLEDEITRALKHPLHKELNRLGARYYISIYEKYDSHNKLLLEF
AKLDGNRMQKMYQHELAELTRWWKDLDFTNKLPFARDRIVEGYFNILGMYFEPECK
DVREFLNRVFALISHVDDTYDVYGTFEEVILFTHAIERWETSALDQLPEYMKIIYQAL
MDVYSQMEEKLAKKADAPTYRLEFAIETVKALFRSYLEIARWSKERYIPTMEEYMTV
ALVSCGAKTILTNSFVGMGDTATREVFEWVSSSPLIVRASDLINALGDDIGGHEEEQK
KGDAATAVECYMKEHGVTKQEAYAEFQKQIDNAWKDLNQEALRPFPVPMTYITRVV
HFTRAIHVIYADSDDGYTHPEKKIKGYITSLLVDPIPM.
(SEQ ID NO: 88) 
ATGGAAGTCACCAGAAGATCCGCTAACTACCATCCATCTGTCTGGGGTGACAAGTT
CTTGGAATACTCCTCTCCAGACCATTTGAAAAATGATTCAGACCACTTGAAGAACG
ATGCTGTCACTGAAAAGAAATTGGAGAAATTGAAGGAAGAAGTTAAGAAGATGTT
GGTCGAAACTGTTCAAAAGCCACAACAGGCTTTGAACTTGATCAACGAAATCCAA
 
AGATTAGGTTTGTCCTTCCACTTCGAACCTGAAATTGAAGCAGCTTTGAGACAAAT
CTCTGTCACTTACCACGAATTGTGTGAATCTACCAACGTCGATGATTTGGACAACG
TTGCTCTTTGTTTCAGAATCTTGAGAGAACATGGTCACAAGGTTTCTTGTGATGTTT
TCCATAAATTCATGGACTCTAACGGTAAGTTGAAAGGTTCTTTCTTAAACGACGCTA
TCGGTTTGTTGTCCTTGTACGAAGCTACCCACTTCAGAGTTCACGGTGAAGATTTG
TTGGAAGACTTATTGTCTTTGACCACATCCAGATTAGAATACTTGAAGTCTCAAATG
AAGTACCCATTGGAAGATGAAATCACCAGAGCCCTTAAACACCCATTGCACAAGG
AATTGAACAGATTGGGGGCTAGATACTACATCTCCATCTATGAAAAGTACGACTCC
CACAACAAGTTGTTGTTGGAATTTGCTAAGTTAGATGGTAACAGAATGCAAAAGAT
GTACCAACACGAACTGGCTGAATTGACCAGATGGTGGAAGGACCTAGACTTCACC
AACAAGTTGCCATTCGCCAGAGACAGAATTGTTGAAGGTTACTTCAACATTCTCGG
TATGTATTTCGAACCAGAATGTAAGGATGTTAGAGAATTCTTGAACCGTGTTTTTGC
CTTAATTTCTCACGTTGATGACACTTACGATGTTTACGGTACTTTCGAAGAAGTTAT
CTTATTCACCCACGCCATTGAAAGGTGGGAAACCTCTGCTTTGGACCAATTGCCAG
AATATATGAAGATTATCTACCAAGCTTTGATGGATGTCTACTCTCAAATGGAAGAGA
AGTTGGCCAAGAAGGCTGATGCTCCAACTTACAGATTGGAATTCGCTATCGAAACC
GTTAAGGCCTTATTTAGATCTTACTTAGAAATTGCTCGTTGGAGTAAGGAAAGATAC
ATCCCAACCATGGAAGAATACATGACTGTCGCTCTAGTCTCCTGCGGTGCTAAGAC
TATCTTGACTAACTCTTTTGTCGGTATGGGTGACACCGCCACTCGTGAAGTCTTCG
AATGGGTTTCCTCTTCTCCATTGATTGTCAGAGCTTCAGATTTGATTAATGCTTTGG
GTGATGATATCGGTGGTCACGAAGAAGAACAAAAGAAGGGTGACGCTGCTACTGC
TGTCGAATGTTACATGAAAGAACACGGTGTCACCAAGCAAGAAGCTTACGCCGAA
TTCCAAAAGCAAATTGACAACGCTTGGAAGGACTTGAACCAAGAAGCCTTGCGTC
CTTTCCCAGTTCCAATGACCTACATCACTCGTGTTGTGCATTTCACTAGAGCTATTC
ACGTCATTTACGCTGACTCCGACGACGGTTATACTCACCCAGAAAAGAAGATCAA
GGGTTACATTACTTCTCTGTTGGTTGACCCAATTCCAATGTGA.
(SEQ ID NO: 91) 
MEVTRRSANYHPSVWGDKFLEYSSPDHLKNDSDHLKNDAVTEKKHEKLKEEVKKM
LVETVQKPQQALNLINEIQRLGLSFHFEPEIEAALRQISVTYHEYCCSTNVDDLHNVAL
CFRILREHGHKVSCDVFHKFMDSNGKLKGSFLNDAIGLLSLYEATHFRVHGEDLLEDL
LSLTTSRLEYLKSQMKYPLEDEITRALKHPLHKELNRLGARYYISIYEKYDSHNKLLLE
FAKLDGNRMQKMYQHELAELTRWWKDLDFTNKLPFARDRIVEGYFWILGMYFEPEC
KDVREFLNRVFALISHVDDTYDVYGTFEEVILFTHAIERWETSALDQLPEYMKIIYQAL
MDVYSQMEEKLAKKADCPTYRLEFAIETVKALFRSYLEIARWSKERYIPTMEEYMTV
 
ALVSVGYKTILTNSFVGMGDTATREVFEWVFSSPLIVRASDLIAALGDDIGGHEEEQK
KGDAATAVECYMKEHGVTKQEAYAEFQKQIDNAWKDLNQEALRPFPVPMTYITRVV
HFTRAIHVIYADSDDGYTRPEKKIKGYITSLLVDPIPM.
(SEQ ID NO: 92) 
ATGGAAGTCACTAGAAGATCTGCTAACTACCATCCATCTGTCTGGGGTGACAAGTT
CTTAGAATACTCTTCTCCAGACCACTTGAAGAACGATTCTGACCATTTGAAGAACG
ACGCTGTCACCGAAAAGAAGCACGAAAAGTTGAAAGAAGAAGTCAAGAAGATGT
TGGTTGAAACTGTCCAAAAGCCACAACAAGCTTTGAACTTGATCAACGAAATCCA
AAGATTGGGTTTGTCTTTCCACTTCGAACCTGAAATTGAAGCGGCTTTGAGACAAA
TCTCAGTTACTTACCACGAATACTGTTGTTCCACTAATGTCGACGATTTGCACAACG
TTGCCCTTTGTTTCAGAATTTTAAGAGAACACGGTCACAAGGTTTCTTGCGATGTT
TTCCATAAATTCATGGATTCCAACGGTAAGTTGAAGGGTTCCTTTTTGAATGATGCT
ATTGGTCTGTTGTCTTTGTACGAAGCTACTCATTTCAGAGTCCACGGTGAAGATTTG
TTGGAAGATCTCTTATCCTTGACCACTTCTAGATTGGAATACTTGAAGTCCCAAATG
AAATACCCATTGGAAGATGAAATCACCAGAGCTCTAAAACACCCATTACACAAGG
AATTGAACCGCTTGGGTGCTCGTTACTACATTTCTATCTACGAAAAGTACGACTCTC
ACAACAAATTGCTTTTGGAATTTGCTAAGTTGGATGGTAACAGAATGCAAAAGATG
TACCAACACGAATTGGCTGAATTGACTAGATGGTGGAAAGACTTGGATTTCACAAA
CAAGTTGCCATTCGCTAGAGACAGAATCGTGGAAGGTTACTTCTGGATTCTTGGTA
TGTATTTTGAACCAGAATGTAAAGACGTCAGAGAATTCTTGAACAGAGTTTTTGCC
TTGATCTCCCACGTCGATGATACTTACGATGTCTACGGTACTTTCGAAGAAGTTATC
TTGTTCACTCACGCTATTGAACGTTGGGAAACCTCTGCCTTAGACCAATTGCCAGA
ATACATGAAGATTATCTACCAAGCTTTGATGGACGTTTACTCTCAAATGGAAGAAA
AGTTGGCCAAGAAGGCTGACTGTCCAACCTACAGATTGGAATTCGCTATCGAAACT
GTTAAGGCCTTGTTTCGTAGTTACTTGGAAATTGCCAGATGGTCTAAGGAAAGATA
CATCCCAACCATGGAAGAATATATGACTGTTGCTTTAGTCTCCGTCGGTTACAAGAC
CATCTTGACTAACTCTTTCGTTGGTATGGGTGACACTGCTACCAGAGAAGTCTTCG
AATGGGTTTTCTCCTCCCCATTAATTGTTAGAGCTTCTGACTTGATTGCTGCCTTGG
GTGATGACATCGGTGGACACGAAGAAGAACAAAAGAAGGGTGACGCTGCTACCG
CCGTCGAATGTTACATGAAGGAACATGGTGTTACCAAGCAAGAAGCTTACGCTGA
ATTCCAAAAGCAAATTGACAACGCTTGGAAGGATTTAAACCAAGAAGCTTTGCGT
CCTTTCCCAGTTCCAATGACCTATATCACTAGAGTTGTTCACTTCACCAGAGCCATT
CACGTTATCTATGCTGATTCCGACGACGGTTACACCAGACCAGAAAAAAAGATCAA
GGGCTACATCACATCCCTATTGGTTGACCCAATTCCAATGTGA.
(SEQ ID NO: 95) 
MSLLVLSTSGSAPLTKKLEVKRRSANYKPSVWGDKFLEYQSPDALKADAEEAKKDA
VEEKKLEKLSEEVAKMLVEHVHKPQQALNLINEIERLGLSHHFLPEIEAALRQISVTYE
ELCRSLSVDDLSNVALCLRILREHGHDVSASVFTKFMDSNGKLKASFLNDAIGLLSLY
EATHFRVAGEDLLESLLSLTTSLLEALKSQLKYPLKDEIRMALKSPLAKEINRLGARYY
IKIYEKYSSHNKLLLEFAILDGNRMQKQYQRELAELTRDWKDLDTDNKLPFARDNIVS
GFFLIYGMYSEPEMKDVREFLNRVFDLISHVDDYYTVYGTFEEVILFTEAFERWTWSA
LDQLPEDMKIIFQALMDVYSQEEEKLAKKDDAPKYRLEFAIETVKALTRSLLEIARWS
KEREIPTLEEYMKVALVSCGFKTILTCSFVGMGTRATREVFEWVSSSPLIVRASELIAM
LGDDIGAHEEEQKEGDAATAIECYMKEYGVTKEEAYAEFQKEIDEAWKDLNQEALRP
FPVPWSYIDRVVNLTRAWHRIYADDHDGYTYPEKKIKGWINSLFHTPIPA.
(SEQ ID NO: 96) 
ATGTCTCTCTTGGTCTTGTCCACATCCGGTTCTGCTCCATTGACCAAGAAGTTGGA
AGTCAAGAGAAGATCTGCTAACTACAAGCCATCTGTCTGGGGTGACAAGTTCTTG
GAGTACCAATCTCCAGACGCATTGAAGGCTGACGCTGAAGAAGCTAAGAAAGATG
CTGTCGAAGAAAAGAAGTTGGAAAAATTGTCTGAAGAAGTTGCTAAGATGTTGGT
TGAACACGTCCACAAGCCACAACAAGCTTTGAATTTGATCAACGAAATTGAACGT
TTGGGTTTGTCTCATCACTTTTTGCCAGAAATCGAAGCCGCCTTGAGACAAATTTC
CGTCACTTACGAAGAATTGTGTCGTTCTTTGAGTGTCGACGACTTATCTAACGTTG
CTTTATGCTTGAGAATCCTGCGTGAACACGGTCACGATGTCTCCGCCTCTGTTTTCA
CCAAATTCATGGACTCCAACGGTAAGTTGAAGGCTTCTTTCTTGAACGATGCTATC
GGTTTGCTTTCCTTGTACGAAGCTACTCATTTCAGAGTTGCCGGTGAAGATTTGTTG
GAATCTTTATTGTCTTTGACCACTTCCTTGTTAGAAGCCTTGAAGTCCCAATTGAAG
TACCCATTGAAAGATGAAATCAGAATGGCTTTGAAGTCCCCTCTGGCCAAGGAAAT
CAACAGATTGGGTGCTAGATACTACATCAAGATCTACGAAAAGTACTCCTCTCACA
ACAAGTTATTGTTGGAATTCGCCATTTTGGATGGTAACAGAATGCAAAAGCAATAC
CAAAGAGAATTGGCTGAATTAACCAGAGACTGGAAGGACCTAGACACTGACAAC
AAGTTGCCATTTGCCAGAGATAACATCGTCTCTGGTTTCTTCTTGATTTACGGCATG
TACTCTGAACCAGAAATGAAGGATGTCAGAGAGTTCTTAAATAGAGTCTTCGACCT
GATCTCCCACGTTGATGACTATTACACTGTTTACGGTACCTTTGAAGAAGTTATCTT
GTTCACTGAAGCTTTCGAAAGATGGACCTGGTCTGCCTTGGACCAATTACCAGAA
GACATGAAAATCATCTTCCAAGCTTTGATGGATGTCTACTCTCAAGAAGAAGAAAA
GCTCGCTAAAAAGGATGATGCTCCAAAGTACAGATTGGAATTCGCTATTGAAACCG
TTAAGGCTTTGACCAGATCCTTGCTCGAAATCGCTAGATGGTCCAAGGAAAGAGA
 
AATCCCAACTTTGGAAGAATATATGAAGGTTGCTTTAGTTTCTTGTGGTTTCAAGAC
CATCTTGACTTGTTCTTTCGTTGGTATGGGTACTCGTGCTACCAGAGAAGTCTTCGA
ATGGGTTTCATCTTCTCCTTTGATTGTCAGAGCTTCCGAATTGATCGCTATGTTGGG
TGACGATATTGGTGCTCACGAAGAAGAACAAAAGGAAGGTGATGCTGCAACTGCC
ATTGAATGTTACATGAAGGAATACGGTGTTACCAAGGAAGAAGCTTACGCTGAATT
TCAAAAGGAAATTGACGAAGCCTGGAAAGATTTGAACCAAGAAGCCCTCAGACC
ATTCCCAGTTCCATGGTCTTACATTGACCGTGTTGTTAACTTGACTAGAGCTTGGCA
CAGGATTTATGCTGACGACCATGACGGTTACACCTACCCAGAAAAGAAGATTAAGG
GTTGGATTAACTCTTTATTCCACACTCCAATTCCAGCTTGA.
(SEQ ID NO: 99) 
MSLLVLSTSGSAPLTKSLEVKRRSANYKPSVWGDKFLEYSSPDALKADAEEAKKDAV
EEKKLEKLKEEVAKMLVETVHKPQQALNLINEIQRLGLSHHFLPEIEAALRQISVTYEE
LCRSLSVDDLSNVALCLRILREHGHDVSASVFTKFMDSNGKLKASFLNDAIGLLSLYE
ATHFRVAGEDLLESLLSLTTSLLEALKSQLKYPLEDEIRRALKSPLAKEINRLGARYYIK
IYEKYSSHNKLLLEFAILDGNRMQKQYQRELAELTRDWKDLDFDNKLPFARDNIVEG
FFLIYGMYSEPEMKDVREFLNRVFDLISHVDDYYTVYGTFEEVILFTEAFERWTWSAL
DQLPEDMKIIFQALMDVYSQEEEKLAKKADAPKYRLEFAIETVKALTRSLLEIARWSK
ERYIPTMEEYMKVALVSCGFKTILTCSFVGMGTRATREVFEWVSSSPLIVRASELIAML
GDDIGAHEEEQKEGDAATAIECYMKEYGVTKQEAYAEFQKQIDNAWKDLNQEALRP
FPVPWSYIDRVVNLTRAWHRIYADDHDGYTYPEKKIKGWITSLFVTPIPA.
(SEQ ID NO: 100) 
ATGTCTCTATTGGTCTTGTCTACCTCTGGTTCTGCTCCATTGACTAAGAGCTTAGAA
GTCAAGAGAAGATCCGCTAACTACAAGCCATCTGTCTGGGGTGACAAGTTTTTGG
AATATAGCTCTCCAGATGCTTTAAAGGCTGATGCTGAAGAAGCCAAGAAGGATGCT
GTTGAAGAAAAAAAATTAGAAAAGTTGAAGGAAGAAGTGGCTAAGATGTTGGTG
GAAACTGTCCACAAGCCACAACAAGCTCTGAACTTGATCAACGAAATCCAAAGAT
TGGGTCTATCCCATCATTTCTTGCCAGAAATTGAAGCTGCTTTGCGTCAAATTTCCG
TTACTTACGAAGAATTATGCAGATCTTTGTCAGTCGATGATTTGTCCAACGTTGCTT
TGTGTTTGCGTATTTTGAGAGAACACGGTCACGATGTCTCTGCTTCCGTCTTCACC
AAGTTCATGGATTCTAACGGTAAGTTAAAGGCTTCTTTCTTGAATGACGCTATTGGT
TTGTTGTCTTTGTACGAAGCTACCCACTTCAGAGTCGCTGGTGAAGACTTGTTGGA
ATCTTTGCTCAGTTTGACTACCTCCTTGTTGGAAGCCCTTAAATCTCAATTGAAATA
CCCATTGGAAGATGAAATCAGAAGAGCCTTGAAGTCTCCATTGGCTAAGGAAATC
AACCGGTTAGGTGCCAGATACTACATTAAGATTTACGAAAAGTACTCATCTCACAA
 
CAAGTTGTTACTCGAATTCGCCATATTGGATGGTAACAGAATGCAAAAGCAATACC
AAAGAGAATTGGCTGAATTGACACGTGACTGGAAGGATCTAGATTTCGATAATAAG
TTGCCTTTTGCCAGAGACAACATTGTCGAAGGTTTCTTCTTGATTTATGGTATGTAC
TCTGAACCAGAAATGAAGGACGTTAGAGAATTTTTGAACAGAGTTTTCGACTTGAT
CTCCCATGTTGACGACTACTACACCGTTTACGGTACTTTCGAAGAAGTTATCCTATT
CACTGAAGCCTTCGAAAGATGGACCTGGTCCGCCTTGGACCAATTGCCAGAGGAC
ATGAAAATCATCTTCCAAGCCTTGATGGACGTCTACTCCCAAGAAGAAGAAAAGCT
AGCTAAGAAGGCTGACGCCCCAAAGTACAGATTGGAATTCGCTATTGAAACTGTC
AAAGCTTTGACCAGATCTTTATTGGAAATCGCTCGTTGGTCTAAGGAAAGATACAT
TCCAACCATGGAAGAATATATGAAGGTTGCTTTGGTTTCTTGTGGTTTCAAGACCAT
CTTGACTTGTTCCTTCGTTGGTATGGGTACCAGAGCTACTAGGGAAGTTTTTGAATG
GGTTTCCTCTTCCCCTCTGATCGTCAGAGCCTCCGAATTGATTGCTATGTTGGGCGA
TGACATCGGTGCTCACGAAGAAGAACAAAAAGAAGGTGATGCTGCTACTGCTATC
GAATGTTACATGAAGGAATACGGTGTTACCAAGCAAGAAGCTTACGCCGAATTCCA
AAAACAAATTGACAACGCTTGGAAGGACTTGAACCAAGAAGCATTAAGACCATTC
CCAGTTCCATGGTCTTACATCGACAGAGTTGTTAACCTAACTAGAGCTTGGCACAG
AATCTACGCTGACGACCACGACGGTTACACCTACCCAGAAAAGAAGATCAAGGGT
TGGATTACTTCTTTGTTCGTCACTCCAATCCCAGCTTGA.
(SEQ ID NO: 103) 
MSLLVLSTSGSAPLTKSSEVKRRSANYKPSVWGDKFLEYSSPDALKADSEEAKKDAV
EEKKLEKLKEEVAKMLVETVQKPQQALNLINEIQRLGLSHHFLPEIEAALRQISVTYEE
LCRSLSVDDLSNVALCFRILREHGHDVSASVFTKFMDSNGKLKGSFLNDAIGLLSLYE
ATHFRVAGEDLLESLLSLTTSRLEALKSQLKYPLEDEITRALKSPLAKEINRLGARYYIK
IYEKYSSHNKLLLEFAILDGNRMQKQYQRELAELTRDWKDLDFDNKLPFARDRIVEG
FFLIYGMYSEPEMKDVREFLNRVFDLISHVDDTYTVYGTFEEVILFTEAFERWTWSAL
DQLPEDMKIIYQALMDVYSQEEEKLAKKADAPKYRLEFAIETVKALTRSLLEIARWSK
ERYIPTMEEYMKVALVSCGYKTILTCSFVGMGTRATREVFEWVSSSPLIVRASELIAML
GDDIGAHEEEQKEGDAATAIECYMKEYGVTKQEAYAEFQKQIDNAWKDLNQEALRP
FPVPWSYITRVVNLTRAWHRIYADDHDGYTYPEKKIKGWITSLFVTPIPA.
(SEQ ID NO: 104) 
ATGTCTTTGTTGGTCTTGTCCACTTCCGGTTCAGCTCCATTGACTAAATCTTCTGAA
GTCAAGAGAAGATCCGCTAACTACAAGCCATCCGTCTGGGGTGACAAGTTCTTAG
AATATAGTTCTCCAGACGCCCTCAAAGCTGATTCTGAGGAAGCCAAAAAGGATGCT
GTCGAAGAAAAGAAATTGGAAAAGTTGAAGGAAGAGGTCGCTAAGATGTTGGTT
 
GAAACCGTTCAAAAGCCACAACAAGCTTTGAATTTAATTAACGAAATCCAACGTTT
GGGTTTGAGCCATCACTTCTTGCCTGAAATCGAAGCTGCTTTAAGACAAATCTCCG
TTACCTACGAAGAATTGTGCAGATCCTTGTCTGTCGATGATTTATCCAACGTTGCTT
TGTGTTTCAGAATCTTGAGAGAACACGGTCACGATGTCTCCGCCTCTGTCTTTACC
AAGTTCATGGACAGTAACGGTAAGTTGAAGGGTTCTTTCTTGAACGACGCTATCGG
TTTGTTATCCTTGTACGAAGCTACCCATTTCAGAGTCGCTGGTGAAGACTTGTTGG
AATCTTTGTTGTCTTTAACAACTTCCAGATTGGAAGCTTTGAAGTCTCAATTGAAAT
ACCCATTGGAAGATGAAATTACTAGAGCTCTAAAATCTCCACTAGCCAAGGAAATT
AATCGGTTAGGTGCCAGATACTACATTAAGATCTATGAAAAGTACTCTTCCCACAAC
AAGCTTTTGCTAGAATTTGCTATTCTAGATGGTAACAGAATGCAAAAGCAATACCA
ACGTGAATTGGCTGAATTGACCCGTGACTGGAAGGACTTGGATTTCGACAACAAG
TTGCCTTTTGCCAGAGACAGAATTGTTGAAGGCTTCTTCCTGATTTACGGTATGTAC
TCTGAACCAGAAATGAAGGATGTCAGAGAATTTTTGAACCGTGTTTTCGATTTGAT
CTCTCACGTTGACGACACTTACACCGTCTACGGTACTTTCGAAGAAGTTATTTTATT
CACTGAAGCTTTCGAAAGATGGACCTGGTCTGCTTTGGACCAATTGCCAGAAGAC
ATGAAAATCATCTACCAAGCTTTGATGGATGTCTACTCCCAAGAAGAAGAAAAGTT
AGCTAAGAAGGCTGACGCCCCAAAGTACAGATTGGAATTCGCCATTGAAACTGTT
AAGGCCTTGACCCGTTCGTTACTTGAAATCGCTAGATGGTCCAAGGAACGTTACAT
CCCAACCATGGAAGAATACATGAAGGTTGCTTTGGTTTCTTGTGGTTACAAGACCA
TCCTGACTTGTTCTTTCGTTGGTATGGGTACCAGAGCTACCAGAGAAGTCTTCGAA
TGGGTTTCCTCCTCCCCATTAATTGTTAGAGCCTCTGAATTGATCGCTATGTTGGGT
GATGACATTGGTGCTCACGAAGAAGAACAAAAGGAAGGTGATGCTGCCACTGCTA
TTGAATGTTACATGAAGGAATACGGTGTTACCAAGCAAGAAGCTTACGCTGAATTC
CAAAAACAAATTGACAACGCTTGGAAGGACTTGAACCAAGAAGCTTTAAGACCAT
TCCCAGTTCCATGGTCTTACATCACTAGAGTTGTTAACTTGACAAGAGCTTGGCAC
AGAATCTACGCAGATGACCACGACGGTTACACTTATCCAGAAAAGAAGATCAAGG
GTTGGATCACCTCTTTGTTCGTGACTCCAATTCCAGCTTGA.
(SEQ ID NO: 107) 
MSLLVLSTSGSAPLTKSSEVKRRSANYKPSVWGDKFLEYSSPDALKADSDEAKKDAV
EEKKLEKLKEEVAKMLVETVQKPQQALNLINEIQRLGLSHHFEPEIEAALRQISVTYHE
LCRSLSVDDLSNVALCFRILREHGHDVSASVFTKFMDSNGKLKGSFLNDAIGLLSLYE
ATHFRVHGEDLLESLLSLTTSRLEALKSQLKYPLEDEITRALKHPLAKEINRLGARYYI
KIYEKYDSHNKLLLEFAILDGNRMQKMYQRELAELTRDWKDLDFTNKLPFARDRIVE
GFFLIYGMYSEPEMKDVREFLNRVFDLISHVDDTYTVYGTFEEVILFTEAFERWEWSA
 
LDQLPEDMKIIYQALMDVYSQEEEKLAKKADAPKYRLEFAIETVKALTRSLLEIARWS
KERYIPTMEEYMKVALVSCGYKTILTCSFVGMGTRATREVFEWVSSSPLIVRASELIA
MLGDDIGAHEEEQKEGDAATAIECYMKEYGVTKQEAYAEFQKQIDNAWKDLNQEAL
RPFPVPWSYITRVVNLTRAIHRIYADDHDGYTYPEKKIKGYITSLFVTPIPA.
(SEQ ID NO: 108) 
ATGTCCCTGTTGGTCTTGTCTACTTCTGGTTCCGCTCCATTGACTAAGTCTTCCGAA
GTTAAGAGAAGATCTGCTAACTACAAGCCATCTGTCTGGGGTGACAAATTCTTGGA
ATACTCTTCTCCAGACGCCTTGAAAGCCGATTCTGACGAAGCTAAGAAAGATGCCG
TCGAAGAAAAGAAGTTGGAAAAGTTGAAGGAAGAAGTCGCTAAGATGTTGGTAG
AAACTGTCCAAAAGCCACAACAAGCGCTTAACTTGATTAACGAAATCCAAAGATT
GGGTTTGTCCCATCACTTTGAACCAGAAATTGAAGCTGCTTTGAGACAAATCTCCG
TTACTTACCACGAATTATGTCGTTCTTTGTCTGTTGACGATCTTTCAAACGTTGCCT
TGTGTTTCAGAATTCTACGTGAACACGGTCACGACGTTAGTGCTTCCGTTTTCACC
AAGTTCATGGATTCCAATGGAAAGCTCAAGGGTTCTTTCTTGAACGATGCCATTGG
TTTATTGTCCTTGTACGAGGCTACTCATTTCAGAGTCCACGGTGAAGATTTGTTAGA
ATCCTTGTTGTCTTTGACTACCTCCAGATTGGAAGCCTTGAAGTCCCAATTGAAGT
ACCCATTGGAAGATGAAATCACTAGAGCTTTGAAGCACCCATTGGCCAAGGAAAT
CAACCGTTTGGGCGCTCGTTACTACATCAAGATTTACGAAAAATACGATTCTCACA
ACAAATTGTTGTTGGAATTTGCTATCTTGGACGGTAACAGAATGCAAAAGATGTAC
CAAAGAGAATTGGCTGAACTGACAAGAGACTGGAAGGATTTGGATTTCACCAACA
AGTTACCTTTCGCCAGAGACAGAATCGTTGAAGGTTTCTTCTTGATCTACGGTATGT
ACTCAGAGCCAGAAATGAAGGATGTCAGAGAATTCTTAAATAGAGTTTTCGACTTG
ATCTCTCATGTTGATGACACTTACACTGTTTACGGTACTTTCGAAGAAGTCATCTTA
TTCACTGAAGCTTTCGAAAGATGGGAATGGTCTGCTCTCGACCAATTGCCAGAAG
ACATGAAGATTATTTACCAGGCTTTGATGGACGTCTACAGTCAAGAAGAAGAAAAA
TTAGCAAAGAAGGCTGATGCTCCAAAGTACAGATTGGAATTCGCTATCGAAACCGT
CAAGGCTCTAACTAGATCCTTGTTGGAAATTGCTCGTTGGTCCAAGGAAAGATATA
TTCCAACCATGGAAGAATATATGAAGGTTGCTTTAGTCTCTTGTGGTTATAAGACCA
TTTTGACCTGCTCTTTCGTCGGTATGGGTACCAGAGCTACCCGTGAAGTTTTTGAAT
GGGTTTCCTCCTCTCCTTTGATCGTTAGAGCTTCTGAATTAATCGCTATGTTAGGTGA
TGACATCGGTGCTCACGAAGAAGAACAAAAGGAAGGTGACGCCGCCACTGCTATT
GAATGTTACATGAAAGAATACGGTGTTACCAAGCAAGAAGCTTACGCAGAATTCCA
AAAGCAAATTGACAACGCTTGGAAGGACTTGAACCAAGAAGCTTTAAGACCATTC
CCAGTTCCATGGTCTTACATTACCAGAGTTGTCAACTTGACCAGAGCCATTCACAG
 
AATCTACGCTGACGACCACGATGGTTACACGTACCCAGAAAAGAAGATTAAGGGT
TACATCACCTCTTTGTTTGTTACTCCAATCCCAGCTTGA.
(SEQ ID NO: 111) 
MSLLVLSTSGSAPTTKSSEVKRRSANYKPSVWGDKFLEYSSPDALKADSDEAKKDAV
TEKKLEKLKEEVAKMLVETVQKPQQALNLINEIQRLGLSFHFEPEIEAALRQISVTYHE
LCRSTNVDDLSNVALCFRILREHGHDVSADVFTKFMDSNGKLKGSFLNDAIGLLSLYE
ATHFRVHGEDLLESLLSLTTSRLEALKSQLKYPLEDEITRALKHPLAKEINRLGARYYI
KIYEKYDSHNKLLLEFAILDGNRMQKMYQHELAELTRDWKDLDFTNKLPFARDRIVE
GFFLIYGMYSEPEMKDVREFLNRVFDLISHVDDTYDVYGTFEEVILFTEAFERWEWSA
LDQLPEDMKIIYQALMDVYSQEEEKLAKKADAPKYRLEFAIETVKALTRSLLEIARWS
KERYIPTMEEYMKVALVSVGYKTILTNSFVGMGTRATREVFEWVSSSPLIVRASELIA
MLGDDIGAHEEEQKKGDAATAIECYMKEYGVTKQEAYAEFQKQIDNAWKDLNQEAL
RPFPVPWSYITRVVNFTRAIHRIYADDHDGYTYPEKKIKGYITSLFVTPIPA.
(SEQ ID NO: 112) 
ATGTCTTTGTTAGTTTTGTCCACATCTGGTAGCGCTCCAACTACCAAGTCTTCCGAA
GTTAAGAGAAGATCTGCTAACTACAAGCCAAGTGTCTGGGGTGACAAGTTTTTAG
AGTACAGCTCTCCAGACGCTTTGAAAGCCGACTCCGATGAAGCCAAAAAGGACGC
TGTCACTGAAAAGAAGTTGGAAAAATTGAAGGAAGAAGTTGCCAAGATGTTGGTC
GAAACTGTTCAAAAGCCACAACAAGCTTTGAACTTGATCAACGAAATCCAAAGAC
TTGGTTTGTCCTTCCACTTCGAACCAGAAATCGAAGCTGCCTTAAGACAAATATCC
GTTACCTACCATGAATTATGTCGTTCCACCAACGTTGATGATTTGTCAAATGTCGCC
TTGTGTTTCAGGATCTTGAGAGAACACGGTCACGACGTCTCTGCCGATGTCTTTAC
TAAATTCATGGACTCCAACGGTAAGTTGAAGGGTTCTTTCTTGAACGATGCTATTG
GTCTACTGTCTTTGTACGAAGCCACTCATTTCAGAGTCCATGGTGAAGATTTGTTG
GAATCTTTGTTGTCATTGACCACTTCCAGACTAGAGGCTCTGAAGTCCCAATTGAA
GTATCCATTAGAAGACGAAATTACCAGAGCTTTGAAGCACCCATTGGCTAAGGAAA
TAAACCGTTTGGGTGCCAGATACTACATCAAGATTTACGAAAAGTACGACTCCCAC
AACAAGTTATTGTTGGAATTCGCTATCTTGGACGGTAACAGAATGCAAAAGATGTA
CCAACACGAATTGGCAGAATTGACCAGAGACTGGAAGGATTTGGATTTCACCAAC
AAGCTTCCTTTTGCCAGAGACAGAATTGTTGAAGGTTTCTTCTTGATTTACGGTATG
TACTCTGAACCAGAAATGAAAGATGTTAGAGAATTCTTGAATAGAGTTTTCGACTT
GATCTCTCACGTTGACGACACTTACGATGTCTACGGTACTTTTGAAGAAGTCATTTT
GTTCACTGAAGCTTTCGAAAGATGGGAATGGAGTGCTTTGGACCAATTACCAGAA
GACATGAAGATCATTTACCAAGCTTTGATGGATGTTTATTCTCAAGAAGAAGAAAA
 
GTTGGCTAAGAAGGCTGACGCCCCAAAGTACAGATTAGAATTTGCCATTGAAACC
GTCAAGGCTTTGACCAGATCCTTGTTGGAAATCGCTAGATGGTCTAAGGAACGTTA
CATTCCAACCATGGAAGAATACATGAAGGTTGCTTTGGTGTCTGTTGGTTACAAGA
CCATCTTAACTAACTCTTTCGTCGGTATGGGTACCCGTGCTACCCGTGAAGTTTTCG
AATGGGTTTCTTCTTCCCCATTGATTGTCAGAGCTTCTGAATTAATTGCTATGTTGG
GCGACGATATCGGTGCTCACGAAGAAGAACAAAAGAAGGGTGATGCTGCTACTGC
TATCGAATGTTACATGAAGGAATATGGTGTCACCAAGCAAGAAGCATATGCTGAAT
TCCAAAAGCAAATTGACAACGCTTGGAAGGACTTGAACCAAGAAGCTTTAAGACC
ATTCCCAGTCCCATGGTCTTACATCACTAGAGTTGTTAACTTCACTAGAGCTATTCA
CAGAATCTACGCTGATGACCACGATGGTTACACTTACCCAGAAAAGAAAATCAAA
GGTTACATTACGTCTTTGTTCGTTACTCCTATCCCAGCTTGA.
(SEQ ID NO: 115) 
MSLLVLSTSGSAPTTKSSEVKRRSANYKPSVWGDKFLEYSSPDALKNDSDELKKDAV
TEKKLEKLKEEVAKMLVETVQKPQQALNLINEIQRLGLSFHFEPEIEAALRQISVTYHE
LCRSTNVDDLSNVALCFRILREHGHKVSADVFTKFMDSNGKLKGSFLNDAIGLLSLYE
ATHFRVHGEDLLEDLLSLTTSRLEYLKSQMKYPLEDEITRALKHPLAKEINRLGARYYI
KIYEKYDSHNKLLLEFAILDGNRMQKMYQHELAELTRDWKDLDFTNKLPFARDRIVE
GYFLIYGMYFEPEMKDVREFLNRVFDLISHVDDTYDVYGTFEEVILFTEAFERWEWS
ALDQLPEDMKIIYQALMDVYSQEEEKLAKKADAPKYRLEFAIETVKALTRSYLEIAR
WSKERYIPTMEEYMKVALVSVGYKTILTNSFVGMGDRATREVFEWVSSSPLIVRASEL
IAMLGDDIGAHEEEQKKGDAATAIECYMKEYGVTKQEAYAEFQKQIDNAWKDLNQE
ALRPFPVPWSYITRVVNFTRAIHRIYADSHDGYTYPEKKIKGYITSLFVTPIPA.
(SEQ ID NO: 116) 
ATGTCTTTGCTAGTCTTGTCTACTTCTGGTTCTGCTCCAACTACTAAGTCCTCCGAA
GTTAAGAGAAGATCTGCTAACTACAAGCCATCTGTCTGGGGTGACAAGTTCTTGGA
ATACTCTTCTCCAGACGCTTTAAAGAACGACTCCGATGAACTGAAGAAGGATGCTG
TCACTGAAAAGAAGTTGGAAAAATTGAAGGAAGAAGTTGCTAAGATGTTGGTCGA
AACTGTTCAAAAGCCACAACAAGCTTTAAACTTGATTAACGAAATCCAAAGATTG
GGTTTGAGCTTCCACTTCGAACCAGAAATCGAAGCTGCTTTGAGACAAATCTCTGT
CACTTACCACGAATTGTGTCGTTCCACCAACGTCGATGATTTGTCCAATGTTGCTTT
ATGTTTCAGAATCTTGCGTGAACACGGTCACAAGGTCAGTGCCGATGTTTTCACCA
AATTCATGGACTCCAACGGTAAGTTGAAGGGTTCTTTCTTGAACGATGCCATTGGT
TTGTTGTCTTTGTACGAAGCTACCCATTTCAGAGTTCACGGTGAAGACTTGCTAGA
AGATTTATTGTCCTTGACCACTTCCAGACTGGAATATTTGAAGTCTCAAATGAAATA
 
TCCATTAGAGGACGAAATCACTAGGGCTTTGAAGCACCCATTGGCCAAGGAAATC
AACCGGTTGGGTGCCAGATACTACATCAAGATTTACGAAAAGTACGACTCTCACAA
CAAGTTGTTGTTGGAATTTGCTATTTTGGATGGTAACAGAATGCAAAAAATGTACC
AACACGAATTAGCTGAATTGACCAGAGACTGGAAAGACTTAGATTTCACCAATAAA
TTACCTTTCGCTAGAGATAGAATCGTTGAAGGTTACTTCTTAATATATGGTATGTACT
TTGAACCAGAAATGAAGGACGTTAGAGAATTCTTGAACAGAGTTTTCGACTTGAT
CTCCCATGTTGACGACACTTACGATGTTTACGGTACCTTTGAAGAAGTTATCTTATT
CACTGAAGCTTTCGAAAGATGGGAATGGTCTGCCTTGGACCAATTGCCAGAAGAC
ATGAAGATCATTTACCAAGCCTTGATGGATGTCTACTCCCAAGAAGAAGAAAAGTT
GGCTAAGAAGGCTGACGCTCCAAAGTACAGATTGGAATTTGCCATTGAAACCGTC
AAGGCTCTAACCAGATCTTACTTGGAAATCGCTCGTTGGTCCAAGGAACGTTACAT
CCCAACCATGGAAGAATATATGAAGGTTGCCTTGGTTTCTGTTGGTTACAAGACTAT
CTTGACTAACTCTTTTGTCGGTATGGGTGACAGAGCTACTAGAGAAGTCTTCGAAT
GGGTTTCCTCTTCTCCATTGATTGTCAGAGCTTCTGAATTGATTGCTATGTTGGGCG
ATGACATTGGTGCTCACGAAGAAGAACAAAAGAAGGGTGATGCTGCCACAGCTAT
TGAATGTTACATGAAAGAGTACGGTGTTACCAAGCAAGAAGCTTACGCTGAATTCC
AAAAGCAAATTGACAACGCTTGGAAGGACTTGAACCAAGAAGCCTTAAGACCATT
CCCAGTTCCATGGTCTTACATAACCAGAGTTGTCAACTTCACCAGAGCCATTCACA
GAATTTACGCAGATTCTCATGACGGTTACACTTACCCTGAAAAGAAAATCAAAGGT
TACATTACTTCACTATTCGTTACTCCAATCCCAGCTTGA.
(SEQ ID NO: 119) 
MSLNVLSTSGSAPTTKSSEVTRRSANYKPSVWGDKFLEYSSPDALKNDSDELKKDAV
TEKKLEKLKEEVAKMLVETVQKPQQALNLINEIQRLGLSFHFEPEIEAALRQISVTYHE
LCRSTNVDDLSNVALCFRILREHGHKVSADVFTKFMDSNGKLKGSFLNDAIGLLSLYE
ATHFRVHGEDLLEDLLSLTTSRLEYLKSQMKYPLEDEITRALKHPLAKEINRLGARYYI
KIYEKYDSHNKLLLEFAILDGNRMQKMYQHELAELTRDWKDLDFTNKLPFARDRIVE
GYFLIYGMYFEPECKDVREFLNRVFALISHVDDTYDVYGTFEEVILFTEAFERWEWSA
LDQLPEYMKIIYQALMDVYSQMEEKLAKKADAPTYRLEFAIETVKALTRSYLEIARW
SKERYIPTMEEYMKVALVSVGYKTILTNSFVGMGDRATREVFEWVSSSPLIVRASDLI
AMLGDDIGAHEEEQKKGDAATAVECYMKEHGVTKQEAYAEFQKQIDNAWKDLNQE
ALRPFPVPWSYITRVVNFTRAIHRIYADSHDGYTYPEKKIKGYITSLFVTPIPM.
(SEQ ID NO: 120) 
ATGTCATTAAACGTCCTCTCTACTTCTGGTTCTGCCCCAACTACTAAGTCATCTGAA
GTTACTAGAAGATCTGCTAACTACAAGCCATCTGTCTGGGGTGACAAGTTCTTGGA
 
GTATTCCTCTCCAGACGCTTTGAAGAACGACTCCGATGAATTGAAGAAAGATGCCG
TCACTGAAAAAAAGTTGGAAAAGTTGAAGGAAGAAGTCGCTAAGATGTTGGTTG
AAACCGTTCAAAAGCCACAACAAGCCTTGAACTTGATCAACGAAATCCAAAGATT
GGGATTGTCTTTCCACTTTGAACCAGAAATTGAAGCCGCTTTGAGACAAATCTCCG
TTACTTACCACGAATTATGCAGATCCACTAATGTCGATGATTTGTCTAACGTTGCTTT
GTGTTTCAGAATCCTAAGAGAACATGGTCACAAGGTTTCTGCTGATGTTTTTACCA
AGTTCATGGACTCCAACGGTAAGCTAAAAGGTTCCTTCTTAAACGATGCTATCGGT
TTGTTGTCTTTGTACGAAGCTACTCATTTCAGAGTTCACGGTGAAGATTTGTTGGA
AGACTTGTTGTCTCTGACTACTTCCAGACTAGAATACTTGAAGTCCCAAATGAAGT
ACCCTTTGGAAGACGAAATCACACGTGCTTTGAAGCACCCATTAGCCAAGGAGAT
CAACAGATTGGGTGCCCGTTACTACATCAAGATTTACGAAAAATACGATTCCCACA
ACAAGCTGTTGCTTGAATTTGCTATCTTGGACGGTAACAGAATGCAAAAAATGTAC
CAACACGAATTGGCTGAATTAACCAGGGATTGGAAGGATTTGGATTTCACCAACA
AATTGCCATTCGCTAGAGACAGAATTGTGGAAGGTTACTTCTTGATTTACGGTATGT
ATTTTGAACCTGAATGTAAGGACGTCCGTGAATTCTTGAATCGTGTTTTCGCTTTGA
TCTCTCATGTTGACGACACTTACGACGTTTACGGTACTTTCGAAGAAGTCATTTTAT
TCACTGAAGCTTTCGAAAGATGGGAATGGAGCGCATTGGACCAATTGCCAGAATA
CATGAAAATCATCTATCAAGCTTTGATGGATGTCTACTCTCAAATGGAAGAAAAATT
GGCTAAGAAGGCTGATGCTCCAACCTACAGATTAGAGTTTGCCATCGAAACTGTTA
AGGCCTTGACCAGATCTTACCTAGAAATTGCCAGATGGTCCAAGGAAAGATACATC
CCAACCATGGAAGAATATATGAAGGTTGCTTTAGTTTCCGTCGGTTACAAGACCAT
CTTGACTAACTCTTTCGTCGGTATGGGCGACAGAGCCACCCGTGAAGTTTTCGAAT
GGGTTAGTTCTTCCCCATTGATTGTCAGAGCTTCTGACTTGATTGCTATGTTGGGTG
ATGACATTGGTGCTCACGAAGAAGAACAAAAGAAGGGTGACGCTGCTACCGCTGT
CGAATGTTACATGAAGGAACACGGTGTTACCAAGCAAGAAGCTTACGCTGAATTC
CAAAAGCAAATTGACAACGCTTGGAAGGACTTGAACCAAGAAGCTTTAAGACCAT
TCCCAGTTCCATGGTCCTACATCACCAGAGTTGTCAACTTCACCAGAGCTATTCAC
AGAATCTACGCTGACTCTCACGATGGTTACACTTACCCGGAAAAGAAGATCAAGG
GTTACATTACCTCTTTGTTCGTCACCCCAATTCCAATGTGA.
(SEQ ID NO: 123) 
MSLNVLSTSGSAPTTKSSEVTRRSANYHPSVWGDKFLEYSSPDALKNDSDELKNDAV
TEKKLEKLKEEVKKMLVETVQKPQQALNLINEIQRLGLSFHFEPEIEAALRQISVTYHE
LCRSTNVDDLSNVALCFRILREHGHKVSADVFHKFMDSNGKLKGSFLNDAIGLLSLYE
ATHFRVHGEDLLEDLLSLTTSRLEYLKSQMKYPLEDEITRALKHPLAKEINRLGARYYI
 
SIYEKYDSHNKLLLEFAKLDGNRMQKMYQHELAELTRDWKDLDFTNKLPFARDRIV
EGYFLIYGMYFEPECKDVREFLNRVFALISHVDDTYDVYGTFEEVILFTHAFERWETS
ALDQLPEYMKIIYQALMDVYSQMEEKLAKKADAPTYRLEFAIETVKALFRSYLEIAR
WSKERYIPTMEEYMKVALVSVGYKTILTNSFVGMGDTATREVFEWVSSSPLIVRASDL
IAMLGDDIGAHEEEQKKGDAATAVECYMKEHGVTKQEAYAEFQKQIDNAWKDLNQE
ALRPFPVPWTYITRVVHFTRAIHRIYADSHDGYTYPEKKIKGYITSLFVTPIPM.
(SEQ ID NO: 124) 
ATGTCTTTGAACGTGTTGTCCACTTCTGGTTCCGCCCCAACTACTAAGTCTTCTGAA
GTTACCAGAAGATCCGCTAACTACCACCCATCTGTCTGGGGTGACAAGTTTTTAGA
ATACAGCTCCCCAGATGCCTTGAAGAATGACTCTGACGAATTGAAGAACGACGCT
GTTACTGAAAAGAAACTAGAAAAGCTAAAAGAAGTCAAGAAGATGTTGGTT
GAAACTGTTCAAAAGCCACAACAAGCTTTGAACTTAATCAACGAAATCCAAAGAT
TGGGTTTGTCTTTCCATTTCGAACCAGAAATTGAAGCCGCTTTGAGACAAATCTCC
GTCACTTACCATGAACTTTGCCGTTCTACCAATGTTGACGACTTGTCAAACGTTGC
CTTGTGTTTCCGTATCTTGAGAGAACACGGTCACAAGGTTTCTGCTGATGTTTTCC
ACAAGTTCATGGACTCTAACGGTAAGTTGAAGGGTTCTTTCTTGAACGATGCCATT
GGTTTGTTGTCCTTGTACGAAGCTACCCACTTCAGAGTTCATGGTGAAGACTTGTT
GGAAGATTTATTGTCCTTGACTACCTCTAGATTGGAATACTTGAAATCCCAAATGAA
ATACCCTTTGGAAGATGAAATCACCAGAGCCTTAAAGCATCCATTGGCCAAGGAAA
TTAACAGATTGGGCGCTAGATACTACATTTCTATCTACGAAAAGTACGACTCCCACA
ACAAATTGCTTTTGGAATTTGCTAAGTTGGACGGTAACAGAATGCAAAAGATGTAC
CAACACGAATTGGCTGAATTGACAAGAGACTGGAAGGACTTGGATTTCACTAACA
AGCTGCCATTCGCCAGAGACAGAATTGTCGAAGGTTACTTCTTAATTTACGGTATGT
ATTTTGAACCAGAGTGTAAGGACGTCAGAGAATTCTTGAACAGAGTCTTCGCTTTG
ATCTCTCACGTTGATGACACTTACGATGTTTACGGTACTTTCGAAGAAGTCATCTTG
TTTACTCACGCTTTCGAAAGGTGGGAAACCTCTGCACTAGACCAATTGCCTGAATA
CATGAAAATCATCTACCAAGCTTTGATGGATGTCTACTCTCAAATGGAAGAAAAGT
TGGCTAAAAAGGCTGATGCTCCAACCTACAGATTAGAATTCGCTATCGAAACTGTT
AAGGCCTTATTCAGATCTTACTTGGAAATCGCTAGATGGTCTAAGGAACGTTACATC
CCAACCATGGAAGAATATATGAAGGTTGCTTTGGTTTCTGTCGGTTACAAGACTATT
TTGACCAACTCCTTCGTTGGTATGGGTGACACCGCTACCCGTGAAGTTTTCGAATG
GGTTAGTTCCTCCCCATTGATTGTTAGAGCTAGTGATTTGATTGCTATGTTGGGTGA
CGATATTGGTGCTCACGAAGAGGAACAAAAGAAGGGTGATGCTGCTACTGCTGTC
GAATGTTACATGAAGGAACACGGTGTTACCAAGCAAGAAGCTTACGCTGAATTCC
 
AAAAGCAAATTGACAACGCTTGGAAGGATTTGAACCAAGAAGCGTTAAGACCATT
CCCAGTTCCATGGACTTATATCACCCGTGTTGTCCACTTCACCAGAGCTATTCACAG
AATCTATGCTGACTCACACGATGGTTACACTTACCCAGAAAAAAAGATCAAGGGTT
ACATAACTTCCCTCTTTGTTACTCCAATTCCAATGTGA.
(SEQ ID NO: 127) 
MSLNVLSTSGSAPTTKSSEVTRRSANYHPSVWGDKFLEYSSPDHLKNDSDHLKNDAV
TEKKLEKLKEEVKKMLVETVQKPQQALNLINEIQRLGLSFHFEPEIEAALRQISVTYHE
LCRSTNVDDLHNVALCFRILREHGHKVSCDVFHKFMDSNGKLKGSFLNDAIGLLSLY
EATHFRVHGEDLLEDLLSLTTSRLEYLKSQMKYPLEDEITRALKHPLHKELNRLGARY
YISIYEKYDSHNKLLLEFAKLDGNRMQKMYQHELAELTRWWKDLDFTNKLPFARDRI
VEGYFLIYGMYFEPECKDVREFLNRVFALISHVDDTYDVYGTFEEVILFTHAFERWET
SALDQLPEYMKIIYQALMDVYSQMEEKLAKKADAPTYRLEFAIETVKALFRSYLEIAR
WSKERYIPTMEEYMKVALVSVGYKTILTNSFVGMGDTATREVFEWVSSSPLIVRASDL
IAMLGDDIGGHEEEQKKGDAATAVECYMKEHGVTKQEAYAEFQKQIDNAWKDLNQE
ALRPFPVPWTYITRVVHFTRAIHVIYADSDDGYTYPEKKIKGYITSLFVDPIPM.
(SEQ ID NO: 128) 
ATGTCTCTTAATGTCTTGTCCACTTCTGGTTCTGCTCCAACCACCAAGTCTTCTGAA
GTTACCAGAAGATCCGCTAACTACCATCCATCTGTCTGGGGTGACAAGTTCTTGGA
ATATTCCTCTCCAGACCACTTGAAGAACGACTCAGATCATTTGAAGAACGATGCTG
TCACTGAAAAAAAATTGGAAAAGCTGAAGGAAGAAGTTAAGAAGATGTTGGTTG
AAACTGTTCAAAAGCCACAACAAGCTTTGAACTTGATCAACGAAATCCAAAGATT
GGGTTTGTCTTTCCACTTCGAACCAGAAATTGAAGCTGCTTTGCGTCAAATCTCTG
TCACGTACCACGAATTGTGCAGATCTACCAACGTCGATGACTTGCACAACGTTGCT
TTGTGTTTCAGAATCTTACGTGAACACGGTCACAAGGTTTCCTGTGATGTTTTCCAT
AAGTTCATGGATTCCAACGGTAAGTTAAAGGGTTCCTTCTTGAACGATGCCATCGG
TTTGTTGAGCTTGTACGAAGCTACCCACTTCAGAGTTCACGGTGAAGACTTATTGG
AAGACCTTCTATCTTTGACTACCTCTAGATTAGAATACTTGAAGTCCCAAATGAAGT
ACCCTCTCGAAGATGAAATCACAAGAGCTTTGAAGCACCCATTGCACAAGGAATT
GAACCGTTTGGGTGCCCGTTACTACATCTCCATTTACGAAAAGTACGATTCTCACA
ACAAGTTGTTGTTGGAATTTGCTAAGTTGGACGGTAACAGAATGCAAAAGATGTAC
CAACACGAATTGGCCGAACTAACTAGATGGTGGAAGGACTTGGATTTCACTAACA
AATTGCCTTTCGCTAGAGACAGAATTGTTGAAGGTTATTTCTTGATCTACGGTATGT
ACTTCGAACCAGAATGTAAGGATGTTAGAGAATTCTTAAATAGAGTTTTCGCCTTG
ATCTCCCACGTCGATGACACCTACGACGTCTATGGTACTTTCGAAGAAGTCATTTG
 
TTCACTCACGCTTTCGAAAGATGGGAAACCTCTGCTCTCGACCAATTGCCAGAATA
TATGAAGATTATTTACCAAGCTTTGATGGATGTCTACTCTCAAATGGAAGAAAAGTT
GGCCAAGAAAGCTGACGCTCCAACTTACAGACTAGAGTTTGCTATCGAAACTGTC
AAGGCCTTATTTAGGTCTTACTTGGAAATTGCCAGATGGTCTAAGGAAAGATACATT
CCAACCATGGAAGAATACATGAAGGTCGCTTTAGTATCTGTTGGTTACAAGACCAT
CTTGACCAACTCTTTCGTCGGTATGGGTGACACTGCTACCAGAGAAGTCTTTGAAT
GGGTTAGTTCCTCCCCATTGATTGTCAGAGCTTCTGACTTGATTGCTATGCTTGGCG
ACGACATTGGTGGTCACGAAGAAGAACAAAAGAAAGGTGACGCTGCCACGGCTG
TTGAGTGTTACATGAAGGAACATGGTGTTACTAAGCAAGAAGCTTACGCTGAATTC
CAAAAGCAAATTGACAACGCTTGGAAAGATTTGAACCAAGAAGCATTAAGACCAT
TCCCAGTTCCATGGACTTACATTACCCGTGTTGTCCATTTCACCAGAGCCATTCACG
TTATCTACGCCGATTCCGATGATGGTTACACTTACCCAGAAAAAAAGATCAAGGGT
TACATCACTTCCTTATTTGTTGACCCAATCCCAATGTGA.
(SEQ ID NO: 131) 
MSLNVLSTSGSAPTTKSSEVTRRSANYHPSVWGDKFLEYSSPDHLKNDSDHLKNDAV
TEKKHEKLKEEVKKMLVETVQKPQQALNLINEIQRLGLSFHFEPEIEAALRQISVTYH
EYCCSTNVDDLHNVALCFRILREHGHKVSCDVFHKFMDSNGKLKGSFLNDAIGLLSL
YEATHFRVHGEDLLEDLLSLTTSRLEYLKSQMKYPLEDEITRALKHPLHKELNRLGAR
YYISIYEKYDSHNKLLLEFAKLDGNRMQKMYQHELAELTRWWKDLDFTNKLPFARD
RIVEGYFLIYGMYFEPECKDVREFLNRVFALISHVDDTYDVYGTFEEVILFTHAIERWE
TSALDQLPEYMKIIYQALMDVYSQMEEKLAKKADCPTYRLEFAIETVKALFRSYLEE
ARWSKERYIPTMEEYMTVALVSVGYKTILTNSFVGMGDTATREVFEWVFSSPLIVRAS
DLIARLGDDIGGHEEEQKKGDAATAVECYMKEHGVTKQEAYAEFQKQIDNAWKDLN
QEALRPFPVPMTYITRVVHFTRAIHVIYADSDDGYTRPEKKIKGYITSLLVDPIPM.
(SEQ ID NO: 132) 
ATGTCTTTGAACGTATTGTCAACTTCTGGTTCCGCTCCAACCACCAAGTCTAGTGA
AGTCACCAGAAGATCTGCTAACTACCACCCATCCGTCTGGGGTGACAAGTTCTTGG
AATACTCCTCTCCAGACCATTTGAAGAACGATTCCGACCACTTGAAGAACGATGCT
GTCACTGAAAAGAAGCACGAAAAACTCAAGGAAGAAGTTAAGAAGATGTTGGTT
GAAACCGTTCAAAAGCCACAACAAGCTTTGAACTTGATCAACGAAATCCAAAGAT
TAGGTTTGTCTTTCCACTTCGAACCAGAAATCGAAGCTGCCTTGAGACAAATCTCT
GTTACCTACCATGAATACTGTTGTTCCACCAACGTGGATGACTTACACAACGTTGCT
TTGTGTTTCAGAATTTTGCGTGAACACGGTCATAAGGTTTCCTGTGATGTCTTCCAC
AAGTTCATGGACTCTAACGGTAAATTGAAGGGTTCTTTCTTGAACGACGCTATTGG
 
CTTGCTGAGCTTGTACGAAGCTACCCACTTTAGAGTTCACGGTGAAGATTTGTTGG
AAGATCTACTAAGTTTAACCACCTCCAGATTAGAATACTTAAAATCTCAAATGAAAT
ACCCATTAGAGGATGAAATTACTCGTGCTTTGAAGCATCCATTGCACAAGGAATTA
AACCGTTTGGGTGCTAGATACTACATTTCAATTTACGAAAAGTACGATTCTCACAAC
AAGTTGTTGTTGGAATTCGCTAAGTTGGACGGTAACCGGATGCAAAAGATGTACCA
ACACGAATTGGCTGAATTGACCCGTTGGTGGAAGGACTTGGATTTCACTAATAAAC
TGCCTTTCGCCAGAGACAGAATCGTTGAAGGTTACTTCCTTATTTACGGTATGTATT
TCGAACCAGAATGCAAGGATGTTAGAGAATTCTTGAATAGAGTCTTCGCTTTGATC
TCCCACGTCGATGACACTTACGACGTTTATGGTACTTTCGAAGAAGTTATCTTGTTT
ACCCACGCTATCGAAAGATGGGAAACTTCTGCATTGGACCAATTACCAGAATACAT
GAAGATCATCTACCAAGCTTTGATGGATGTCTACTCTCAAATGGAAGAGAAGTTGG
CCAAGAAAGCTGACTGTCCAACTTACAGATTGGAATTTGCCATTGAAACCGTCAA
GGCTCTTTTCAGATCCTACTTGGAAGAAGCCAGATGGTCTAAAGAAAGATATATTC
CTACCATGGAAGAATATATGACTGTTGCCTTGGTTTCTGTTGGTTACAAGACCATCT
TGACTAACTCTTTCGTCGGTATGGGTGACACTGCTACAAGAGAAGTCTTTGAATGG
GTTTTCTCTTCCCCATTGATTGTCAGAGCTTCTGACTTGATTGCTAGATTGGGTGAT
GACATTGGTGGTCACGAAGAAGAACAAAAGAAGGGTGATGCTGCTACTGCCGTCG
AATGTTACATGAAGGAACACGGTGTTACTAAGCAAGAAGCTTACGCTGAATTCCAA
AAGCAAATTGACAACGCTTGGAAGGACTTAAACCAAGAAGCCTTAAGACCATTCC
CAGTTCCAATGACTTACATCACTAGAGTTGTCCATTTCACGAGAGCTATCCACGTTA
TCTACGCCGATTCCGACGATGGTTACACTCGTCCAGAAAAGAAGATCAAGGGTTAC
ATTACCTCTTTGTTAGTTGACCCAATCCCAATGTGA.
(SEQ ID NO: 135) 
MRKKETERPTADFKPSLWRNKFLKGAKDFKTVDREATAERLEALKEEVRRMITDEED
KPVQGLRLIDEVKRLGVAYHFEKEIADALQKLSPEYIESNSPDLATVSLAFRLLRQGGI
KISADVFEKFYDDAGRFKSSLINDVQGLLSLYEAAYCAVLGESILDEAIAFTTKHLKEL
VAQDALTPKLQEQINQALARPERQTLPLLEARYFRSFIQKSSDAKYNKTLLNFAILDHN
ILLELHKEELNELTKWWKELDFSKKLPYARDRLVAIYFFCLGMEAEPQYAFGREIVTR
LDTILSHIHDYYFFYGTLEELELFTEAVQRWESEAISTLPEDMKLIYRELLDAFNEIEES
MAKQGRSWLVEYAKEAFQKVVGAYFILAKWASEGYVPTIEEYMPIALISNAMQFVIT
VSFLGMGDFATKEVFEWISSMPKVVKAASIIGLLLDDMQGHEFGQKIGSWASAIECYT
KQHGVSKEEAIKAFEEEVANARKQINAELMKKPQVVARPLLGLILNLARAITFIYAED
DGYFHSKLIKDQIASVLGDELPF.
(SEQ ID NO: 136) 
ATGAGAAAGAAGGAAACCGAAAGACCAACCGCTGACTTCAAGCCATCTTTATGGA
GAAACAAGTTCTTGAAGGGTGCCAAGGACTTTAAGACTGTTGACCGGGAAGCTAC
TGCTGAAAGATTGGAAGCTTTGAAGGAGGAAGTCAGAAGAATGATTACCGATGAA
GAGGACAAGCCAGTTCAAGGTTTGAGATTGATCGATGAAGTAAAGAGATTGGGTG
TTGCTTACCATTTCGAAAAGGAAATTGCTGATGCTTTGCAAAAATTGAGCCCAGAA
TACATTGAATCTAACTCTCCAGACTTGGCCACTGTCTCCTTGGCTTTCAGATTATTG
AGACAAGGTGGTATCAAGATTTCTGCTGATGTTTTCGAAAAATTCTACGATGATGC
CGGTAGATTCAAGTCCAGTTTGATTAATGATGTCCAAGGTTTGCTTTCATTGTACGA
AGCTGCCTACTGTGCTGTCTTAGGTGAATCCATCTTGGACGAAGCTATTGCCTTCAC
CACTAAGCATTTGAAGGAGTTAGTTGCTCAAGATGCTTTGACTCCAAAGCTACAAG
AACAAATCAACCAAGCTCTAGCCAGACCAGAACGTCAAACTTTGCCTTTGTTGGA
AGCAAGATACTTTAGATCCTTCATCCAAAAGTCTTCTGACGCTAAGTACAACAAGA
CCCTCTTGAACTTCGCCATCTTGGACCACAACATCCTATTGGAATTGCACAAGGAA
GAATTGAACGAACTGACCAAATGGTGGAAGGAACTCGATTTCTCCAAGAAGTTGC
CATACGCTAGAGACAGATTGGTCGCCATCTATTTCTTCTGTTTGGGTATGGAAGCTG
AACCACAATACGCTTTTGGTCGTGAAATTGTTACCAGATTAGACACCATCTTGTCTC
ACATTCACGACTACTACTTCTTTTACGGTACTTTGGAAGAATTGGAATTGTTCACTG
AAGCCGTTCAAAGATGGGAATCTGAAGCTATCTCTACCCTGCCAGAAGACATGAAA
TTAATCTATAGAGAATTGTTGGACGCTTTCAACGAAATCGAAGAATCCATGGCTAA
ACAAGGTAGATCTTGGTTGGTTGAATATGCTAAAGAAGCTTTCCAAAAGGTTGTTG
GTGCTTACTTCATTTTGGCTAAGTGGGCTTCCGAAGGTTACGTTCCAACTATTGAAG
AATACATGCCAATCGCTTTGATTTCCAACGCTATGCAATTCGTTATCACTGTCTCTTT
CTTAGGTATGGGTGACTTCGCTACCAAGGAAGTCTTTGAATGGATTTCTTCCATGCC
AAAGGTCGTCAAGGCTGCTTCCATCATTGGTTTGTTGTTGGATGACATGCAAGGTC
ACGAATTCGGTCAAAAGATCGGCTCCTGGGCTTCTGCCATCGAATGTTACACTAAG
CAACACGGTGTCTCTAAAGAAGAGGCTATTAAGGCTTTCGAAGAAGTTGCTA
ACGCCAGAAAGCAAATCAATGCCGAATTGATGAAGAAGCCTCAAGTTGTCGCTCG
TCCATTGTTGGGTTTGATTTTAAACTTGGCTAGAGCTATCACATTCATCTACGCTGA
AGATGATGGTTACTTCCACTCTAAGTTGATCAAGGACCAAATTGCTTCTGTCTTGG
GAGACGAATTGCCATTCTGA.
(SEQ ID NO: 139) 
MRKKETERPTADFKPSLWRNHFLKGAKDFKTVDREATAERLEALKEEVRRMITDEED
KPVQGLRLIDEVKRLGVAYHFEKEIADAIQKLSPEYIESNSPDLATVSLAFRLLRQGGIK
ISADVFEKFYDDEGRFKSSLINDVQGLLSLYEAAYMAVRGESILDEAIAFTTTHLKELV
 
AQDALTPKLAEQINQALARPERQTLPLLEARYFRSFIQSSSDAKYNKTLLNFAILDHNI
LLELHKEELNELTKWWKELDFSKKLPYARDRLVALYFFDLGMEFEPQYAFGRKIVTRL
DTILSHIHDYYFFYGTLEELELFTEAVQRWESEAISTLPEDMKLIYRELLDAFNEIEESM
AKQGRSWLVRYAKEAFQKVVGAYFILAKWASEGYVPTIEEYMPIALISNAMQFVITVS
FLGMGDFATKEVFEWISSNPKVVKAASVIGLLLDDMQGHEFGQKIGSWASAIECYTK
QHGVSKEEAIKAFEEEVANARKQINAELMKKPQVVARPLLGTILNLARAITFIYAEDD
GYFHSKLIKDQIASVLGDELPF.
(SEQ ID NO: 140) 
ATGAGAAAGAAAGAAACCGAAAGACCAACTGCGGATTTCAAGCCATCTTTGTGGA
GAAACCACTTCTTGAAGGGTGCTAAGGATTTCAAGACTGTCGACAGAGAAGCCAC
CGCTGAACGTTTGGAAGCTTTGAAAGAAGTCAGAAGAATGATTACTGATGAA
GAAGACAAGCCAGTTCAAGGTTTGAGATTGATTGACGAAGTTAAGAGATTAGGTG
TCGCTTACCATTTCGAAAAGGAAATCGCTGACGCTATCCAAAAGTTGTCTCCAGAA
TACATTGAATCCAACTCCCCAGACCTAGCTACTGTCTCTTTGGCCTTCCGTTTGTTG
AGACAAGGTGGTATCAAGATTTCTGCCGATGTCTTCGAAAAGTTCTACGATGACGA
AGGTAGATTCAAATCATCCCTTATTAACGATGTTCAAGGTTTGTTATCCTTGTACGA
AGCTGCTTACATGGCCGTTAGAGGTGAATCTATCCTAGACGAAGCTATAGCTTTTAC
CACGACTCATTTGAAGGAATTGGTTGCTCAAGATGCTTTGACTCCAAAGTTGGCCG
AACAAATCAACCAAGCTTTAGCTCGTCCAGAAAGACAAACTTTACCATTGTTGGA
AGCCAGGTATTTCAGATCTTTCATCCAATCCTCCTCTGACGCCAAGTACAACAAGA
CCTTATTGAACTTCGCCATTTTGGACCACAACATCCTCTTGGAATTGCACAAGGAA
GAATTGAATGAATTGACCAAGTGGTGGAAGGAATTGGATTTCTCTAAGAAGTTGCC
ATACGCTCGTGACAGATTAGTTGCTTTGTATTTCTTCGACTTGGGTATGGAATTCGA
ACCACAATACGCCTTTGGTCGAAAAATTGTCACCAGATTGGACACTATCTTGTCTC
ACATTCACGACTACTACTTCTTTTACGGTACTTTGGAAGAGTTGGAACTCTTCACA
GAAGCTGTCCAAAGATGGGAATCTGAAGCTATTTCTACTCTGCCTGAAGACATGAA
ATTGATTTACAGAGAATTATTGGATGCTTTTAACGAAATTGAAGAATCCATGGCTAA
GCAAGGTAGATCCTGGTTGGTGAGATACGCTAAGGAAGCCTTTCAAAAGGTCGTT
GGTGCCTACTTCATTTTGGCTAAATGGGCTTCCGAAGGTTACGTTCCAACCATCGA
AGAATATATGCCAATCGCTTTGATCTCTAATGCTATGCAATTCGTCATCACCGTCTCT
TTCTTAGGTATGGGTGACTTCGCTACCAAGGAAGTTTTCGAATGGATTTCTTCTAAC
CCAAAGGTTGTCAAGGCTGCTTCTGTCATTGGTCTATTGTTGGATGATATGCAAGGT
CACGAATTCGGTCAAAAGATCGGTTCTTGGGCTTCTGCTATCGAATGTTACACTAA
GCAACACGGTGTTTCCAAGGAAGAAGCTATCAAGGCCTTTGAAGAAGAAGTTGCT
 
AACGCCAGAAAGCAAATCAACGCTGAATTGATGAAGAAGCCTCAAGTTGTTGCCA
GACCATTGTTGGGTACCATCTTAAACTTGGCTAGAGCTATCACTTTCATCTACGCTG
AAGATGACGGTTACTTCCACAGCAAATTAATCAAGGACCAAATTGCTTCCGTTTTG
GGCGATGAATTGCCATTCTGA.
(SEQ ID NO: 143) 
MRKKETERPTADFKPSLWRNHFLKGAKDFKTVDREATAERLEALKEEVRRMITDEED
KPVQGLRLIDEVKRLGVAYHFEKEIADAIQKLSPEYIHSNSPDLATVSLAFRLLRQGGI
KISADVFEKFYDDEGRFKSSLINDVQGLLSLYEAAYMAVRGESILDEAIAFTTTHLKSL
VAQDALTPKLAEQINQALARPERQTLPLLEARYFRSFIQSTSDALYNKTLLNFAILDFNI
LLELHKEELNELTKWWKELDFSKKLPYARDRLVALYFFDLGMEFEPQYAFGRKIVTRL
NTILSHIDDYYFFYGTLEELELFTEAVQRWNSEAISTLPEDMKLIYRELLDAFNEIEESM
AKQGRSWLVRYAKEAFQKVVGAYFVLAKWASEGYVPTIEEYMPIALISNAMQFVITV
SFLGMGDFATKEVFEWISSNPKVVKAASVIGLLLDDMQGHEFGQKIGSWASAIECYT
KQHGVSKEEAIKAFEEEVANARKQINAELMKKPQVVARPLLGTILNLARAITFIYAED
DGYTHSYLIKDQIASVLGDELPF.
(SEQ ID NO: 144) 
ATGAGAAAGAAGGAAACCGAAAGACCAACTGCTGACTTTAAACCATCTTTGTGGA
GAAACCACTTCTTGAAGGGTGCCAAGGATTTCAAGACTGTCGATCGTGAAGCTAC
CGCTGAAAGATTGGAAGCTTTGAAAGAAGTCCGCAGAATGATTACTGACGAG
GAAGACAAGCCAGTTCAAGGTTTGAGATTAATCGACGAAGTTAAGAGATTAGGTG
TTGCTTACCATTTCGAAAAAGAGATTGCCGACGCTATCCAAAAGCTGTCTCCAGAA
TACATTCACAGCAACTCCCCAGACTTGGCTACTGTTTCTTTGGCTTTCAGATTACTA
AGACAAGGTGGTATCAAGATCTCCGCTGATGTCTTCGAAAAGTTCTATGATGACGA
AGGTAGATTCAAGTCCTCTCTAATTAACGATGTTCAAGGTTTGTTGTCCTTGTACGA
AGCTGCTTACATGGCTGTTAGAGGCGAATCTATCTTAGACGAAGCCATTGCATTCAC
CACTACTCATTTGAAGTCTTTGGTTGCTCAAGATGCTTTGACTCCAAAGTTGGCTG
AACAAATTAACCAAGCTTTGGCAAGACCAGAACGTCAAACTTTGCCATTGTTGGA
AGCCCGTTACTTCAGATCTTTCATTCAATCCACCTCTGATGCCTTGTACAACAAGAC
CTTGTTGAACTTCGCCATATTGGACTTCAACATCTTATTGGAATTGCACAAGGAAG
AATTGAATGAATTGACCAAGTGGTGGAAGGAACTGGACTTCTCTAAGAAACTCCC
ATACGCCAGAGACAGATTGGTCGCTCTATATTTTTTCGATTTGGGTATGGAATTTGA
ACCACAATACGCCTTCGGTAGAAAGATCGTTACCAGATTGAACACTATCTTGTCTC
ACATTGACGACTACTACTTCTTCTACGGTACTTTGGAAGAATTAGAATTGTTCACTG
AAGCAGTCCAAAGATGGAACTCTGAAGCTATCTCTACCTTGCCAGAAGATATGAAA
 
TTAATTTACAGAGAATTGTTGGACGCTTTTAACGAAATCGAAGAATCCATGGCCAA
ACAAGGTCGTTCCTGGTTAGTAAGATACGCTAAGGAAGCTTTCCAAAAGGTTGTTG
GTGCTTACTTCGTTTTGGCTAAATGGGCTAGTGAAGGTTACGTTCCAACCATTGAA
GAATATATGCCAATTGCTTTGATTTCAAATGCTATGCAATTCGTCATCACCGTCTCTT
TCTTGGGTATGGGTGATTTTGCTACGAAGGAAGTCTTCGAATGGATTTCTTCCAACC
CTAAGGTCGTCAAGGCTGCTTCTGTTATCGGTTTGTTATTGGATGACATGCAAGGTC
ACGAATTCGGTCAAAAGATTGGATCCTGGGCTTCTGCTATTGAATGTTACACCAAG
CAACACGGTGTCTCCAAGGAGGAAGCTATCAAGGCCTTTGAAGAAGTTGCTA
ACGCCAGAAAGCAAATCAACGCCGAATTGATGAAGAAGCCTCAAGTTGTCGCTCG
TCCATTATTGGGTACCATCTTGAACTTGGCCAGAGCTATCACTTTCATCTACGCTGA
AGACGATGGTTACACTCACTCTTACTTGATCAAGGACCAAATTGCTTCCGTCTTGG
GTGACGAACTACCATTCTGA.
(SEQ ID NO: 147) 
MRKKETFRPTADFKPSLWRNHFLKGAKDFKTVDRTATQERLEALKEEVRRMITDEED
KPVQGLRLIDEVQRLGVAYHFEKEIEDAIQKLSPEYIHSNSPDLATVSLAFRLLRQGGIK
ISADVFEKFYDDEGRFKSSLINDVQGLLSLYEAAYMAVRGESILDEAIAFTTTHLKSLV
AQDALTPKLAEQINQALARPERQTLPLLEARYFRSFIQSTSDALYNKTLLNFAILDFNIL
LELHKEELNELTKWWKELDFSKKLPYARDRLVALYFFDLGMEFEPQYAFGRKIVTRL
NYILSHIDDYYFFYGTLEELELFTEAVQRWNSEAISTLPEDMKLIYRELLDAFNEIEES
MAKQGRSWLVRYAKEAFQKVVGAYFVLAKWASEGYVPTIEEYMPIALTSCAMTFVIT
VSFLGMGDFATKEVFEWISDNPKVVKAASVIGLLLDDMQGHEFGQKIGSVASAIECYT
KQHGVSKEEAIKAFEEEVANARKQINAELMKKPQVVARPLLGTILNLARAITFIYAED
DGYTHSYLIKDQIASVLGDELPF.
(SEQ ID NO: 148) 
ATGAGAAAGAAAGAAACTTTCAGACCAACTGCTGACTTTAAACCATCCTTATGGA
GAAACCACTTCTTGAAGGGTGCTAAGGATTTCAAGACTGTTGACAGAACTGCTAC
TCAAGAAAGATTGGAAGCTTTGAAAGAAGAAGTTAGAAGAATGATTACTGATGAA
GAAGACAAGCCTGTTCAAGGTTTGAGATTGATTGATGAAGTTCAAAGATTGGGTG
TCGCTTACCATTTCGAAAAGGAAATCGAAGATGCTATCCAAAAGTTGTCTCCAGAA
TACATTCACTCCAACTCCCCAGACTTGGCCACCGTTTCATTGGCTTTCCGTTTGCTA
AGACAAGGTGGTATTAAGATCTCTGCTGATGTTTTCGAAAAGTTCTACGATGACGA
AGGTAGATTCAAATCTTCTTTGATCAACGACGTCCAAGGTTTGTTGAGCTTATACG
AAGCTGCTTACATGGCTGTTAGAGGTGAATCTATCCTCGACGAAGCTATCGCTTTCA
CCACCACTCATTTGAAGTCTTTAGTTGCTCAAGATGCTCTAACCCCAAAGTTGGCC
 
GAACAAATTAACCAAGCTTTGGCTAGACCAGAAAGACAAACTTTGCCATTGTTAG
AAGCCCGTTACTTCAGATCCTTCATTCAGTCCACTTCCGACGCTTTGTACAACAAG
ACCCTGCTTAACTTCGCCATCTTGGATTTCAACATTTTATTGGAATTACACAAGGAA
GAATTGAACGAATTGACCAAGTGGTGGAAGGAATTGGACTTCTCTAAGAAATTGC
CATACGCTCGTGACAGATTGGTTGCCCTCTATTTCTTCGATTTGGGTATGGAATTTG
AACCACAATACGCGTTTGGTAGAAAGATTGTCACCAGATTGAACTACATCTTGTCT
CACATTGACGACTACTACTTTTTCTACGGTACTTTGGAAGAATTAGAGTTGTTCACT
GAAGCTGTCCAAAGATGGAACTCTGAAGCCATCTCTACCTTGCCAGAAGATATGAA
ACTAATTTACAGAGAATTATTGGACGCCTTCAATGAAATTGAAGAATCCATGGCTAA
ACAAGGCCGTTCTTGGTTGGTCAGATACGCCAAGGAAGCTTTCCAAAAGGTTGTT
GGTGCTTATTTCGTTTTGGCTAAGTGGGCTTCTGAAGGTTACGTTCCAACCATCGA
AGAATATATGCCAATCGCTCTTACATCATGTGCTATGACTTTCGTCATCACTGTCTCT
TTCTTGGGTATGGGTGACTTTGCTACCAAGGAAGTCTTTGAATGGATTAGTGACAA
CCCAAAGGTCGTCAAGGCCGCCTCTGTTATCGGTTTGTTATTGGACGACATGCAAG
GTCACGAATTCGGTCAAAAGATTGGTTCCGTTGCTAGCGCTATTGAATGTTACACC
AAGCAACACGGTGTTTCCAAGGAAGAAGCAATCAAGGCTTTCGAAGAAGAAGTC
GCTAACGCCAGAAAGCAAATCAATGCTGAATTGATGAAGAAGCCTCAAGTTGTCG
CGCGTCCATTGTTGGGTACCATTTTAAACTTGGCCAGAGCTATTACCTTCATCTACG
CTGAAGATGATGGTTACACTCACTCTTACTTGATCAAGGACCAAATCGCTTCTGTCT
TGGGTGATGAATTGCCATTCTGA.
(SEQ ID NO: 151) 
MRSKETFRPTADFKPSLWRNHFLKGAKDFKTVDRTATQERHEALKEEVRRMITDAED
KPVQGLRLIDEVQRLGVAYHFEKEIEDAIQKLSPEYIHSNSPDLATVSLAFRLLRQGGIK
ISADVFEKFYDDEGRFKSSLINDVQGLLSLYEAAYMAVRGESILDEAIAFTTTHLKSLV
AQDALTPKLAEQINQALARPERQTLPLLEARYFRSFIQSTSDALYNKTLLNFAILDFNIL
LELHKEELNELTKWWKELDFTKKLPYARDRLVALYFFDLGMEFEPQYAFGRKIVTRL
NYILSIIDDYYFFYGTLEELELFTEAVQRWNSEAIDTLPEDMKLIYRELLDAFNEIEEDM
AKQGRSHLVRYAKEAFQKVVGAYFVLAKWASEGYVPTIEEYMPIALTSCAMTFVITV
SFLGMGDFATKEVFEWISDNPKVVKAASVIGLLLDDMQGHEFGQKIGHVASAIECYT
KQHGVSKEEAIKAFEEEVANARKDINAELMKKPQVVARPLLGTILNLARAITFIYKED
DGYTHSYLIKDQIASVLGDELPF.
(SEQ ID NO: 152) 
ATGAGATCCAAGGAAACTTTCAGACCAACTGCTGATTTTAAGCCATCTTTGTGGAG
AAACCACTTCTTGAAGGGTGCTAAGGATTTCAAGACTGTTGACAGAACCGCTACT
 
CAAGAAAGACACGAAGCCTTGAAGGAAGAAGTCAGAAGAATGATCACTGACGCT
GAAGACAAGCCAGTTCAAGGTTTGCGTTTGATTGACGAAGTTCAAAGATTGGGTG
TCGCTTATCATTTCGAAAAGGAAATCGAAGATGCCATCCAAAAATTGTCCCCAGAA
TACATTCACTCCAACTCTCCAGATTTGGCTACCGTCTCCTTAGCCTTCCGTCTATTG
AGGCAGGGTGGTATCAAGATTTCTGCTGACGTTTTCGAAAAGTTCTACGATGACGA
AGGTAGATTCAAGTCTTCTTTGATCAACGACGTACAAGGTTTGTTATCTTTGTACGA
AGCTGCTTACATGGCTGTTAGAGGTGAGTCCATCTTAGATGAAGCCATTGCTTTCA
CCACCACTCATTTGAAGTCCTTGGTTGCTCAAGATGCTTTGACTCCAAAGTTGGCT
GAACAAATCAACCAAGCTTTGGCACGTCCTGAAAGACAAACTTTGCCATTGTTGG
AAGCTAGATATTTCAGATCCTTCATTCAATCAACCTCCGATGCTTTATACAACAAGA
CCTTGTTGAACTTTGCTATTCTAGACTTCAACATCTTGCTGGAATTGCATAAGGAAG
AATTAAATGAATTGACTAAGTGGTGGAAGGAACTAGATTTCACAAAAAAATTGCCA
TACGCTAGAGACAGATTAGTTGCTTTATACTTCTTCGATTTGGGTATGGAATTCGAA
CCACAATACGCCTTCGGCAGAAAGATCGTCACCAGATTGAACTACATCTTGTCTATT
ATCGATGACTACTACTTCTTTTACGGTACTTTGGAAGAATTGGAATTGTTCACCGAA
GCTGTCCAAAGATGGAACAGCGAAGCTATCGACACCTTACCAGAAGACATGAAAT
TAATTTACAGAGAACTTTTGGACGCCTTCAACGAAATTGAAGAAGACATGGCCAA
GCAAGGGCGTTCTCACTTGGTGAGATACGCTAAGGAGGCTTTCCAAAAGGTCGTC
GGTGCTTACTTTGTCTTGGCCAAATGGGCTAGTGAAGGTTACGTTCCAACCATTGA
AGAATATATGCCAATTGCTCTAACTTCTTGTGCTATGACTTTCGTTATCACTGTCTCT
TTCTTGGGTATGGGTGACTTCGCTACGAAGGAAGTCTTCGAATGGATTTCTGACAA
CCCAAAGGTTGTTAAGGCTGCTTCTGTCATCGGTCTGCTGTTGGATGATATGCAAG
GTCACGAATTTGGTCAAAAGATTGGTCACGTTGCCTCTGCGATAGAATGTTACACT
AAGCAACACGGTGTTTCTAAGGAAGAAGCCATCAAAGCTTTTGAAGAAGAAGTTG
CCAACGCCAGAAAGGACATTAATGCTGAATTGATGAAAAAGCCTCAAGTTGTCGC
TAGACCACTCTTAGGAACCATCTTGAACTTGGCCAGAGCTATTACCTTCATCTACAA
GGAAGACGATGGTTACACCCACTCATACTTGATCAAGGACCAAATCGCTTCCGTTT
TGGGTGATGAATTGCCATTCTGA.
(SEQ ID NO: 155) 
MRSKETFRPTADFKPSLWRNHFLKGASDFKTVDHTATQERHEALKEEVRRMITDAED
KPVQGLRLIDEVQRLGVAYHFEKEIEDAIQKLSPNYIHSNSPDLATVSLAFRLLRQGGI
KISADVFEKFYDDEGRFKSSLINDVQGLLSLYEAAYMAVRGESILDEAIAFTTTHLKSL
VAQDALTPKLAEQINHALARPERKTLPLLEARYFRSFIQSTSDHLYNKTLLNFAKLDFN
ILLELHKEELNELTKWWKELDFTTKLPYARDRLVALYFFDLGMEFEPQYAFGRKIVTR
 
LNYILSIIDDYYFFYGTLEELELFTEAVQRWNSEAIDTLPEDMKLIYRTLLDAFNEIEED
MAKQGRSHLVRYAKEAFQKVVGAYFVLAKWASEGYVPTIEEYMPIALTSCAMTFVIT
VSFLGMGDFATKEVFEWISDNPKVVKAASVIGLLLDDMQGHEFEQKIGHVASAIECY
TKQHGVSKEEAIKAFEEEVANARKDINEELMKKPQVVARPLLGTILNLARAITFIYKE
DDGYTHSYLIKDQIASVLGDELPF.
(SEQ ID NO: 156) 
ATGAGATCTAAAGAAACTTTCCGTCCTACCGCTGACTTCAAGCCATCTTTGTGGAG
AAACCATTTCTTGAAGGGTGCTTCCGATTTCAAGACCGTTGACCACACTGCTACTC
AAGAACGTCACGAAGCTTTGAAGGAAGAAGTTAGAAGGATGATTACTGATGCTGA
AGACAAGCCAGTTCAAGGTTTGAGATTGATCGATGAAGTCCAACGTTTGGGTGTC
GCTTACCACTTCGAAAAGGAAATCGAAGACGCTATTCAAAAGTTGTCTCCAAACTA
TATTCACTCCAACTCCCCAGACTTGGCCACCGTCTCATTGGCTTTCAGACTATTAAG
ACAAGGTGGTATCAAGATTTCTGCTGATGTTTTCGAAAAGTTCTACGACGATGAAG
GTAGATTCAAGTCTTCTCTAATTAACGATGTCCAAGGTTTATTGTCTTTGTACGAAG
CTGCTTATATGGCTGTCAGAGGTGAATCCATCTTGGACGAGGCTATTGCTTTCACCA
CCACTCATCTAAAGTCCTTGGTCGCTCAAGATGCTTTGACTCCAAAGTTAGCTGAG
CAGATCAACCATGCTTTGGCCAGACCAGAAAGAAAAACCTTGCCATTGTTGGAAG
CCCGTTATTTCAGATCCTTCATCCAATCAACCTCTGACCATTTGTACAACAAGACCC
TGTTGAACTTTGCCAAGTTGGATTTCAACATTTTATTAGAATTGCACAAGGAAGAA
CTGAATGAATTGACTAAGTGGTGGAAGGAATTGGACTTCACTACTAAGTTGCCATA
CGCTAGAGACAGATTGGTTGCCTTATACTTCTTTGATTTGGGTATGGAATTTGAACC
ACAATACGCCTTCGGTAGAAAGATCGTTACCAGACTGAACTACATCCTCTCTATCAT
TGATGACTACTACTTCTTCTACGGTACCTTGGAAGAATTGGAATTGTTCACTGAAGC
TGTTCAAAGATGGAACAGTGAAGCAATTGACACCTTGCCAGAAGACATGAAATTA
ATCTACAGAACTTTACTTGACGCTTTCAACGAAATTGAAGAAGATATGGCTAAGCA
AGGTCGTTCTCACTTGGTCAGATACGCTAAAGAGGCTTTCCAAAAGGTCGTTGGTG
CTTACTTCGTCTTGGCAAAATGGGCTTCCGAAGGTTACGTTCCAACTATCGAAGAA
TACATGCCAATCGCCTTGACATCTTGTGCTATGACCTTCGTCATTACTGTCTCTTTCT
TGGGTATGGGTGACTTTGCTACCAAGGAAGTCTTCGAATGGATTAGCGACAACCCA
AAGGTTGTCAAGGCTGCCTCTGTTATCGGTTTGTTGTTGGACGACATGCAAGGTCA
CGAATTCGAACAAAAGATTGGTCACGTTGCCTCCGCCATAGAATGTTACACCAAGC
AACACGGTGTTTCCAAGGAAGAAGCTATCAAGGCCTTTGAAGAAGAAGTTGCTAA
CGCTAGAAAGGATATCAACGAAGAATTGATGAAAAAGCCTCAAGTTGTTGCTAGA
CCATTGCTTGGTACTATTTTGAATTTGGCTAGAGCTATCACTTTTATCTACAAGGAA
 
GATGATGGTTACACTCACTCTTACTTGATCAAGGATCAAATTGCTTCTGTTTTGGGC
GACGAATTGCCATTCTGA.
(SEQ ID NO: 159) 
MRSKETFRPTADFKPSLWRNHFLKGASDFKTVDHTATQERHEALKEEVRRMITDAED
KPVQGLRLIDEVQRLGVAYHFEKEIEDAIQKLSPNYIHSNSPDLATVSLAFRLLRQGGI
KISADVFEKFYDDEGRFKSSLINDVQGLLSLYEAAYMAVRGESILDEAIAFTTTHLKSL
VAQDALTPKLAEQINHALARPLRKTLPRLEARYFRSMIQSTSDHLYNKTLLNFAKLDF
NILLELHKEELNELTKWWKELDFTTKLPYARDRLVALYFFDLGMEFEPQYAFGRKIMT
QLNYILSIIDDYYFFYGTLEELELFTEAVQRWNIEAVDTLPEYMKLIYRTLLDAFNEIEE
DMAKQGRSHLVRYAKEAFQKVVGAYFVLAKWASEGYVPTIEEYMPIALTSCAMTFVI
TVSFLGMGDFATKEVFEWISDNPKVVKAASVIGLLLDDMQGHEFEQKRGHVASAIEC
YTKQHGVSKEEAIKAFEEEVANARKDINEELMMKPQVVARPLLGTILNLARAITFIYK
EDDGYTHSYLIKDQIASVLGDEVPF.
(SEQ ID NO: 160) 
ATGAGATCTAAGGAAACTTTCAGACCAACTGCTGATTTCAAGCCATCTTTGTGGAG
AAACCACTTCTTGAAGGGTGCCAGTGATTTCAAGACTGTTGACCACACTGCTACC
CAAGAAAGACACGAAGCTTTGAAGGAAGAAGTCAGAAGAATGATTACTGATGCT
GAAGACAAGCCAGTTCAAGGTTTGAGATTAATCGACGAAGTTCAAAGATTGGGTG
TTGCCTATCATTTCGAAAAGGAAATTGAAGACGCCATTCAAAAATTATCCCCAAAC
TACATCCACTCCAACTCTCCAGACTTGGCCACCGTTAGCTTGGCTTTCCGGTTATTA
AGACAAGGTGGTATCAAGATCTCTGCTGACGTCTTCGAAAAGTTCTACGACGATGA
AGGTCGTTTCAAGTCCTCATTGATTAACGATGTCCAAGGTTTGTTGTCTTTGTACGA
AGCTGCTTACATGGCTGTTAGAGGTGAATCTATTTTGGATGAAGCCATTGCTTTCAC
CACCACTCACTTGAAGTCTCTAGTGGCTCAAGATGCATTGACCCCAAAGTTGGCTG
AACAAATCAACCATGCTTTGGCCAGACCATTGCGTAAGACCTTGCCAAGATTGGA
AGCTCGTTATTTCAGATCCATGATCCAATCTACTTCCGACCACTTATACAACAAGAC
ACTATTGAACTTCGCTAAGTTGGACTTCAATATTTTGCTAGAATTGCACAAGGAAG
AATTGAATGAACTCACCAAATGGTGGAAGGAATTGGACTTTACCACCAAGCTACCA
TACGCTAGAGACAGATTGGTTGCTTTGTACTTCTTCGATTTGGGCATGGAATTCGAG
CCACAATACGCCTTCGGTAGAAAGATCATGACTCAATTGAACTACATTTTGTCCATC
ATCGATGACTACTACTTCTTTTACGGTACTTTGGAAGAATTGGAATTGTTCACTGAA
GCTGTCCAAAGATGGAACATCGAAGCTGTCGACACTTTGCCAGAATATATGAAATT
GATTTACAGAACTTTATTGGATGCTTTCAACGAAATCGAAGAAGATATGGCCAAAC
AAGGTCGTTCCCATTTGGTCAGATACGCTAAGGAAGCTTTCCAAAAGGTCGTTGGT
 
GCTTACTTCGTCTTGGCCAAGTGGGCTTCTGAAGGTTACGTTCCAACCATTGAAGA
ATACATGCCAATCGCTCTAACCTCTTGTGCTATGACCTTCGTTATCACTGTCTCTTTC
TTAGGTATGGGTGATTTTGCTACTAAAGAAGTTTTCGAATGGATTTCTGACAACCCA
AAGGTTGTTAAGGCCGCTTCTGTCATTGGTTTGTTGTTGGATGACATGCAAGGTCA
CGAATTTGAACAAAAGAGAGGTCACGTTGCCTCCGCTATTGAATGTTACACCAAGC
AACACGGTGTCTCCAAGGAAGAAGCTATTAAAGCTTTTGAAGAAGAAGTTGCTAA
CGCTCGTAAAGACATCAACGAAGAATTGATGATGAAGCCTCAAGTTGTCGCCAGA
CCTCTGTTGGGTACCATCTTGAACTTGGCTAGAGCTATCACTTTCATCTACAAGGAA
GATGACGGTTACACCCACTCTTACTTGATCAAGGACCAAATAGCTTCTGTTTTGGG
TGACGAAGTCCCATTCTGA.
(SEQ ID NO: 163) 
MRSKETFRPTADFHPSLWRNHFLKGASDFKTVDHTATQERHEALKEEVRRMITDAED
KPVQKLRLIDEVQRLGVAYHFEKEIEDAIQKLSPNYIHSNSPDLATVSLAFRLLRQGGI
KISADVFEKFYDDEGRFKSSLINDVQGLLSLYEAAYMAVRGEHILDEAIAFTTTHLKSL
VAQDAVTPKLAEQINHALYRPLRKTLPRLEARYFRSMIQSTSDHLYNKTLLNFAKLDF
NILLELHKEELNELTKWWKELDFTTKLPYARDRLVALYFWDLGMEFEPQYAFGRKIM
TQLNYILSIIDDTYFFYGTLEELSLFTEAVQRWNIEAVDTLPEYMKLIYRTLLDAFNEIE
EDMAKQGRSHLVRYAKEAFQKVVGAYFVLAKWASEGYVPTIEEYMPIALTSCAMTF
VITVSFLGMGDFATKEVFEWISDNPKVVKAASVICLLLDDMQGHEFEQKRGHVASAIE
CYTKQHGVSKEEAIKMFEEEVANARKDINEELMMKPQVVARPLLGTILNLARAIDFIY
KEDDGYTHSYLIKDQIASVLGDEVPF.
(SEQ ID NO: 164) 
ATGAGATCAAAGGAAACTTTCCGTCCAACCGCTGACTTCCACCCATCTTTGTGGAG
AAACCATTTCTTGAAAGGTGCATCTGATTTCAAGACTGTTGACCACACTGCTACTC
AAGAAAGACACGAAGCTTTAAAGGAAGAAGTCAGAAGAATGATCACTGACGCTG
AAGACAAGCCAGTTCAAAAGTTGAGATTGATCGATGAAGTCCAAAGATTAGGTGT
TGCCTACCACTTCGAAAAGGAAATCGAAGATGCTATCCAGAAATTGTCTCCAAACT
ATATTCACAGCAACTCTCCAGACTTGGCCACCGTCTCCTTGGCTTTCAGACTGTTG
AGACAAGGTGGTATCAAGATTTCTGCTGATGTCTTCGAAAAGTTCTACGATGACGA
AGGTAGATTCAAGTCTTCTTTGATCAACGATGTTCAAGGTTTGCTCTCTTTGTACGA
AGCTGCCTATATGGCTGTTCGTGGTGAACACATTCTAGATGAAGCCATTGCTTTCAC
CACAACCCATTTGAAGTCCCTCGTTGCTCAAGATGCTGTCACTCCAAAGTTGGCTG
AACAAATCAACCACGCTTTATACCGTCCTCTTAGAAAGACCTTACCTAGATTAGAA
GCGCGTTATTTCAGATCCATGATTCAATCTACCTCCGATCATCTCTACAACAAGACC
 
TTGTTGAACTTCGCTAAGTTGGACTTCAACATTTTGTTGGAATTGCACAAGGAAGA
GTTGAACGAATTGACTAAATGGTGGAAGGAACTTGACTTCACCACCAAGCTGCCA
TACGCCAGAGACAGATTGGTCGCCCTTTACTTCTGGGACTTGGGTATGGAATTCGA
ACCACAATACGCTTTCGGTAGAAAGATCATGACCCAATTGAACTACATCCTAAGTAT
CATCGATGACACTTACTTCTTCTACGGTACTTTGGAAGAGTTATCCTTGTTCACTGA
AGCCGTCCAAAGATGGAACATCGAAGCTGTTGATACGTTGCCAGAATACATGAAAT
TGATTTACAGAACTTTGTTGGACGCTTTCAACGAAATTGAAGAAGACATGGCCAA
GCAAGGTAGATCTCACTTGGTTAGATACGCTAAGGAAGCCTTCCAAAAGGTTGTTG
GTGCTTACTTTGTCTTGGCCAAGTGGGCATCTGAAGGTTACGTTCCAACTATTGAA
GAATACATGCCAATCGCTTTGACTTCTTGTGCTATGACCTTTGTTATTACTGTCTCTT
TCTTGGGTATGGGTGACTTTGCTACCAAGGAAGTTTTTGAATGGATCTCTGACAAC
CCAAAGGTCGTCAAGGCTGCTTCCGTGATCTGTTTGCTATTAGATGATATGCAAGGT
CACGAATTTGAACAAAAGAGAGGTCATGTCGCTTCCGCCATCGAATGTTACACCA
AGCAACACGGTGTCTCCAAGGAAGAAGCTATCAAGATGTTCGAAGAAGAAGTTGC
TAACGCTAGAAAGGACATTAATGAAGAATTGATGATGAAACCACAAGTTGTTGCTC
GTCCATTGTTGGGCACCATTTTGAATTTAGCTAGAGCTATTGACTTCATTTACAAGG
AAGATGACGGTTACACTCACTCTTACTTGATCAAGGATCAAATTGCCTCCGTTTTG
GGTGACGAAGTCCCATTCTGA.
(SEQ ID NO: 167) 
MSSKETFRPTADFHPSLWRNHFLKGASDFKTVDHTATQERHEALKEEVRRMITDAED
KPVQKLRLIDEVQRLGVAYHFEKEIEDAIQKLSPNYIHSNSPDLATVSLAFRLLRQGGI
KISCDVFEKFKDDEGRFKSSLINDVQGLLSLYEAAYMAVRGEHILDEAIAFTTTHLKSL
VAQDAVTPKLAEQINHALYRPLRKTLPRLEARYFMSMINSTSDHLYNKTLLNFAKLDF
NILLELHKEELNELTKWWKELDFTTKLPYARDRLVALYFWDLGTEFEPQYAFGRKIMT
QLNYILSIIDDTYDAYGTLEELSLFTEAVQRWNIEAVDTLPEYMKLIYRTLLDAFNEIEE
DMAKQGRSHLVRYAKEAFQKVIGAYFVLAKWFSEGYVPTIEEYMPIALTSCAMTFVIT
VSFLGMGDFATKEVFEWISDNPKVVKAASVICLLLDDMQGHEFEQKRGHVASAIECY
TKQHGVSKEEAIKMFEEEVANAWKDINEELMMKPQVVARPLLGTILNLARAIDFIYKE
DDGYTHSYLIKDQIASVLGDEVPF.
(SEQ ID NO: 168) 
ATGTCTTCCAAGGAAACCTTCAGACCAACTGCTGATTTCCACCCATCTTTGTGGAG
AAACCATTTCTTAAAGGGTGCTTCCGATTTCAAGACTGTTGACCACACTGCCACCC
AAGAAAGACACGAAGCTTTGAAGGAAGAAGTTAGAAGAATGATCACTGATGCTG
AAGACAAGCCTGTTCAAAAGTTACGTTTGATTGACGAAGTCCAAAGACTAGGTGT
 
TGCTTACCACTTCGAAAAGGAAATTGAAGACGCTATCCAAAAATTGAGTCCAAACT
ACATTCACTCCAACTCACCAGACTTGGCTACTGTTTCTTTGGCCTTCAGATTATTGA
GACAAGGTGGTATCAAGATCTCTTGTGATGTTTTCGAAAAGTTCAAGGATGACGAA
GGTAGATTCAAATCTTCTTTGATTAACGATGTCCAAGGCTTGCTGTCCTTATACGAA
GCCGCTTACATGGCTGTCCGTGGTGAACACATCTTGGACGAAGCTATTGCTTTCAC
CACCACCCACTTGAAGTCCTTAGTGGCTCAAGATGCCGTTACTCCAAAGTTGGCTG
AACAAATCAACCACGCTTTGTACAGACCATTGAGAAAGACCTTGCCAAGATTGGA
AGCTCGTTACTTCATGTCCATGATTAACTCCACTTCTGACCATTTGTACAACAAGAC
ACTACTCAACTTCGCTAAGTTGGATTTCAACATCTTGTTGGAATTGCACAAGGAAG
AATTGAACGAATTGACCAAATGGTGGAAGGAATTAGACTTCACCACTAAGTTGCCA
TACGCCAGAGACAGATTGGTCGCATTGTATTTCTGGGATTTGGGTACCGAATTTGA
GCCACAATACGCTTTCGGTCGTAAAATCATGACTCAATTGAACTACATTTTGTCTAT
CATAGACGATACATACGACGCTTACGGTACTTTGGAAGAACTTTCTTTGTTCACTGA
AGCTGTTCAAAGATGGAACATTGAAGCTGTCGACACCCTACCAGAATACATGAAG
TTAATTTACAGAACTTTGCTAGACGCCTTTAATGAAATCGAAGAAGATATGGCCAA
GCAAGGTAGATCTCATTTGGTTAGATACGCCAAAGAAGCCTTCCAGAAGGTCATCG
GTGCCTATTTCGTCTTGGCTAAGTGGTTCTCTGAAGGTTACGTTCCAACCATTGAA
GAATATATGCCAATTGCTTTGACTTCCTGCGCTATGACTTTTGTTATCACCGTATCAT
TCTTGGGTATGGGTGACTTTGCTACCAAGGAAGTTTTTGAATGGATTTCCGACAAC
CCAAAGGTCGTCAAGGCTGCCTCCGTTATCTGTTTGTTGTTGGACGACATGCAAGG
TCACGAATTCGAACAAAAGAGAGGTCATGTTGCTTCTGCTATTGAATGTTACACCA
AGCAACACGGTGTCTCTAAGGAAGAAGCTATCAAGATGTTCGAAGAAGAAGTCGC
TAACGCTTGGAAGGACATCAATGAAGAATTGATGATGAAACCTCAAGTTGTTGCTA
GACCATTGTTAGGTACCATTTTGAACTTGGCCAGAGCTATTGATTTCATCTACAAGG
AAGATGATGGTTACACTCACTCTTACTTGATCAAGGACCAAATCGCGTCTGTTTTG
GGTGATGAAGTCCCATTTTGA.
(SEQ ID NO: 171) 
MSSKETFRPTADFHPSLWRNHFLKGASDFKTVDHTATQERHEALKEEVRRMITDAED
KPVQKLRLIDEVQRLGVAYHFEKEIEDAIQKLCPNYIHSNSPDLHTVSLAFRLLRQGGI
KISCDVFEKFKDDEGRFKSSLINDVQGLLSLYEAAYMAVRGEHILDEAIAFTTTHLKSL
VAQDHVTPKLAEQINHALYRPLRKTLPRLEARYFMSMINSTSDHLYNKTLLNFAKLDF
NILLELHKEELNELTKWWKELDFTTKLPYARDRLVELYFWDLGTYFEPQYAFGRKIM
TQLNYILSIIDDTYDAYGTLEELSLFTEAVQRWNIEAVDTLPEYMKLIYRTLLDAFNEIE
EDMAKQGRSHCVRYAKEAFQKVIGAYFVQAKWFSEGYVPTIEEYMPIALTSCAYTFVI
 
TNSFLGMGDFATKEVFEWISDNPKVVKAASVICLLLDDMQGHEFEQKRGHVASAIEC
YTKQHGVSKEEAIKMFEEEVANAWKDINEELMMKPTVVARPLLGTILNLARAIDFIYK
EDDGYTHSYLIKDQIASVLGDHVPF.
(SEQ ID NO: 172)

ATGTCTTCCAAGGAAACTTTCAGACCAACTGCTGACTTCCACCCATCTTTGTGGAG
AAATCATTTCTTGAAGGGTGCTTCTGACTTCAAAACTGTCGATCACACCGCCACCC
AAGAAAGACACGAAGCTTTGAAAGAAGTCAGAAGAATGATCACTGATGCTG
AAGACAAGCCTGTCCAAAAATTGAGATTGATTGATGAAGTCCAAAGACTAGGTGT
TGCTTACCACTTCGAAAAGGAAATCGAGGATGCTATCCAAAAGTTGTGTCCAAACT
ACATTCACTCCAACTCTCCAGATTTGCACACTGTTTCCTTGGCTTTCAGATTATTGA
GACAAGGTGGTATTAAGATTTCATGTGATGTCTTTGAAAAGTTCAAGGATGATGAA
GGTAGATTCAAGTCTTCTTTGATCAACGATGTCCAAGGTTTGTTGTCCCTATACGAA
GCCGCTTATATGGCTGTCAGAGGTGAACACATTTTGGACGAAGCTATTGCTTTCACT
ACCACTCACTTGAAGAGCCTAGTCGCTCAAGATCATGTCACTCCAAAGTTGGCTG
AACAAATCAACCACGCTTTGTACAGACCTCTTAGAAAGACCTTACCAAGATTGGA
AGCTCGTTATTTCATGTCCATGATTAATTCTACCTCCGACCATTTGTACAACAAGAC
TCTCTTGAACTTCGCTAAGTTGGATTTCAACATTTTGTTGGAATTGCACAAGGAAG
AATTAAACGAATTGACCAAATGGTGGAAAGAACTAGATTTCACCACCAAGTTGCCA
TACGCTAGAGACAGATTGGTTGAATTATACTTCTGGGACTTGGGCACCTACTTTGA
ACCACAATACGCCTTCGGTCGGAAGATCATGACTCAATTGAACTATATCTTGTCCAT
CATCGATGACACTTACGACGCTTACGGTACTCTGGAAGAATTGTCTTTATTCACTGA
AGCTGTCCAAAGATGGAACATCGAAGCTGTTGACACCTTGCCAGAATACATGAAA
TTAATTTACCGTACTTTGTTGGACGCATTTAATGAAATTGAAGAAGACATGGCCAA
GCAAGGTCGTAGTCATTGCGTTAGATACGCTAAGGAAGCTTTCCAAAAGGTTATTG
GTGCTTACTTCGTTCAGGCTAAGTGGTTCTCTGAAGGTTACGTTCCAACCATTGAA
GAATACATGCCAATCGCCTTGACTTCTTGTGCTTACACCTTTGTGATCACCAACTCT
TTCTTGGGTATGGGTGACTTTGCTACCAAGGAAGTTTTCGAATGGATTTCCGACAA
CCCAAAGGTCGTCAAGGCTGCCTCCGTTATCTGTTTGTTATTGGACGACATGCAAG
GTCACGAATTCGAACAAAAGAGAGGTCACGTTGCCTCTGCTATCGAATGTTACACT
AAACAACACGGTGTCTCTAAGGAAGAAGCCATCAAGATGTTCGAAGAAGAAGTT
GCTAACGCTTGGAAGGACATCAACGAAGAATTGATGATGAAGCCAACAGTTGTTG
CCAGACCATTGTTAGGTACTATCTTGAACTTGGCGCGTGCCATTGACTTCATCTACA
AGGAAGATGACGGTTACACCCACTCTTACTTGATTAAGGACCAAATAGCTTCTGTT
TTGGGTGATCATGTTCCATTCTGA.

| **Primer** | **Sequence (from 5' to 3')** |
|---|---|
| SEQ ID NO:57 | CATGATATCGACAAAGGAAAAGGGGCCTGTTCACATTG GAATTGGTTGGACC |
| SEQ ID NO:58 | gtaagaatttttgaaaattcaatataaATGGAAGTCAAGCGTAGATC |
| SEQ ID NO:61 | GATATCGACAAAGGAAAAGGGGCCTGTTCACATTGGGA TTGGTTGAACTAG |
| SEQ ID NO:62 | gaatttttgaaaattcaatataaATGGAAGTTAAGAGAAGATCTGC |
| SEQ ID NO:65 | CATGATATCGACAAAGGAAAAGGGGCCTGTTCACATTG GGATTGGTTGGACC |
| SEQ ID NO:66 | gtaagaatttttgaaaattcaatataaATGGAAGTCAAGCGGAGATC |
| SEQ ID NO:69 | CATGATATCGACAAAGGAAAAGGGGCCTGTTCACATTG GGATTGGTTGGACC |
| SEQ ID NO:70 | gtaagaatttttgaaaattcaatataaATGGAAGTCAAGAGAAGATCCG |
| SEQ ID NO:73 | CATGATATCGACAAAGGAAAAGGGGCCTGTTCACATTG GGATTGGTTGAACC |
| SEQ ID NO:74 | gtaagaatttttgaaaattcaatataaATGGAAGTCAAGAGAAGATCCG |
| SEQ ID NO:77 | CATGATATCGACAAAGGAAAAGGGGCCTGTTCACATTG GGATTGGTTGAACC |
| SEQ ID NO:78 | gtaagaatttttgaaaattcaatataaATGGAAGTGAAGAGAAGATCTG |
| SEQ ID NO:81 | CATGATATCGACAAAGGAAAAGGGGCCTGTTCACATTG GGATTGGTTGGACC |
| SEQ ID NO:82 | taagaatttttgaaaattcaatataaATGGAAGTTAAGAGAAGATCTGC |
| SEQ ID NO:85 | TCGACAAAGGAAAAGGGGCCTGTTCACATTGGAATTGG TTGAACTAACAAAG |
| SEQ ID NO:86 | taagaatttttgaaaattcaatataaATGGAAGTTACTAGAAGATCCGC |
| SEQ ID NO:89 | CATGATATCGACAAAGGAAAAGGGGCCTGTTCACATTG GAATTGGGTCAACC |
| SEQ ID NO:90 | agtaagaatttttgaaaattcaatataaATGGAAGTCACCAGAAGATCC |
| SEQ ID NO:93 | CATGATATCGACAAAGGAAAAGGGGCCTGTTCACATTG GAATTGGGTCAACC |
| SEQ ID NO:94 | taagaatttttgaaaattcaatataaATGGAAGTCACTAGAAGATCTGC |
| SEQ ID NO:97 | CATGATATCGACAAAGGAAAAGGGGCCTGTTCAAGCTG GAATTGGAGTGTGG |
| SEQ ID NO:98 | gtaagaatttttgaaaattcaatataaATGTCTCTCTTGGTCTTGTCC |
| SEQ ID NO:101 | CATGATATCGACAAAGGAAAAGGGGCCTGTTCAAGCTG GGATTGGAGTGAC |
| SEQ ID NO:102 | gtaagaatttttgaaaattcaatataaATGTCTCTATTGGTCTTGTCTACC |
| SEQ ID NO:105 | CATGATATCGACAAAGGAAAAGGGGCCTGTTCAAGCTG GAATTGGAGTCAC |
| SEQ ID NO:106 | gtaagaatttttgaaaattcaatataaATGTCTTTGTTGGTCTTGTCC |
| SEQ ID NO:109 | CATGATATCGACAAAGGAAAAGGGGCCTGTTCAAGCTG GGATTGGAGTAACAA |
| SEQ ID NO:110 | gtaagaatttttgaaaattcaatataaATGTCCCTGTTGGTCTTGTC |
| SEQ ID NO:113 | CATGATATCGACAAAGGAAAAGGGGCCTGTTCAAGCTG GGATAGGAGTAACG |
| SEQ ID NO:114 | gtaagaatttttgaaaattcaatataaATGTCTTTGTTAGTTTTGTCCAC |
| SEQ ID NO:117 | CATGATATCGACAAAGGAAAAGGGGCCTGTTCAAGCTG GGATTGGAGTAACG |
| SEQ ID NO:118 | gtaagaatttttgaaaattcaatataaATGTCTTTGCTAGTCTTGTCTAC |
| SEQ ID NO:121 | CATGATATCGACAAAGGAAAAGGGGCCTGTTCACATTG GAATTGGGGTGACG |
| SEQ ID NO:122 | gtaagaatttttgaaaattcaatataaATGTCATTAAACGTCCTCTCTAC |
| SEQ ID NO:125 | TATCGACAAAGGAAAAGGGGCCTGTTCACATTGGAATT GGAGTAACAAAGAG |
| SEQ ID NO:126 | gtaagaatttttgaaaattcaatataaATGTCTTTGAACGTGTTGTCC |
| SEQ ID NO:129 | GATATCGACAAAGGAAAAGGGGCCTGTTCACATTGGGA TTGGGTCAACAAATAAG |
| SEQ ID NO:130 | gtaagaatttttgaaaattcaatataaATGTCTCTTAATGTCTTGTCCAC |
| SEQ ID NO:133 | CATGATATCGACAAAGGAAAAGGGGCCTGTTCACATTG GGATTGGGTCAAC |
| SEQ ID NO:134 | gtaagaatttttgaaaattcaatataaATGTCTTTGAACGTATTGTCAAC |
| SEQ ID NO:137 | CATGATATCGACAAAGGAAAAGGGGCCTGTTCAGAATG GCAATTCGTCTCCC |
| SEQ ID NO:138 | gtaagaatttttgaaaattcaatataaATGAGAAAGAAGGAAACCGAAA G |
| SEQ ID NO:141 | CATGATATCGACAAAGGAAAAGGGGCCTGTTCAGAATG GCAATTCATCGCC |
| SEQ ID NO:142 | gtaagaatttttgaaaattcaatataaATGAGAAAGAAAGAAACCGAAA G |
| SEQ ID NO:145 | CATGATATCGACAAAGGAAAAGGGGCCTGTTCAGAATG GTAGTTCGTCACCC |
| SEQ ID NO:146 | gtaagaatttttgaaaattcaatataaATGAGAAAGAAGGAAACCGAAA G |
| SEQ ID NO:149 | CATGATATCGACAAAGGAAAAGGGGCCTGTTCAGAATG GCAATTCATCACCC |
| SEQ ID NO:150 | gtaagaatttttgaaaattcaatataaATGAGAAAGAAAGAAACTTTCA GACC |
| SEQ ID NO:153 | CATGATATCGACAAAGGAAAAGGGGCCTGTTCAGAATG GCAATTCATCACCC |
| SEQ ID NO:154 | gtaagaatttttgaaaattcaatataaATGAGATCCAAGGAAACTTTCAG |
| SEQ ID NO:157 | CATGATATCGACAAAGGAAAAGGGGCCTGTTCAGAATG GCAATTCGTCGC |
| SEQ ID NO:158 | gtaagaatttttgaaaattcaatataaATGAGATCTAAAGAAACTTTCCG TC |
| SEQ ID NO:161 | CATGATATCGACAAAGGAAAAGGGGCCTGTTCAGAATG GGACTTCGTCACC |
| SEQ ID NO:162 | gtaagaatttttgaaaattcaatataaATGAGATCTAAGGAAACTTTCAG AC |
| SEQ ID NO:165 | CATGATATCGACAAAGGAAAAGGGGCCTGTTCAGAATG GGACTTCGTCACC |
| SEQ ID NO:166 | gtaagaatttttgaaaattcaatataaATGAGATCAAAGGAAACTTTCCG |
| SEQ ID NO:169 | CATGATATCGACAAAGGAAAAGGGGCCTGTTCAAAATG GGACTTCATCACCC |
| SEQ ID NO:170 | gtaagaatttttgaaaattcaatataaATGTCTTCCAAGGAAACCTTC |
| SEQ ID NO:173 | GATATCGACAAAGGAAAAGGGGCCTGTTCAGAATGGAA |
| | CATGATCACCCAAAAC |
| SEQ ID NO:174 | gtaagaatttttgaaaattcaatataaATGTCTTCCAAGGAAACTTTCAG |

## Claims

1. A valencene synthase, **characterized in that** the valencene synthase comprises Pfam domains numbered PF01397 and PF03936 and has valencene synthase activity.

2. The valencene synthase according to claim 1, **characterized in that** the valencene synthase comprises (a) an intact protein or functional protein fragment derived from a plant; or (b) a derivative valencene synthase having at least 80% or higher amino acid sequence homology to (a).

3. The valencene synthase according to claim 2, **characterized in that** the plant is selected from plants of the family *Rutaceae* or plants of the family *Ranunculaceae Juss.*

4. The valencene synthase according to claim 2 or 3, **characterized in that** the plant is selected from plants of the genus *Cimicifuga* or plants of the genus *Citrus L.*

5. The valencene synthase according to claim 4, **characterized in that** the plants of the genus *Cimicifuga* comprise *Actaea asiatica,* preferably *Actaea racemosa,* and the plants of the genus *Citrus L.* comprise *Citrus maxima.*

6. The valencene synthase according to any one of claims 1-5, **characterized in that** the valencene catalyzes the synthesis of valencene in an expression system.

7. The valencene synthase according to claim 6, **characterized in that** the expression system comprises a fungal expression system.

8. The valencene synthase according to claim 7, **characterized in that** the fungal expression system comprises a yeast expression system.

9. The valencene synthase according to claim 8, **characterized in that** the yeast expression system comprises a *Saccharomyces cerevisiae* expression system.

10. The valencene synthase according to any one of claims 1-9, **characterized in that** the amino acid sequence of the valencene synthase comprises (a) or (b):
(a) any one of SEQ ID NOs: 1-3;
(b) a derivative valencene synthase having at least 83%, 85%, 87%, 89%, 91%, 93%, 95%, 97%, or 99% or higher amino acid sequence homology to (a).

11. Use of the valencene synthase according to any one of claims 1-10 in the preparation of a fragrance composition.

12. A nucleic acid molecule, **characterized in that** the nucleic acid molecule comprises a nucleotide sequence encoding the valencene synthase according to any one of claims 1-10 or a nucleotide sequence having bases complementarity thereto.

13. The nucleic acid molecule according to claim 12, **characterized in that** the nucleic acid molecule comprises an amino acid sequence having at least 90%, 91%, 92%, 93%, 94%, 95%, 96%, 97%, 98%, 99%, or 100% sequence homology to any one of nucleotide sequences as set forth in SEQ ID Nos: 4-6.

14. A recombinant expression vector containing the nucleic acid molecule according to claim 12 or 13, **characterized in that** the original vector of the recombinant expression vector is pZY900, pYR017, or pYR018.

15. A transformant containing the nucleic acid molecule according to claim 12 or 13, or the recombinant expression vector according to claim 14, wherein the host cell of the transformant is selected from a fungal expression system, preferably a yeast expression system, more preferably a *Saccharomyces cerevisiae* expression system.

16. A recombinant *Saccharomyces cerevisiae,* **characterized in that** the recombinant *Saccharomyces cerevisiae* contains the nucleic acid molecule according to claim 12 or 13, or the recombinant expression vector according to claim 14; and preferably, the original vector of the recombinant expression vector is pZY900, pYR017, or pYR018.

17. A fragrance composition, **characterized in that** the fragrance composition comprises 10-99% by mass of valencene; and the valencene is synthesized under catalysis of the valencene synthase according to any one of claims 1-10.

18. A preparation method for the fragrance composition according to claim 17, **characterized in that** the preparation method comprises fermenting and culturing the transformant according to claim 15 or the recombinant *Saccharomyces cerevisiae* according to claim 16, and recovering the fragrance composition from a fermentation product.

19. The preparation method according to claim 18, **characterized in that** the recovery method comprises extracting the fermentation product with an oil phase.

20. The preparation method according to claim 19, **characterized in that** the oil phase comprises at least one of isopropyl myristate or n-dodecane.

21. The preparation method according to claim 19, **characterized in that** the recovery method is: during fermentation, adding the oil phase; and after the fermentation is complete, collecting the oil phase and performing separation to obtain a fragrance composition enriched in the oil phase.

22. The preparation method according to claim 21, **characterized in that** the method for collecting the oil phase comprises centrifugation.

23. The preparation method according to claim 21, **characterized in that** the method for the separation comprises rectification.

24. Use of the fragrance composition according to claim 17 or a fragrance composition obtained by the preparation method according to any one of claims 18-23 in the preparation of a fragrance product.

25. A fragrance product containing the fragrance composition according to claim 17 or a fragrance composition obtained by the preparation method according to any one of claims 18-23.

26. The fragrance product according to claim 25, **characterized in that** the fragrance product comprises: a personal care product, a fabric care product, a cleaning product, a deodorant, a home care product, a fabric product, an air care product, a fragrance delivery system, a cosmetic preparation, a disinfectant, or a food product.

27. The fragrance product according to claim 26, **characterized in that** the personal care product comprises: floral water, a skin care product, a hair care product, an antiperspirant, a feminine care product, a baby care product, an oral hygiene product, or a health care product.

28. The fragrance product according to claim 27, **characterized in that** the skin care product comprises: a scented soap, a liquid soap, or a shower gel.

29. The fragrance product according to claim 27, **characterized in that** the oral hygiene product comprises a dental hygiene product.

30. The fragrance product according to claim 26, **characterized in that** the cosmetic preparation comprises a perfume, a fragrance liquid, or an essential oil.

31. The fragrance product according to claim 26, **characterized in that** the cleaning product comprises a stain-removing composition.

32. The fragrance product according to claim 31, **characterized in that** the stain-removing composition comprises: a dishwashing detergent, a glass cleaner, a metal cleaner, a countertop cleaner, a floor cleaner, a carpet cleaner, a toilet cleaner, or a bleaching additive.
